# EUROPEAN PATENT APPLICATION

(11) **EP 1 589 016 A1**
(43) Date of publication of application: **26.10.2005**
(21) Application number: 04290980.4
(22) Date of filing: 13.04.2004
(51) Int. Cl.: C07D 405/12, C07D 333/76, C07D 319/16, C07D 307/91, C07D 307/79, C07D 295/18, C07D 209/10, C07C 323/60, C07C 317/44, A61K 31/496, A61K 31/475, A61K 31/445, A61K 31/436, A61K 31/4025

(54) **Thio-substituted tricyclic and bicyclic aromatic methanesulfinyl derivatives**

(71) Applicant: CEPHALON FRANCE, 94700 Maisons-Alfort (FR)
(72) Inventor: Lesur, Brigitte, 77420 Champs-sur-Marne (FR); Yue, Christophe, 94300 Vincennes (FR); Chasset, Sophie, 77176 Nandy (FR)
(74) Representative: Bernasconi, Jean Raymond

(57) **Abstract**

The present invention is related to chemical compositions, processes for the preparation thereof and uses of the composition. Particularly, the present invention relates to compositions of compounds of Formula (A): 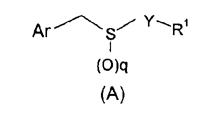 wherein Ar, Y, R¹ and q are as defined herein; and their use in the treatment of diseases, including treatment of sleepiness, promotion of wakefulness, treatment of Parkinson's disease, cerebral ischemia, stroke, sleep apneas, eating disorders, stimulation of appetite and weight gain, treatment of attention deficit hyperactivity disorder ("ADHD"), enhancing function in disorders associated with hypofunctionality of the cerebral cortex, including, but not limited to, depression, schizophrenia, fatigue, in particular, fatigue associated with neurologic disease, such as multiple sclerosis, chronic fatigue syndrome, and improvement of cognitive dysfunction.

## Description

### FIELD OF THE INVENTION

The present invention is related to chemical compositions, processes for the preparation thereof and uses of the composition. Particularly, the present invention relates to compositions of compounds of Formula (A): 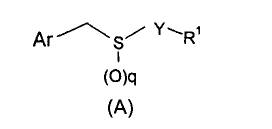 and their use in the treatment of diseases, including treatment of sleepiness, promotion and/or improvement of wakefulness, preferably improvement of wakefulness in patients with excessive sleepiness associated with narcolepsy, sleep apnea, preferably obstructive sleep apnea/hypopnea, and shift work disorder ; treatment of Parkinson's disease ; Alzheimer's disease ; cerebral ischemia ; stroke ; eating disorders ; attention deficit disorder ("ADD"), attention deficit hyperactivity disorder ("ADHD") ; depression ; schizophrenia ; fatigue, preferably fatigue associated with cancer or neurological diseases, such as multiple sclerosis and chronic fatigue syndrome ; stimulation of appetite and weight gain and improvement of cognitive dysfunction.

### BACKGROUND OF THE INVENTION

The compounds disclosed herein are related to the biological and chemical analogs of modafinil. Modafinil, C₁₅H₁₅NO₂S, also known as 2-(benzhydrylsulfinyl) acetamide, or 2-[(diphenylmethyl) sulfinyl] acetamide, a synthetic acetamide derivative with wake-promoting activity, has been described in French Patent No. 78 05 510 and in U.S. Patent No. 4,177,290 ("the '290 patent"). It has been approved by the United States Food and Drug Administration for use in the treatment of excessive daytime sleepiness associated with narcolepsy. Methods for preparing modafinil and several derivatives are described in the '290 patent. The levorotatory isomer of modafinil, along with additional modafinil derivatives are described in U.S. Patent No. 4,927,855, and are reported to be useful for treatment of hypersomnia, depression, Alzheimer's disease and to have activity towards the symptoms of dementia and loss of memory, especially in the elderly.

Modafinil has also been described as a useful agent in the treatment of Parkinson's disease (U.S. Patent No. 5,180,745); in the protection of cerebral tissue from ischemia (U.S. Patent No. 5,391,576); in the treatment of urinary and fecal incontinence (U.S. Patent No. 5,401,776); and in the treatment of sleep apneas and disorders of central origin (U.S. Patent No. 5,612,379). In addition, modafinil may be used in the treatment of eating disorders, or to promote weight gain or stimulate appetite in humans or animals (U.S. Patent No. 6,455,588), or in the treatment of attention deficit hyperactivity disorder (U.S. Patent No. 6,346,548), or fatigue, especially fatigue associated with multiple sclerosis (US Patent No. 6,488,164). U.S. Pat. No. 4,066,686 describes various benzhydrylsulphinyl derivatives as being useful in therapy for treating disturbances of the central nervous system.

Several published patent applications describe derivative forms of modafinil and the use of modafinil derivatives in the treatment of various disorders. For example, PCT publication WO 99/25329 describes various substituted phenyl analogs of modafinil as being useful for treating drug-induced sleepiness, especially sleepiness associated with administration of morphine to cancer patients. U.S. Pat No. 5,719,168 and PCT Publication No. 95/01171 describes modafinil derivatives that are useful for modifying feeding behavior. PCT Publication No. 02/10125 describes several modafinil derivatives of modafinil, along with various polymorphic forms of modafinil.

Additional publications describing modafinil derivatives include U.S. Pat. No. 6,492,396, and PCT Publication No. WO 02/10125.

Terauchi, H, et al. described nicotinamide derivatives useful as ATP-ase inhibitors (Terauchi, H, et al, *J. Med. Chem*., 1997*, 40,* 313-321)*.* In particular, several N-alkyl substituted 2-(Benzhydrylsulfinyl) nicotinamides are described.

U.S. Pat. Nos. 4,980,372 and 4,935,240 describe benzoylaminophenoxybutanoic acid derivatives. In particular, sulfide derivatives of modafinil containing a phenyl and substituted phenyl linker between the sulfide and carbonyl, and a substituted aryl in the terminal amide position, are disclosed.

Other modafinil derivatives have been disclosed wherein the terminal phenyl groups are constrained by a linking group. For example, in U.S. Pat. No. 5,563,169, certain xanthenyl and thiaxanthenyl derivatives having a substituted aryl in the terminal amide position are reported.

Other xanthenyl and thiaxanthenyl derivatives are disclosed in Annis, I; Barany, G. *Pept. Proc. Am. Pept. Symp. 15*^{*th*} (Meeting Date 1997) 343-344, 1999 (preparation of a xanthenyl derivative of Ellman's Reagent, useful as a reagent in peptide synthesis); Han, Y.; Barany, G. *J*. *Org. Chem*., 1997, *62*, 3841-3848 (preparation of S-xanthenyl protected cysteine derivatives, useful as a reagent in peptide synthesis); and El-Sakka, I.A., et al. *Arch. Pharm. (Weinheim),* 1994, *327*, 133-135 (thiaxanthenol derivatives of thioglycolic acid).

Thus, there is a need for novel classes of compounds that possess the beneficial properties. It has been discovered that a class of compounds, referred to herein as substituted thioacetamides, are useful as agents for treating or preventing various diseases or disorders disclosed herein.

### SUMMARY OF THE INVENTION

The present invention in one aspect is directed to various novel compounds of structure: 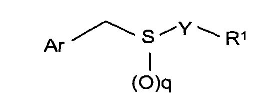 and its stereoisomeric forms, mixtures of stereoisomeric forms, or pharmaceutically acceptable salt forms thereof, wherein the constituent members are defined *infra*.

Another object of the present invention is to provide pharmaceutical compositions comprising the compounds of the present invention wherein the compositions comprise one or more pharmaceutically acceptable excipients and a therapeutically effective amount of at least one of the compounds of the present invention, or a pharmaceutically acceptable salt or ester form thereof.

Another object of the present invention is to provide methods of treating or preventing diseases or disorders, including treatment of sleepiness, promotion and/or improvement of wakefulness, preferably improvement of wakefulness in patients with excessive sleepiness associated with narcolepsy, sleep apnea, preferably obstructive sleep apnea/hypopnea, and shift work disorder ; treatment of Parkinson's disease ; Alzheimer's disease ; cerebral ischemia ; stroke ; eating disorders ; attention deficit disorder ("ADD"), attention deficit hyperactivity disorder ("ADHD") ; depression ; schizophrenia ; fatigue, preferably fatigue associated with cancer or neurological diseases, such as multiple sclerosis and chronic fatigue syndrome ; stimulation of appetite and weight gain and improvement of cognitive dysfunction.

These and other objects, features and advantages of the substituted benzylthioalkyl will be disclosed in the following detailed description of the patent disclosure.

### DETAILED DESCRIPTION OF THE INVENTION

In a first embodiment, the present invention provides novel compounds of formula (A): 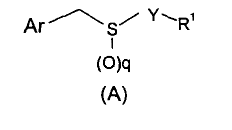 wherein :
Ar is: 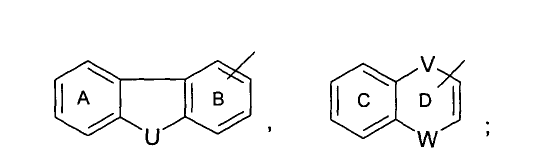
Wherein:
U is CH₂, CR²⁵R²⁶, O, S(O)_{y}, NR¹⁰, C(=O), C(=S), CHOH, CHOR¹⁴, C=NOR¹⁴, or C=NNR¹²R¹³;
V and W are independently selected from a bond, CH₂, CR²⁵R²⁶, O, S(O)_{y}, NR¹⁰, C(=O), C(=S), CHOH, CHOR¹⁴, C=NOR¹⁴, or C=NNR¹²R¹³;
rings A, B, and C are optionally substituted with one to three groups selected from F, Cl, Br, I, OR²², OR²⁷, NR²³R²⁴, NHOH, NO₂, CN, CF₃, C₁-C₆ alkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
ring D is optionally substituted with one group selected from C₁-C₆ alkyl, phenyl, and 5-10 membered heteroaryl;
   - Y is: C₁-C₆ alkylene; or
   (C₁-C₄ alkylene)ₘ-Z-(C₁-C₄ alkylene)ₙ;
   wherein said alkylene groups are optionally substituted with one to three R²⁰ groups;
   - Z is: O, NR^{10A}, S(O)_{y}, CR²¹=CR²¹, C≡C, C₆-C₁₀ arylene, 5-10 membered heteroarylene, C₃-C₆ cycloalkylene, or 3-6 membered heterocycloalkylene; wherein said arylene, heteroarylene, cycloalkylene, and heterocycloalkylene groups are optionally substituted with one to three R²⁰ groups;
   - R¹ is: selected from H, NR¹²R¹³, NR²¹C(=O)R¹⁴, C(=O)R¹⁴, CO₂R¹¹, OC(=O)R¹¹, C(=O)NR¹²R¹³, C(=NR¹¹)NR¹²R¹³, OC(=O)NR¹²R¹³, NR²¹S(O)₂R¹¹, NR²¹C(=O)NR¹²R¹³, NR²¹(SO₂)R¹²R¹³, and C(=O)NR¹¹ OR²²;
   - R¹⁰ and R^{10A}: are each independently selected from H, C₁-C₆ alkyl, C₆-C₁₀ aryl, C(=O)R¹⁴, and S(O)_{y}R¹⁴; wherein said alkyl and aryl groups are optionally substituted with one to three R²⁰ groups;
   - R¹¹: at each occurrence is independently selected from H, C₁-C₆ alkyl, and C₆-C₁₀ aryl; wherein said alkyl and aryl groups are optionally substituted with one to three R²⁰ groups;
   - R¹² and R¹³: at each occurrence are each independently selected from H, C₁-C₆ alkyl, C₆-C₁₀ aryl, and NR²³R²⁴, or R¹² and R¹³, together with the nitrogen to which they are attached, form a 3-7 membered heterocyclic ring;
   wherein said alkyl and aryl groups and heterocyclic ring are optionally substituted with one to three R²⁰ groups;
   - R¹⁴: at each occurrence is independently selected from C₁-C₆ alkyl, C₆-C₁₀ aryl, and alkylaryl;
   wherein said alkyl, aryl and alkylaryl groups are optionally substituted with one to three R²⁰ groups;
   - R²⁰: at each occurrence is independently selected from F, Cl, Br, I, OR²², OR²⁷, NR²³R²⁴, NHOH, NO₂, CN, CF₃, C₁-C₆ alkyl optionally substituted with OH, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
   - R²¹: at each occurrence is independently selected from H and C₁-C₆ alkyl;
   - R²²: at each occurrence is independently selected from H, C₁-C₆ alkyl optionally substituted with OH, arylalkyl and C₆-C₁₀ aryl;
   - R²³ and R²⁴: at each occurrence are each independently selected from H, C₁-C₆ alkyl, and C₆-C₁₀ aryl, or R²³ and R²⁴, together with the nitrogen to which they are attached, form a 3-7 membered heterocyclic ring optionally substituted with =O;
   - R²⁵ and R²⁶: at each occurrence are each independently selected from H, C₁-C₆ alkyl, and C₆-C₁₀ aryl, or R²⁵ and R²⁶, together with the carbon to which they are attached, form a 3-7 membered heterocyclic ring;
   - R²⁷: at each occurrence is independently the residue of an amino acid after the hydroxyl group of the carboxyl group is removed;
m is 0 or 1;
n is 0 or 1;
q is 0,1, or 2;
y is 0, 1, or 2;
   with the exclusion of the compounds wherein:
   U is CH₂, C(=O), CH(CH₃), S or C = NNHPh ; and
   Y is CH₂ ; and
   R¹ isH;
   and with the exclusion of the compounds wherein :
   U is CH₂ ; and
   Y is C₁-C₆ alkylene optionally substituted with C₁-C₆ alkylene ; and
   R¹ is CONH₂, or CO₂R¹¹ with R¹¹ = H or C₁-C₆ alkyl ;
   and with the exclusion of the compounds :
   ■ 3-[(methylthio)methyl]-2-phenyl-1H-inden-1-one
   ■ 3-[(methylsulfinyl)methyl]-2-phenyl-1H-inden-1-one
   and the stereoisomeric forms, mixtures of stereoisomeric forms or pharmaceutically acceptable salts forms thereof.

In a second embodiment, the present invention provides novel compounds of formula (I): 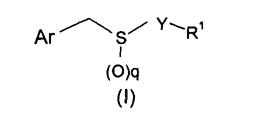 wherein
- Ar is: 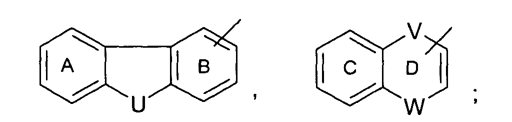 U is CH₂, CR²⁵R²⁶, O, S(O)_{y}, NR¹⁰, C(=O), C(=S), CHOH, CHOR¹⁴, C=NOR¹⁴, or C=NNR¹²R¹³;
V and W are independently selected from a bond, CH₂, CR²⁵R²⁶, O, S(O)_{y}, NR¹⁰, C(=O), C(=S), CHOH, CHOR¹⁴, C=NOR¹⁴, or C=NNR¹²R¹³;
rings A, B, and C are optionally substituted with one to three groups selected from F, Cl, Br, I, OR²², OR²⁷, NR²³R²⁴, NHOH, NO₂ CN, CF₃, C₁-C₆ alkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
ring D is optionally substituted with one group selected from C₁-C₆ alkyl, phenyl, and 5-10 membered heteroaryl;
- Y is: C₁-C₆ alkylene;
(C₁-C₄ alkylene)ₘ-Z¹-(C₁-C₄ alkylene)ₙ;
C₁-C₄ alkylene-Z²-C₁-C₄ alkylene;
wherein said alkylene groups are optionally substituted with one to three R²⁰ groups;
- Z¹ is: CR²¹=CR²¹, C ≡C, C₆-C₁₀ arylene, 5-10 membered heteroarylene,
C₃-C₆ cycloalkylene, or 3-6 membered heterocycloalkylene; wherein said arylene, heteroarylene, cycloalkylene, and heterocycloalkylene groups are optionally substituted with one to three R²⁰ groups;
- Z² is: O, NR^{10A}, or S(O)_{y};
- R¹ is: selected from H, NR¹²R¹³, NR²¹C(=O)R¹⁴, C(=O)R¹⁴, CO₂R¹¹, OC(=O)R¹¹, C(=O)NR¹²R¹³, C(=NR¹¹)NR¹²R¹³, OC(=O)NR¹²R¹³, NR²¹S(O)₂R¹¹, NR²¹C(=O)NR¹²R¹³, NR²¹S(O)₂NR¹²R¹³, and C(=O)NR¹¹ OR²²;
- R¹⁰ and R^{10A} are: each independently selected from H, C₁-C₆ alkyl, C₆-C₁₀ aryl, C(=O)R¹⁴, and S(O)_{y}R¹⁴; wherein said alkyl and aryl groups are optionally substituted with one to three R²⁰ groups;
- R¹¹: at each occurrence is independently selected from H, C₁-C₆ alkyl, and C₆-C₁₀ aryl; wherein said alkyl and aryl groups are optionally substituted with one to three R²⁰ groups;
- R¹² and R¹³: at each occurrence are each independently selected from H, C₁-C₆ alkyl, C₆-C₁₀ aryl, and NR²³R²⁴, or R¹² and R¹³, together with the nitrogen to which they are attached, form a 3-7 membered heterocyclic ring;
wherein said alkyl and aryl groups and heterocyclic ring are optionally substituted with one to three R²⁰ groups;
- R¹⁴: at each occurrence is independently selected from C₁-C₆ alkyl, C₆-C₁₀ aryl, and alkylaryl;
wherein said alkyl, aryl and alkylaryl groups are optionally substituted with one to three R²⁰ groups;
- R²⁰: at each occurrence is independently selected from F, Cl, Br, I, OR²², OR²⁷, NR²³R²⁴, NHOH, NO₂, CN, CF₃, C₁-C₆ alkyl optionally substituted with OH, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
- R²¹: at each occurrence is independently selected from H and C₁-C₆ alkyl;
- R²²: at each occurrence is independently selected from H, C₁-C₆ alkyl optionally substituted with OH, arylalkyl and C₆-C₁₀ aryl;
- R²³ and R²⁴: at each occurrence are each independently selected from H, C₁-C₆ alkyl, and C₆-C₁₀ aryl, or R²³ and R²⁴, together with the nitrogen to which they are attached, form a 3-7 membered heterocyclic ring optionally substituted with =O;
- R²⁵ and R²⁶: at each occurrence are each independently selected from H, C₁-C₆ alkyl, and C₆-C₁₀ aryl, or R²⁵ and R²⁶, together with the carbone to which they are attached, form a 3-7 membered heterocyclic ring;
- R²⁷: at each occurrence is independently the residue of an amino acid after the hydroxyl group of the carboxyl group is removed;
- m: is 0 or 1;
- n: is 0 or 1;
- q: is 0, 1, or 2;
- y: is 0, 1, or 2;
and the stereoisomeric forms, mixtures of stereoisomeric forms or pharmaceutically acceptable salts forms thereof.

In another embodiment, the present invention includes a compound of formula (II): 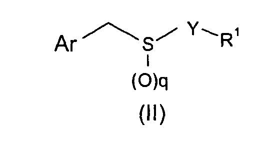 wherein
- Ar is :: 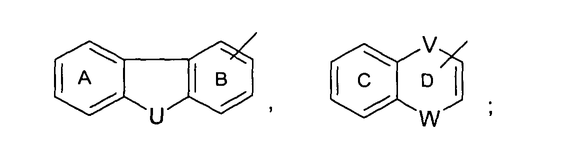 U is CH₂, O, S(O)_{y}, or NR¹⁰;
V and W are independently selected from a bond, O, S(O)_{y}, or NR¹⁰ ;
rings A, B, and C are optionally substituted with one to three groups selected from F, Cl, Br, I, OR²², OR²⁷, NR²³R²⁴, NHOH, NO₂, CN, CF₃, C₁-C₆ alkyl, phenyl, arylalkyl, and C(=O)R²²;
ring D is optionally substituted with one group selected from C₁-C₆ alkyl, and phenyl;
- Y is: C₁-C₆ alkylene;
C₁-C₄ alkylene-Z¹-(C₁-C₄ alkylene)ₙ;
C₁-C₄ alkylene-Z²-C₁-C₄ alkylene;
wherein said alkylene groups are optionally substituted with one to three R²⁰ groups;
- Z¹ is: CR²¹=CR²¹, C≡C, C₆-C₁₀ arylene, 5-10 membered heteroarylene,
C₃-C₆ cycloalkylene, or 3-6 membered heterocycloalkylene; wherein said arylene, heteroarylene, cycloalkylene, and heterocycloalkylene groups are optionally substituted with one to three R²⁰ groups;
- Z² is: O, NR^{10A}, or S(O)_{y};
- R¹ is: selected from NR²¹C(=O)R¹⁴, C(=O)R¹⁴, CO₂R¹¹, OC(=O)R¹¹, C(=O)NR¹²R¹³, C(=NR¹¹)NR¹²R¹³, OC(=O)NR¹²R¹³, NR²¹S(O)₂R¹¹, NR²¹C(=O)NR¹²R¹³, NR²¹S(O)₂NR¹²R¹³, and C(=O)NR¹¹ OR²²;

R¹⁰ and R^{10A} are each independently selected from H, C₁-C₆ alkyl, C(=O)R¹⁴, and S(O)_{y}R¹⁴; wherein said alkyl and aryl groups are optionally substituted with one to three R²⁰ groups;
R¹¹ at each occurrence is independently selected from H, and C₁-C₆ alkyl; wherein said alkyl and aryl groups are optionally substituted with one to three R²⁰ groups;
R¹² and R¹³ at each occurrence are each independently selected from H, and C₁-C₆ alkyl, and NR²³R²⁴, or R¹² and R¹³, together with the nitrogen to which they are attached, form a 3-7 membered heterocyclic ring;
   wherein said alkyl and aryl groups and heterocyclic ring are optionally substituted with one to three R²⁰ groups;
R¹⁴ at each occurrence is independently selected from C₁-C₆ alkyl and C₆-C₁₀ aryl; wherein said alkyl, aryl and alkylaryl groups are optionally substituted with one to three R²⁰ groups;
R²⁰ at each occurrence is independently selected from F, Cl, Br, I, OR²², OR²⁷, NR²³R²⁴, NHOH, NO₂, CN, CF₃, C₁-C₆ alkyl optionally substituted with OH, phenyl, =O, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
R²¹ at each occurrence is independently selected from H and C₁-C₆ alkyl;
R²² at each occurrence is independently selected from H, C₁-C₆ alkyl optionally substituted with OH, phenyl, and benzyl;
R²³ and R²⁴ at each occurrence are each independently selected from H, C₁-C₆ alkyl, and C₆-C₁₀ aryl, or R²³ and R²⁴, together with the nitrogen to which they are attached, form a 3-7 membered heterocyclic ring optionally substituted with =O;
R²⁵ and R²⁶ at each occurrence are each independently selected from H, C₁-C₆ alkyl, and phenyl, or R²⁵ and R²⁶, together with the carbon to which they are attached, form a 3-7 membered heterocyclic ring;
R²⁷ at each occurrence is independently the residue of an amino acid after the hydroxyl group of the carboxyl group is removed;
n is 0 or 1;
q is 0, 1, or 2;
y is 0, 1, or 2;
and the stereoisomeric forms, mixtures of stereoisomeric forms or pharmaceutically acceptable salts forms thereof.

An additional aspect of the present invention includes compounds of formula (A) and formulas (I) and (II) wherein Y is C₁-C₆ alkylene, C₁-C₄ alkylene-Z¹-C₁-C₄ alkylene, or C₁-C₄ alkylene-Z²-C₁-C₄ alkylene, wherein said alkylene groups are optionally substituted with one to three C₁-C₆ alkyl groups; Z¹ is CR²¹=CR²¹, C≡C, or phenyl; Z² is O, NR^{10A}, or S(O)_{y}; R¹ is selected from NR²¹C(=O)R¹⁴, C(=O)R¹⁴, CO₂R¹¹, OC(=O)R¹¹, and C(=O)NR¹²R¹³. In other aspects, Y is C₁-C₆ alkylene, or C₁-C₄ alkylene-Z¹-C₁-C₄ alkylene. In additional aspects, Y is C₁-C₆ alkylene. In further aspects, R¹ is C(=O)NR¹²R¹³.

In certain aspects of the present invention, there are included compounds of formula (A) and formulas (I) and (II) wherein Ar is dibenzofuranyl. Other aspects include compounds where Ar is dibenzothienyl. Other aspects include compounds where Ar is fluorenyl. Other aspects include compounds where Ar is phenylbenzofuranyl. Other aspects include compounds where Ar is phenylbenzothiophenyl. Other aspects include compounds where Ar is phenylindolyl. Other aspects include compounds where Ar is phenylbenzodioxinyl.

In additional aspects of the present invention, there are included compounds of formula (A) and formulas (I) and (II) wherein Ar has any of the values of the previous embodiments and q is 1.

Other aspects of the present invention include compounds of formula (A) and formulas (I) and (II) wherein Ar and q have any of the values of the previous embodiments, and Y is C₁-C₆ alkylene, particularly those where Y is CH₂ or CH₂CH₂, and most particularly those where Y is CH₂.

Additional aspects of the present invention include compounds of formula (A) and formulas (I) and (II) wherein Ar and q have any of the values of the previous embodiments, and Y is (C₁-C₄ alkylene)ₘ-Z¹-(C₁-C₄ alkylene)ₙ wherein Z¹ is CR²¹=CR²¹, C ≡C, C₆-C₁₀ arylene, 5-10 membered heteroarylene, C₃-C₆ cycloalkylene, or 3-6 membered heterocycloalkylene. Other aspects include those compounds where Y is C₁-C₄ alkylene-Z¹. Other aspects include those where Y is Z¹-C₁-C₄ alkylene. Additional aspects include compounds where Y is C₁-C₄ alkylene-Z¹-C₁-C₄ alkylene.

Further aspects of the present invention include compounds of formula (A) and formulas (I) and (II) wherein Ar, Y, and q have any of the values of the previous embodiments, and Z¹ is CR²¹=CR²¹, or C≡C. Other aspects include compounds where Z¹ is C₆-C₁₀ arylene, or C₃-C₆ cycloalkylene, particularly those where Z¹ is phenyl. Other aspects include compounds where Z¹ is 5-10 membered heteroarylene, or 3-6 membered heterocycloalkylene.

Further aspects of the present invention include compounds of formula (A) and formulas (I) and (II) wherein Ar and q have any of the values of the previous embodiments, and Y is (C₁-C₄ alkylene)ₘ-Z²-(C₁-C₄ alkylene)ₙ wherein Z² is O, NR^{10A}, or S(O)_{y}. Other aspects include those compounds where Y is C₁-C₄ alkylene-Z², wherein R¹ cannot be H. Other aspects include those compounds where Y is C₁-C₄ alkylene-Z²-C₁-C₄ alkylene. Additional aspects include any of the above embodiments of Y wherein Z² is O. Additional aspects include any of the above embodiments of Y wherein Z² is NR^{10A}.

Further aspects of the present invention include compounds of formula (A) and formulas (I) and (II) wherein Ar, Y, Z¹, and Z², and q have any of the values of the previous embodiments, and R¹ can be any value selected from the following 12 enumerated paragraphs:
- 1.: H.
- 2.: NR¹²R¹³.
- 3.: NR²¹C(=O)R¹⁴.
- 4.: C(=O)R¹⁴.
- 5.: CO₂R¹¹.
- 6.: OC(=O)R¹¹.
- 7.: C(=O)NR¹²R¹³.
- 8.: C(=NR¹¹)NR¹²R¹³.
- 9.: OC(=O)NR¹²R¹³.
- 10.: NR²¹S(O)₂R¹¹.
- 11.: NR²¹C(=O)NR¹²R¹³.
- 12.: NR²¹S(O)₂NR¹²R¹³.
- 13.: C(=O)NR¹¹OR²².

Other additional aspects of the present invention include compounds of formula (A) and formulas (I) and (II) wherein Ar, Y, Z¹, and Z², and q have any of the values of the previous embodiments, and R¹ can be a combination of the values selected from the previous 13 enumerated paragraphs. The preceding 13 enumerated paragraphs may be combined to further define additional preferred embodiments of compounds of the present invention. For example, one such combination includes NR¹²R¹³, NR²¹C(=O)R¹⁴, C(=O)R¹⁴, CO₂R¹¹, OC(=O)R¹¹, C(=O)NR¹²R¹³, C(=NR¹¹)NR¹²R¹³, OC(=O)NR¹²R¹³, NR²¹S(O)₂R¹¹, NR²¹C(=O)NR¹²R¹³, and NR²¹S(O)₂NR¹²R¹³, C(=O)NR¹¹ NR²³R²⁴, C(=O)NR¹¹ OR²², and C(=O)NR¹¹ OR²².

Another such combination includes NR¹²R¹³, wherein R¹² and R¹³ are each independently selected from H and C₁-C₆ alkyl; NR²¹C(=O)R¹⁴; C(=O)NR¹²R¹³; C(=NR¹¹)NR¹²R¹³, and NR²¹C(=O)NR¹²R¹³.

A third such combination includes C(=O)R¹⁴, CO₂R¹¹, OC(=O)R¹¹, C(=O)NR¹²R¹³, OC(=O)NR¹²R¹³, NR²¹S(O)₂R¹¹, and NR²¹S(O)₂NR¹²R¹³.

A fourth such combination includes NR²¹C(=O)R¹⁴, C(=O)R¹⁴, CO₂R¹¹, OC(=O)R¹¹, C(=O)NR¹²R¹³, and C(=O)NR¹¹ OR²².

A fifth such combination includes NR²¹C(=O)R¹⁴, and C(=O)NR¹²R¹³.

In still further aspects of the present invention, there are included compounds of formulas (III) and (IV): 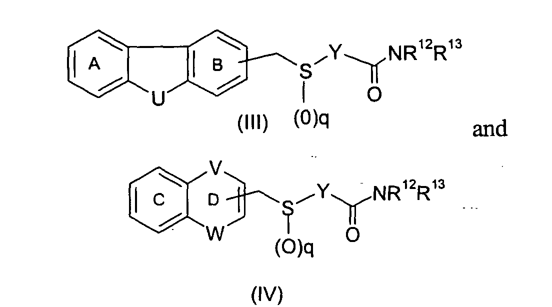 wherein U, Vand W have any of the values of the previous embodiments.

Additional aspects of the present invention include compounds of formula (A) and formulas (I) through (IV) wherein Ar, Y, Z¹, Z², R¹, and q have any of the values of the previous embodiments, and R¹² and R¹³ are each independently selected from H and C₁-C₆ alkyl.

Other aspects of the present invention include compounds of formula (A) and formulas (I) through (IV) wherein Ar, Y, Z¹, Z², R¹, and q have any of the values of the previous embodiments, and R¹² and R¹³ together with the nitrogen to which they are attached, form a 3-7 membered heterocyclic ring, particularly those where the heterocyclic ring is a heterocycloalkyl group, and more particularly those where the heterocyclic group is pyrrolidine or piperidine. In certain aspects, the heterocyclic ring is substituted with one R²⁰. In other aspects, the heterocyclic ring is unsubstituted.

In a preferred embodiment, Ar is dibenzofuranyl (U=O), dibenzothiophenyl (U=S) or fluorenyl (U=CH₂ or CR²⁵R²⁶).

Preferably, the ring A or B of such Ar groups is substituted with Cl, F or OR²² wherein R²² represents preferably a (C₁-C₆) alkyl group, such as a OCH₃ group.

In another preferred embodiment, Ar is benzofuranyl (W is a bond and V is O), benzothiophenyl (W is a bond and V is S), indol (W is a bond and V is N), or benzodioxinyl (W=V=O).

Preferably, the ring D of such Ar groups is substituted with a phenyl or Cl.

In accordance with a preferred embodiment, q is 0 or 1.

Preferably, Y is an unsubstituted (C₁-C₆) alkylene group, more preferably a CH₂ or CH₂CH₂ group and most preferably a CH₂ group.

In accordance with a preferred embodiment, R¹ is C(=O)NR¹²R¹³, wherein R¹² and R¹³ independently represents H, NR²³R²⁴, (C₁-C₆) alkyl, or form a 3-7-membered heterocyclic ring together with the nitrogen atom to which they are attached.

In accordance with a preferred embodiment, R¹² and/or R¹³ represent a NR²³R²⁴ wherein R²³ and R²⁴ together with the nitrogen to which they are attached, form a 3-7-membered heterocyclic ring, notably a 5-6-membered heterocyclic ring such as morpholine.

In accordance with another preferred embodiment, R¹² and/or R¹³ represent a (C₁-C₆) alkyl group selected from methyl, ethyl, propyl, i-propyl, optionally substituted with one to three R²⁰ groups.

In that context, preferred substituents of R¹² and/or R¹³ representing a (C₁-C₆) alkyl group are OR²² , NR²³R²⁴.

Examples of substituents OR²² are notably OH and O(C₁-C₆) alkyl optionally substituted by OH such as OCH₂CH₂OH.

Examples of substituents NR²³R²⁴ are those wherein R²³ and R²⁴ together with the nitrogen to which they are attached, form a 3-7-membered heterocyclic ring, notably a 5-6-membered heterocyclic ring such as pyrrolidine, piperazine or morpholine, the pyrrolidine being optionally substituted by =O.

In accordance with another preferred embodiment, R¹² and/or R¹³ form a 3-7-membered heterocyclic ring together with the nitrogen atom to which they are attached wherein the heterocyclic ring is preferably a 5-6-membered heterocyclic ring such as pyrrolidinyl, piperazinyl, piperidinyl or morpholinyl.

Heterocyclic ring may be substituted or unsubstituted.

Preferred heterocyclic ring substituents are OR²², =O, C(=O)R²², CO₂R²², C(=O)NR²³R²⁴, (C₁-C₆) alkyl optionally substituted with one to three OH.

Examples of substituents OR²² are notably OH.

Examples of substituents C(=O)R²² are notably those wherein R²² is H or a (C₁-C₆) alkyl group, such as C(=O)H or C(=O)CH₃.

Examples of substituents CO₂R²² are notably those wherein R²² is (C₁-C₆) alkyl or arylalkyl such as CO₂tBu, CO₂Et and CO₂CH₂Ph.

Examples of C(=O)NR²³R²⁴ substituents are notably those wherein R²³ and R²⁴ independently represent H or (C₁-C₆) alkyl such as C(=O)NH₂, C(=O)N(CH₃)₂, C(=O)NH(iPr), or those wherein R²³ and R²⁴, together with the nitrogen to which they are attached, form a 3-7-membered heterocyclic ring, notably a 5-6-membered heterocyclic ring such as pyrrolidine.

Examples of (C₁-C₆) alkyl groups substituted with one to three R²⁰ groups are notably methyl, ethyl, propyl, -CH₂CH₂CH₂OH.

In a preferred embodiment of the present invention there are provided compounds of formula (A) and formula (I): 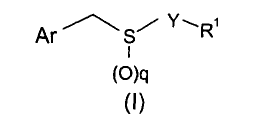 wherein Ar, q, Y-R¹ are defined in the table 1 below.

The positions on the Ar groups are numbered as follows:

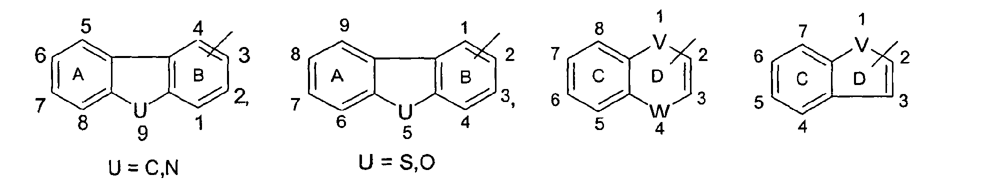

**Table 1**

| **Ex.n°** | **Ring A** | **q** | **Y-R**^{**1**} |
|---|---|---|---|
| 16 | Dibenzofuran-2-yl | 0 | CH₂CONH₂ |
| 36 | Dibenzofuran-2-yl | 1 | CH₂CONH₂ |
| | Dibenzofuran-2-yl | 0 | CH₂CON(CH₃)₂ |
| 54 | Dibenzofuran-2-yl | 1 | CH₂CON(CH₃)₂ |
| | Dibenzofuran-2-yl | 0 | CH₂CO-N-pyrrolidinyl |
| 55 | Dibenzofuran-2-yl | 1 | CH₂CO-N-pyrrolidinyl |
| | Dibenzofuran-2-yl | 0 | CH₂CONHCH(CH₃)₂ |
| 56 | Dibenzofuran-2-yl | 1 | CH₂CONHCH(CH₃)₂ |
| | Dibenzofuran-2-yl | 0 | CH₂CO-1-(4-tert-butoxycarbonyl)-piperazinyl |
| 57 | Dibenzofuran-2-yl | 1 | CH₂CO-1-piperazinyl |
| | Dibenzofuran-2-yl | 0 | CH₂CO-1-(4-acetyl)-piperazinyl |
| 58 | Dibenzofuran-2-yl | 1 | CH₂CO-1-(4-acetyl)-piperazinyl |
| | Dibenzofuran-2-yl | 0 | CH₂CONHCH₂CH₂OH |
| 59 | Dibenzofuran-2-yl | 1 | CH₂CONHCH₂CH₂OH |
| | Dibenzofuran-2-yl | 0 | CH₂CO-1-(4-hydroxy)piperidinyl |
| 60 | Dibenzofuran-2-yl | 1 | CH₂CO-1-(4-hydroxy)piperidinyl |
| | Dibenzofuran-2-yl | 0 | CH₂CONHCH₂CH₂OCH₂CH₂OH |
| 61 | Dibenzofuran-2-yl | 1 | CH₂CONHCH₂CH₂OCH₂CH₂OH |
| 15 | Dibenzofuran-2-yl | 0 | CH₂CO-1-[4-(2-hydroxyethyl)-piperazinyl] |
| 34 | Dibenzofuran-2-yl | 1 | CH₂CO-1-[4-(2-hydroxyethyl)-piperazinyl] |
| | Dibenzofuran-2-yl | 0 | CH₂CO-1-(4-formyl)-piperazinyl |
| 62 | Dibenzofuran-2-yl | 1 | CH₂CO-1-(4-formyl)-piperazinyl |
| 63 | Dibenzofuran-2-yl | 1 | CH₂CO-1-(4-tert-butoxycarbonyl)-piperazinyl |
| 35 | Dibenzofuran-2-yl | 1 | CH₂CO-1-(4-ethoxycarbonyl)-piperazinyl |
| 64 | Dibenzofuran-2-yl | 1 | CH₂CO-1-(4-methyl)-piperazinyl |
| | Dibenzofuran-2-yl | 0 | CH₂CO-1-(4-ethyl)-piperazinyl |
| 65 | Dibenzofuran-2-yl | 1 | CH₂CO-1-(4-ethyl)-piperazinyl |
| | Dibenzofuran-2-yl | 0 | CH₂CO-1-(4-propyl)-piperazinyl |
| 66 | Dibenzofuran-2-yl | 1 | CH₂CO-1-(4-propyl)-piperazinyl |
| | Dibenzofuran-2-yl | 0 | CH₂CON-morpholinyl |
| 67 | Dibenzofuran-2-yl | 1 | CH₂CON-morpholinyl |
| | Dibenzofuran-2-yl | 0 | CH₂CO-N-ethyl-N-(2-hydroxy-ethyl) |
| 68 | Dibenzofuran-2-yl | 1 | CH₂CO-N-ethyl-N-(2-hydroxy-ethyl) |
| | Dibenzofuran-2-yl | 0 | CH₂CONHN-morpholinyl |
| 69 | Dibenzofuran-2-yl | 1 | CH₂CONHN-morpholinyl |
| | Dibenzofuran-2-yl | 0 | CH₂CO-4-(2-oxo-piperazinyl) |
| 70 | Dibenzofuran-2-yl | 1 | CH₂CO-4-(2-oxo-piperazinyl) |
| 71 | Dibenzofuran-2-yl | 1 | CH₂CO-1-(4-isopropylaminocarbonyl)-piperazinyl |
| 72 | Dibenzofuran-2-yl | 1 | CH₂CO-1-(4-aminocarbonyl)-piperazinyl |
| 73 | Dibenzofuran-2-yl | 1 | CH₂CO-1-(4-pyrrolidinylcarbonyl)-piperazinyl |
| 74 | Dibenzofuran-2-yl | 1 | CH₂CO-1-(4-dimethylaminocarbonyl)-piperazinyl |
| 75 | Dibenzofuran-2-yl | 1 | CH₂CO-1-(4-benzyloxycarbonyl)-piperazinyl |
| | Dibenzofuran-2-yl | 0 | CH₂CH₂CONH₂ |
| 76 | Dibenzofuran-2-yl | 1 | CH₂CH₂CONH₂ |
| | Dibenzofuran-2-yl | 0 | CH₂CH₂CO-1-piperazinyl-N-Boc |
| 77 | Dibenzofuran-2-yl | 1 | CH₂CH₂CO-1-piperazinyl |
| 78 | Dibenzofuran-2-yl | 1 | CH₂CH₂CO-1-(4-acetyl)-piperazinyl |
| 79 | Dibenzofuran-2-yl | 1 | CH₂CON-[3-(2-oxo-pyrrolidin-1-yl)-propyl] |
| | Dibenzofuran-2-yl | 0 | CH₂CON-(2-pyrrolidin-1-yl-ethyl) |
| 80 | Dibenzofuran-2-yl | 1 | CH₂CON-(2-pyrrolidin-1-yl-ethyl) |
| | Dibenzofuran-2-yl | 0 | CH₂CON-(2-piperidin-1-yl-ethyl) |
| 81 | Dibenzofuran-2-yl | 1 | CH₂CON-(2-piperidin-1-yl-ethyl) |
| | Dibenzofuran-2-yl | 0 | CH₂CON-(2-morpholin-4-yl-ethyl) |
| 82 | Dibenzofuran-2-yl | 1 | CH₂CON-(2-morpholin-4-yl-ethyl |
| | Dibenzofuran-2-yl | 0 | H |
| 83 | Dibenzofuran-2-yl | 1 | H |
| | 6-Chloro-dibenzofuran-2-yl | 0 | CH₂CO-1-piperazinyl-N-Boc |
| 84 | 6-Chloro-dibenzofuran-2-yl | 1 | CH₂CO-1-piperazinyl |
| | 6-Chloro-dibenzofuran-2-yl | 0 | CH₂CO-1-(4-acetyl)-piperazinyl |
| 85 | 6-Chloro-dibenzofuran-2-yl | 1 | CH₂CO-1-(4-acetyl)-piperazinyl |
| 22 | 8-Chloro-dibenzofuran-2-yl | 0 | CH₂CO-1-piperazinyl-N-Boc |
| 41 | 8-Chloro-dibenzofuran-2-yl | 1 | CH₂CO-1-piperazinyl |
| | 8-Chloro-dibenzofuran-2-yl | 0 | CH₂CONH₂ |
| 86 | 8-Chloro-dibenzofuran-2-yl | 1 | CH₂CONH₂ |
| 23 | 8-Chloro-dibenzofuran-2-yl | 0 | CH₂CO-1-(4-acetyl)-piperazinyl |
| 42 | 8-Chloro-dibenzofuran-2-yl | 1 | CH₂CO-1-(4-acetyl)-piperazinyl |
| 146 | 8-Chloro-dibenzofuran-2-yl | 2 | CH₂CO-1-(4-acetyl)-piperazinyl |
| 21 | 8-Chloro-dibenzofuran-2-yl | 0 | CH₂CO-1-(4-hydroxyethyl)-piperazinyl |
| 40 | 8-Chloro-dibenzofuran-2-yl | 1 | CH₂CO-1-(4-hydroxyethyl)-piperazinyl |
| 17 | 8-Methoxy-dibenzofuran-2-yl | 0 | CH₂CONH₂ |
| 37 | 8-Methoxy-dibenzofuran-2-yl | 1 | CH₂CONH₂ |
| | 8-Methoxy-dibenzofuran-2-yl | 0 | CH₂CO-1-piperazinyl-N-Boc |
| 87 | 8-Methoxy-dibenzofuran-2-yl | 1 | CH₂CO-1-piperazinyl |
| | 8-Methoxy-dibenzofuran-2-yl | 0 | CH₂CO-1-(4-acetyl)-piperazinyl |
| 88 | 8-Methoxy-dibenzofuran-2-yl | 1 | CH₂CO-1-(4-acetyl)-piperazinyl |
| 26 | 8-Fluoro-dibenzofuran-2-yl | 0 | CH₂CO-1-piperazinyl-N-Boc |
| 45 | 8-Fluoro-dibenzofuran-2-yl | 1 | CH₂CO-1-piperazinyl |
| | 8-Fluoro-dibenzofuran-2-yl | 0 | CH₂CO-1-(4-acetyl)-piperazinyl |
| 89 | 8-Fluoro-dibenzofuran-2-yl | 1 | CH₂CO-1-(4-acetyl)-piperazinyl |
| 19 | 8-Fluoro-dibenzofuran-2-yl | 0 | CH₂CONH₂ |
| 38 | 8-Fluoro-dibenzofuran-2-yl | 1 | CH₂CONH₂ |
| | 4-Chloro-dibenzofuran-2-yl | 0 | CH₂CONH₂ |
| 90 | 4-Chloro-dibenzofuran-2-yl | 1 | CH₂CONH₂ |
| | 4-Fluoro-dibenzofuran-2-yl | 0 | CH₂CONH₂ |
| 91 | 4-Fluoro-dibenzofuran-2-yl | 1 | CH₂CONH₂ |
| | 4-Chloro-dibenzofuran-2-yl | 0 | CH₂CO-1-(4-acetyl)-piperazinyl |
| 92 | 4-Chloro-dibenzofuran-2-yl | 1 | CH₂CO-1-(4-acetyl)-piperazinyl |
| | 4-Fluoro-dibenzofuran-2-yl | 0 | CH₂CO-1-(4-acetyl)-piperazinyl |
| 93 | 4-Fluoro-dibenzofuran-2-yl | 1 | CH₂CO-1-(4-acetyl)-piperazinyl |
| | 4-Fluoro-8-chloro-dibenzofuran-2-yl | 0 | CH₂CONH₂ |
| 94 | 4-Fluoro-8-chloro-dibenzofuran-2-yl | 1 | CH₂CONH₂ |
| | Dibenzofuran-4-yl | 0 | CH₂CONHCH₂CH₂OH |
| 95 | Dibenzofuran-4-yl | 1 | CH₂CONHCH₂CH₂OH |
| | Dibenzofuran-4-yl | 0 | CH₂CONH₂ |
| 96 | Dibenzofuran-4-yl | 1 | CH₂CONH₂ |
| | Dibenzofuran-4-yl | 0 | CH₂CO-1-(4-acetyl)-piperazinyl |
| 97 | Dibenzofuran-4-yl | 1 | CH₂CO-1-(4-acetyl)-piperazinyl |
| | Dibenzofuran-4-yl | 0 | CH₂CO-1-(4-hydroxy)piperidinyl |
| 98 | Dibenzofuran-4-yl | 1 | CH₂CO-1-(4-hydroxy)piperidinyl |
| | Dibenzofuran-4-yl | 0 | CH₂CO-1-piperazinyl-N-Boc |
| 99 | Dibenzofuran-4-yl | 1 | CH₂CO-1-piperazinyl |
| | Dibenzofuran-4-yl | 0 | CH₂CON-(2-pyrrolidin-1-yl-ethyl) |
| 100 | Dibenzofuran-4-yl | 1 | CH₂CON-(2-pyrrolidin-1-yl-ethyl) |
| | Dibenzofuran-4-yl | 0 | CH₂CON-[3-(2-oxo-pyrrolidin-1-yl)-propyl] |
| 101 | Dibenzofuran-4-yl | 1 | CH₂CON-[3-(2-oxo-pyrrolidin-1-yl)-propyl] |
| | Dibenzofuran-4-yl | 0 | CH₂CON-(2-piperidin-1-yl-ethyl) |
| 102 | Dibenzofuran-4-yl | 1 | CH₂CON-(2-piperidin-1-yl-ethyl) |
| | Dibenzofuran-4-yl | 0 | CH₂CON-(2-morpholin-4-yl-ethyl) |
| 103 | Dibenzofuran-4-yl | 1 | CH₂CON-(2-morpholin-4-yl-ethyl) |
| | Dibenzofuran-4-yl | 0 | CH₂CO-1-(4-hydroxyethyl)-piperazinyl |
| 104 | Dibenzofuran-4-yl | 1 | CH₂CO-1-(4-hydroxyethyl)-piperazinyl |
| | Dibenzofuran-3-yl | 0 | CH₂CO-1-piperazinyl-N-Boc |
| 105 | Dibenzofuran-3-yl | 1 | CH₂CO-1-piperazinyl |
| | Dibenzofuran-1-yl | 0 | CH₂CO-1-piperazinyl-N-Boc |
| 106 | Dibenzofuran-1-yl | 1 | CH₂CO-1-piperazinyl |
| | Dibenzofuran-3-yl | 0 | CH₂CO-1-(4-acetyl)-piperazinyl |
| 107 | Dibenzofuran-3-yl | 1 | CH₂CO-1-(4-acetyl)-piperazinyl |
| 25 | Dibenzothiophen-2-yl | 0 | CH₂CO-1-piperazinyl-N-Boc |
| 44 | Dibenzothiophen-2-yl | 1 | CH₂CO-1-piperazinyl |
| | Dibenzothiophen-2-yl | 0 | CH₂CO-1-(4-acetyl)-piperazinyl |
| 108 | Dibenzothiophen-2-yl | 1 | CH₂CO-1-(4-acetyl)-piperazinyl |
| | Dibenzothiophen-2-yl | 0 | CH₂CO-1-(4-ethoxycarbonyl)-piperazinyl |
| 109 | Dibenzothiophen-2-yl | 1 | CH₂CO-1-(4-ethoxycarbonyl)-piperazinyl |
| 18 | Dibenzothiophen-2-yl | 0 | CH₂CONH₂ |
| 39 | Dibenzothiophen-2-yl | 1 | CH₂CONH₂ |
| | Dibenzothiophen-4-yl | 0 | CH₂CO-1-(4-acetyl)-piperazinyl |
| 110 | Dibenzothiophen-4-yl | 1 | CH₂CO-1-(4-acetyl)-piperazinyl |
| | Fluoren-1-yl | 0 | CH₂CONHCH₂CH₂OH |
| 111 | Fluoren-1-yl | 1 | CH₂CONHCH₂CH₂OH |
| | Fluoren-1-yl | 0 | CH₂CO-1-(4-acetyl)-piperazinyl |
| 112 | Fluoren-1-yl | 1 | CH₂CO-1-(4-acetyl)-piperazinyl |
| | Fluoren-1-yl | 0 | CH₂CO-1-(4-hydroxy)piperidinyl |
| 113 | Fluoren-1-yl | 1 | CH₂CO-1-(4-hydroxy)piperidinyl |
| | Fluoren-1-yl | 0 | CH₂CONH₂ |
| 114 | Fluoren-1-yl | 1 | CH₂CONH₂ |
| | Fluoren-1-yl | 0 | CH₂CO-1-(4-hydroxyethyl)-piperazinyl |
| 115 | Fluoren-1-yl | 1 | CH₂CO-1-(4-hydroxyethyl)-piperazinyl |
| | Fluoren-1-yl | 0 | CH₂CO-1-piperazinyl-N-Boc |
| 116 | Fluoren-1-yl | 1 | CH₂CO-1-piperazinyl |
| | Fluoren-2-yl | 0 | CH₂CONH₂ |
| 117 | Fluoren-2-yl | 1 | CH₂CONH₂ |
| | Fluoren-2-yl | 0 | CH₂CON(CH₃)₂ |
| 118 | Fluoren-2-yl | 1 | CH₂CON(CH₃)₂ |
| | Fluoren-2-yl | 0 | CH₂CO-N-pyrrolidinyl |
| 119 | Fluoren-2-yl | 1 | CH₂CO-N-pyrrolidinyl |
| | Fluoren-2-yl | 0 | CH₂CONHCH(CH₃)₂ |
| 120 | Fluoren-2-yl | 1 | CH₂CONHCH(CH₃)₂ |
| | Fluoren-2-yl | 0 | CH₂CONHCH₂CH₂OH |
| 121 | Fluoren-2-yl | 1 | CH₂CONHCH₂CH₂OH |
| | Fluoren-2-yl | 0 | CH₂CO-1-(4-hydroxy)piperidinyl |
| 122 | Fluoren-2-yl | 1 | CH₂CO-1-(4-hydroxy)piperidinyl |
| | Fluoren-2-yl | 0 | CH₂CO-1-(4-acetyl)-piperazinyl |
| 123 | Fluoren-2-yl | 1 | CH₂CO-1-(4-acetyl)-piperazinyl |
| | Fluoren-4-yl | 0 | CH₂CONH₂ |
| 124 | Fluoren-4-yl | 1 | CH₂CONH₂ |
| | Fluoren-4-yl | 0 | CH₂CON(CH₃)₂ |
| 125 | Fluoren-4-yl | 1 | CH₂CON(CH₃)₂ |
| | Fluoren-4-yl | 0 | CH₂CONHCH(CH₃)₂ |
| 126 | Fluoren-4-yl | 1 | CH₂CONHCH(CH₃)₂ |
| | Fluoren-4-yl | 0 | CH₂CONHCH₂CH₂OH |
| 127 | Fluoren-4-yl | 1 | CH₂CONHCH₂CH₂OH |
| | Fluoren-4-yl | 0 | CH₂CO-1-(4-hydroxy)piperidinyl |
| 128 | Fluoren-4-yl | 1 | CH₂CO-1-(4-hydroxy)piperidinyl |
| | Fluoren-4-yl | 0 | CH₂CO-1-(4-acetyl)-piperazinyl |
| 129 | Fluoren-4-yl | 1 | CH₂CO-1-(4-acetyl)-piperazinyl |
| | Fluoren-4-yl | 0 | CH₂CO-1-piperazinyl-N-Boc |
| 130 | Fluoren-4-yl | 1 | CH₂CO-1-piperazinyl |
| 24 | Fluoren-4-yl | 0 | CH₂CO-1-(4-hydroxyethyl)-piperazinyl |
| 43 | Fluoren-4-yl | 1 | CH₂CO-1-(4-hydroxyethyl)-piperazinyl |
| | Fluoren-4-yl | 0 | CH₂CO-1-(4-formyl)-piperazinyl |
| 131 | Fluoren-4-yl | 1 | CH₂CO-1-(4-formyl)-piperazinyl |
| | 2-Phenylbenzofuran-3-yl | 0 | CH₂CONH₂ |
| 132 | 2-Phenylbenzofuran-3-yl | 1 | CH₂CONH₂ |
| | 2-Phenylbenzofuran-3-yl | 0 | CH₂CO-N-pyrrolidinyl |
| 133 | 2-Phenylbenzofuran-3-yl | 1 | CH₂CO-N-pyrrolidinyl |
| | 2-Phenybenzofuran-3-yl | 0 | CH₂CH₂CONH₂ |
| 134 | 2-Phenylbenzofuran-3-yl | 1 | CH₂CH₂CONH₂ |
| | 2-Phenylbenzofuran-3-yl | 0 | CH₂CH₂CO-N-pyrrolidinyl |
| 135 | 2-Phenylbenzofuran-3-yl | 1 | CH₂CH₂CO-N-pyrrolidinyl |
| 20 | 2-Phenylbenzofuran-3-yl | 0 | CH₂CON(CH₃)₂ |
| 46 | 2-Phenylbenzofuran-3-yl | 1 | CH₂CON(CH₃)₂ |
| | 2-Phenylbenzofuran-3-yl | 0 | CH₂CH₂CON(CH₃)₂ |
| 136 | 2-Phenylbenzofuran-3-yl | 1 | CH₂CH₂CON(CH₃)₂ |
| | 2-Phenylbenzofuran-3-yl | 0 | CH₂CONHCH(CH₃)₂ |
| 137 | 2-Phenylbenzofuran-3-yl | 1 | CH₂CONHCH(CH₃)₂ |
| | 2-Phenylbenzofuran-3-yl | 0 | CH₂CH₂CONHCH(CH₃)₂ |
| 138 | 2-Phenylbenzofuran-3-yl | 1 | CH₂CH₂CONHCH(CH₃)₂ |
| | 2-Phenylbenzofuran-3-yl | 0 | CH₂CO-1-(4-acetyl)-piperazinyl |
| 139 | 2-Phenylbenzofuran-3-yl | 1 | CH₂CO-1-(4-acetyl)-piperazinyl |
| | 2-Phenylbenzofuran-3-yl | 0 | CH₂CH₂CO-1-(4-acetyl)-piperazinyl |
| 140 | 2-Phenylbenzofuran-3-yl | 1 | CH₂CH₂CO-1-(4-acetyl)-piperazinyl |
| | 3-Phenylbenzothiophen-2-yl | 0 | CH₂CONH₂ |
| 141 | 3-Phenylbenzothiophen-2-yl | 1 | CH₂CONH₂ |
| 27 | 3-phenylbenzothiophen-2-yl | 0 | CH2-CO-*N*-pyrrolidinyl |
| 28 | 3-phenylbenzothiophen-2-yl | 0 | CH2-CON(CH₃)₂ |
| 29 | 3-phenylbenzothiophen-2-yl | 0 | CH2-CONHCH(CH₃)₂ |
| 30 | 3-phenylbenzothiophen-2-yl | 0 | CH2-CO-1-(4-hydroxy)-piperidinyl |
| 31 | 3-phenylbenzothiophen-2-yl | 0 | CH2-CO-1-(4-acetyl)-piperazinyl |
| 32 | 3-phenylbenzothiophen-2-yl | 0 | CH2-CONH(CH₂)₂OH |
| 47 | 3-phenylbenzothiophen-2-yl | 1 | CH2-CO-*N*-pyrrolidinyl |
| 48 | 3-phenylbenzothiophen-2-yl | 1 | CH2-CON(CH₃)₂ |
| 49 | 3-phenylbenzothiophen-2-yl | 1 | CH2-CONHCH(CH₃)₂ |
| 50 | 3-phenylbenzothiophen-2-yl | 1 | CH2-CO-1-(4-hydroxy)-piperidinyl |
| 51 | 3-phenylbenzothiophen-2-yl | 1 | CH2-CO-1-(4-acetyl)-piperazinyl |
| 52 | 3-phenylbenzothiophen-2-yl | 1 | CH2-CONH(CH₂)₂OH |
| 33 | 3-phenyl-1,4-benzodioxin-2-yl | 0 | CH₂CO-1-(4-acetyl)-piperazinyl |
| 53 | 3-phenyl-1,4-benzodioxin-2-yl | 1 | CH₂CO-1-(4-acetyl)-piperazinyl |
| | 3-phenyl-1,4-benzodioxin-2-yl | 0 | CH₂CONHCH(CH₃)₂ |
| 142 | 3-phenyl-1,4-benzodioxin-2-yl | 1 | CH₂CONHCH(CH₃)₂ |
| | 3-phenyl-1,4-benzodioxin-2-yl | 0 | CH₂CONH₂ |
| 143 | 3-phenyl-1,4-benzodioxin-2-yl | 1 | CH₂CONH₂ |
| | 6,7-dichloro-3-phenyl-1,4-benzodioxin-2-yl | 0 | CH₂CONH₂ |
| 144 | 6,7-dichloro-3-phenyl-1,4-benzodioxin-2-yl | 1 | CH₂CONH₂ |
| | 6,7-dichloro-3-phenyl-1,4-benzodioxin-2-yl | 0 | CH₂CO-1-(4-acetyl)-piperazinyl |
| 145 | 6,7-dichloro-3-phenyl-1,4-benzodioxin-2-yl | 1 | CH₂CO-1-(4-acetyl)-piperazinyl |
| 148 | 3-phenyl-1H-indol-2-yl | 0 | CH₂CONH₂ |
| 149 | 3-phenyl-1H-indol-2-yl | 1 | CH₂CONH₂ |

In a second embodiment, the present invention provides a method for treatment of diseases comprising administering to a subject in need thereof a therapeutically effective amount of a compound of formula (A) and formula (I), or a pharmaceutically acceptable salt thereof. In a preferred embodiment, the present invention is to provide methods of treating or preventing diseases or disorders, including treatment of sleepiness, promotion and/or improvement of wakefulness, preferably improvement of wakefulness in patients with excessive sleepiness associated with narcolepsy, sleep apnea, preferably obstructive sleep apnea/hypopnea, and shift work disorder ; treatment of Parkinson's disease ; Alzheimer's disease ; cerebral ischemia ; stroke ; eating disorders ; attention deficit disorder ("ADD"), attention deficit hyperactivity disorder ("ADHD") ; depression ; schizophrenia ; fatigue, preferably fatigue associated with cancer or neurological diseases, such as multiple sclerosis and chronic fatigue syndrome ; stimulation of appetite and weight gain and improvement of cognitive dysfunction.

In a third embodiment, the present invention provides a pharmaceutical compositions comprising the compounds of formula (A) and formula (I) wherein the compositions comprise one or more pharmaceutically acceptable excipients and a therapeutically effective amount of at least one of the compounds of the present invention, or a pharmaceutically acceptable salt or ester form thereof.

In a fourth embodiment, the present invention provides for the use of compounds of formula (A) and formula (I) or pharmaceutically acceptable salts thereof for the manufacture of a medicament for the treatment of a disease or disorder.

These and other objects, features and advantages of the benzyl-thioalkyl derivatives will be disclosed in the following detailed description of the patent disclosure.

### Definitions

The following terms and expressions contained herein are defined as follows:

As used herein, the term "about" refers to a range of values from ± 10% of a specified value. For example, the phrase "about 50 mg" includes ± 10% of 50, or from 45 to 55 mg.

As used herein, a range of values in the form "x-y" or "x to y", or "x through y", include integers x, y, and the integers therebetween. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

As used herein "stable compound" or "stable structure" refers to a compound that is sufficiently robust to survive isolation to a useful degree of purity from a reaction mixture, and preferably capable of formulation into an efficacious therapeutic agent. The present invention is directed only to stable compounds.

As used herein, the term "alkyl" refers to a straight-chain, or branched alkyl group having 1 to 8 carbon atoms, such as methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl, octyl, etc. The alkyl moiety of alkyl-containing groups, such as alkoxy, alkoxycarbonyl, and alkylaminocarbonyl groups, has the same meaning as alkyl defined above. Lower alkyl groups, which are preferred, are alkyl groups as defined above which contain 1 to 4 carbons. A designation such as "C₁-C₄ alkyl" refers to an alkyl radical containing from 1 to 4 carbon atoms.

As used herein, the term "alkenyl" refers to a straight chain, or branched hydrocarbon chains of 2 to 8 carbon atoms having at least one carbon-carbon double bond. A designation "C₂-C₈ alkenyl" refers to an alkenyl radical containing from 2 to 8 carbon atoms. Examples of alkenyl groups include ethenyl, propenyl, isopropenyl, 2,4-pentadienyl, etc.

As used herein, the term "alkynyl" refers to a straight chain, or branched hydrocarbon chains of 2 to 8 carbon atoms having at least one carbon-carbon triple bond. A designation "C₂-C₈ alkynyl" refers to an alkynyl radical containing from 2 to 8 carbon atoms. Examples include ethynyl, propynyl, isopropynyl, 3,5-hexadiynyl, etc.

As used herein, the term "alkylene" refers to a substituted or unsubstituted, branched or straight chained hydrocarbon of 1 to 8 carbon atoms, which is formed by the removal of two hydrogen atoms. A designation such as "C₁-C₄ alkylene" refers to an alkylene radical containing from 1 to 4 carbon atoms. Examples include methylene (-CH₂-), propylidene (CH₃CH₂CH=), 1,2-ethandiyl (-CH₂CH₂-), etc.

As used herein, the term "phenylene" refers to a phenyl group with an additional hydrogen atom removed, i.e. a moiety with the structure of:

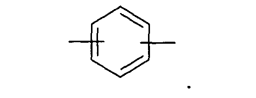

As used herein, the terms "carbocycle", "carbocyclic" or "carbocyclyl" refer to a substituted or unsubstituted, stable monocyclic or bicyclic hydrocarbon ring system which is saturated, partially saturated or unsaturated, and contains from 3 to 10 ring carbon atoms. Accordingly the carbocyclic group may be aromatic or non-aromatic, and includes the cycloalkyl and aryl compounds defined herein. The bonds connecting the endocyclic carbon atoms of a carbocyclic group may be single, double, triple, or part of a fused aromatic moiety.

As used herein, the term "cycloalkyl" refers to a saturated or partially saturated mono- or bicyclic alkyl ring system containing 3 to 10 carbon atoms. A designation such as "C₅-C₇ cycloalkyl" refers to a cycloalkyl radical containing from 5 to 7 ring carbon atoms. Preferred cycloalkyl groups include those containing 5 or 6 ring carbon atoms. Examples of cycloalkyl groups include such groups as cyclopropyl, cyclobutyl, cyclopentyl, cyclohexl, cycloheptyl, cyclooctyl, pinenyl, and adamantanyl.

As used herein, the term "aryl" refers to a substituted or unsubstituted, mono- or bicyclic hydrocarbon aromatic ring system having 6 to 12 ring carbon atoms. Examples include phenyl and naphthyl. Preferred aryl groups include unsubstituted or substituted phenyl and naphthyl groups. Included within the definition of "aryl" are fused ring systems, including, for example, ring systems in which an aromatic ring is fused to a cycloalkyl ring. Examples of such fused ring systems include, for example, indane, indene, and tetrahydronaphthalene.

As used herein, the terms "heterocycle", "heterocyclic" or "heterocyclyl" refer to a substituted or unsubstituted carbocyclic group in which the ring portion includes at least one heteroatom such as O, N, or S. The nitrogen and sulfur heteroatoms may be optionally oxidized, and the nitrogen may be optionally substituted in non-aromatic rings. Heterocycles are intended to include heteroaryl and heterocycloalkyl groups.

As used herein, the term "heterocycloalkyl" refers to a cycloalkyl group in which one or more ring carbon atoms are replaced by at least one hetero atom such as -O-, -N-, or -S-. Examples of heterocycloalkyl groups include pyrrolidinyl, pyrrolinyl, imidazolidinyl, imidazolinyl, pirazolidinyl, pirazolinyl, pyrazalinyl, piperidyl, piperazinyl, morpholinyl, thiomorpholinyl, tetrahydrofuranyl, dithiolyl, oxathiolyl, dioxazolyl, oxathiazolyl, pyranyl, oxazinyl, oxathiazinyl, and oxadiazinyl.

As used herein, the term "heteroaryl" refers to an aromatic group containing 5 to 10 ring carbon atoms in which one or more ring carbon atoms are replaced by at least one hetero atom such as -O-, -N-, or -S-. Examples of heteroaryl groups include pyrrolyl, furanyl, thienyl, pyrazolyl, imidazolyl, thiazolyl, isothiazolyl, isoxazolyl, oxazolyl, oxathiolyl, oxadiazolyl, triazolyl, oxatriazolyl, furazanyl, tetrazolyl, pyridyl, pyrazinyl, pyrimidinyl, pyridazinyl, triazinyl, indolyl, isoindolyl, indazolyl, benzofuranyl, isobenzofuranyl, purinyl, quinazolinyl, quinolyl, isoquinolyl, benzoimidazolyl, benzothiazolyl, benzothiophenyl, thianaphthenyl, benzoxazolyl, benzisoxazolyl, cinnolinyl, phthalazinyl, naphthyridinyl, and quinoxalinyl. Included within the definition of "heteroaryl" are fused ring systems, including, for example, ring systems in which an aromatic ring is fused to a heterocycloalkyl ring. Examples of such fused ring systems include, for example, phthalamide, phthalic anhydride, indoline, isoindoline, tetrahydroisoquinoline, chroman, isochroman, chromene, and isochromene.

As used herein, the term "arylalkyl" refers to an alkyl group that is substituted with an aryl group. Examples of arylalkyl groups include, but are not limited to, benzyl, bromobenzyl, phenethyl, benzhydryl, diphenylmethyl, triphenylmethyl, diphenylethyl, naphthylmethyl, etc.

As used herein, the term "amino acid" refers to a group containing both an amino group and a carboxyl group. Embodiments of amino acids include α-amino, β-amino, γ-amino acids. The α-amino acids have a general formula HOOC-CH(side chain)-NH2. In certain embodiments, substituent groups for the compounds of the present invention include the residue of an amino acid after removal of the hydroxyl moiety of the carboxyl group thereof; i.e., groups of formula -C(=O)CH(NH2)-(side chain). The amino acids can be in their D, L or racemic configurations. Amino acids include naturally-occurring and non-naturally occurring moieties. The naturally-occurring amino acids include the standard 20 α-amino acids found in proteins, such as glycine, serine, tyrosine, proline, histidine, glutamine, etc. Naturally-occurring amino acids can also include non-α-amino acids (such as β-alanine, γ-aminobutyric acid, homocysteine, etc.), rare amino acids (such as 4-hydroxyproline, 5-hydroxylysine, 3-methylhistidine, etc.) and non-protein amino acids (such as citrulline, ornithine, canavanine, etc.). Non-naturally occurring amino acids are well-known in the art, and include analogs of natural amino acids. See Lehninger, A. L. Biochemistry, 2nd ed.; Worth Publishers: New York, 1975; 71-77, the disclosure of which is incorporated herein by reference. Non-naturally occurring amino acids also include α-amino acids wherein the side chains are replaced with synthetic derivatives.

Representative side chains of naturally occurring and non-naturally occurring α-amino acids are shown below in Table A.

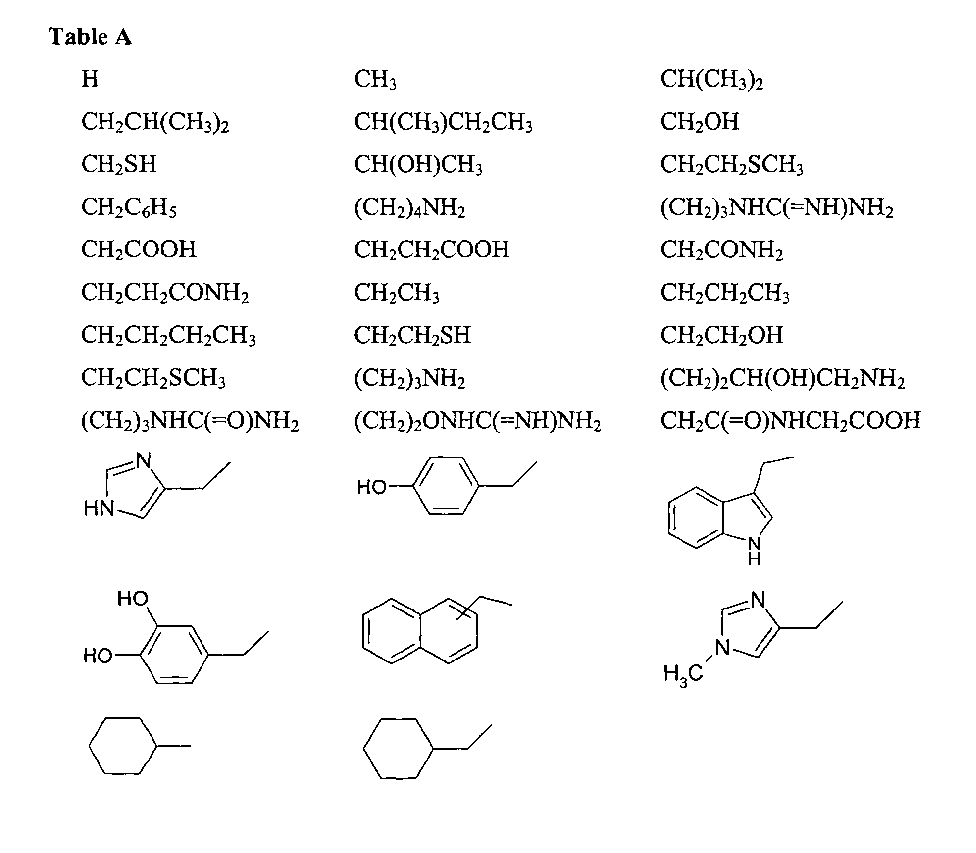

As used herein, the term "subject" refers to a warm blooded animal such as a mammal, preferably a human, or a human child, which is afflicted with, or has the potential to be afflicted with one or more diseases and conditions described herein.

As used herein, a "therapeutically effective amount" refers to an amount of a compound of the present invention effective to prevent or treat the symptoms of particular disorder. Such disorders include, but are not limited to, those pathological and neurological disorders associated with the aberrant activity of the receptors described herein, wherein the treatment or prevention comprises inhibiting, inducing, or enhancing the activity thereof by contacting the receptor with a compound of the present invention.

As used herein, the term "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

As used herein, the term "unit dose" refers to a single dose which is capable of being administered to a patient, and which can be readily handled and packaged, remaining as a physically and chemically stable unit dose comprising either the active compound itself, or as a pharmaceutically acceptable composition, as described hereinafter.

All other terms used in the description of the present invention have their meanings as is well known in the art.

In another aspect, the present invention is directed to pharmaceutically acceptable salts of the compounds described above. As used herein, "pharmaceutically acceptable salts" includes salts of compounds of the present invention derived from the combination of such compounds with non-toxic acid or base addition salts.

Acid addition salts include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, para-toluenesulfonic, succinic, and benzoic acid, and related inorganic and organic acids.

Base addition salts include those derived from inorganic bases such as ammonium and alkali and alkaline earth metal hydroxides, carbonates, bicarbonates, and the like, as well as salts derived from basic organic amines such as aliphatic and aromatic amines, aliphatic diamines, hydroxy alkamines, and the like. Such bases useful in preparing the salts of this invention thus include ammonium hydroxide, potassium carbonate, sodium bicarbonate, calcium hydroxide, methylamine, diethylamine, ethylenediamine, cyclohexylamine, ethanolamine and the like.

In addition to pharmaceutically-acceptable salts, other salts are included in the invention. They may serve as intermediates in the purification of the compounds, in the preparation of other salts, or in the identification and characterization of the compounds or intermediates.

The pharmaceutically acceptable salts of compounds of the present invention can also exist as various solvates, such as with water, methanol, ethanol, dimethylformamide, ethyl acetate and the like. Mixtures of such solvates can also be prepared. The source of such solvate can be from the solvent of crystallization, inherent in the solvent of preparation or crystallization, or adventitious to such solvent. Such solvates are within the scope of the present invention.

The present invention also encompasses the pharmaceutically acceptable prodrugs of the compounds disclosed herein. As used herein, "prodrug" is intended to include any compounds which are converted by metabolic processes within the body of a subject to an active agent that has a formula within the scope of the present invention. Since prodrugs are known to enhance numerous desirable qualities of pharmaceuticals (e.g., solubility, bioavailability, manufacturing, etc.) the compounds of the present invention may be delivered in prodrug form. Conventional procedures for the selection and preparation of suitable prodrug derivatives are described, for example, in *Prodrugs,* Sloane, K. B., Ed.; Marcel Dekker: New York, 1992, incorporated by reference herein in its entirety

It is recognized that compounds of the present invention may exist in various stereoisomeric forms. As such, the compounds of the present invention include both diastereomers and enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. *Stereochemistry of Organic Compounds*; Wiley: New York, 1994, and Jacques, J, et al. *Enantiomers, Racemates, and Resolutions*; Wiley: New York, 1981, each incorporated by reference herein in their entireties.

It is further recognized that functional groups present on the compounds of Formula I may contain protecting groups. For example, the amino acid side chain substituents of the compounds of Formula I can be substituted with protecting groups such as benzyloxycarbonyl or t-butoxycarbonyl groups. Protecting groups are known per se as chemical functional groups that can be selectively appended to and removed from functionalities, such as hydroxyl groups and carboxyl groups. These groups are present in a chemical compound to render such functionality inert to chemical reaction conditions to which the compound is exposed. Any of a variety of protecting groups may be employed with the present invention. Preferred protecting groups include the benzyloxycarbonyl (Cbz; Z) group and the tert-butyloxycarbonyl (Boc) group. Other preferred protecting groups according to the invention may be found in Greene, T.W. and Wuts, P.G.M., *Protective Groups in Organic Synthesis,* 2d. Ed., Wiley & Sons, 1991.

### Synthesis

The compounds of the present invention may be prepared in a number of methods well known to those skilled in the art, including, but not limited to those described below, or through modifications of these methods by applying standard techniques known to those skilled in the art of organic synthesis. All processes disclosed in association with the present invention are contemplated to be practiced on any scale, including milligram, gram, multigram, kilogram, multikilogram or commercial industrial scale.

It will be appreciated that the compounds of the present invention may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well known in the art how to prepare such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from active starting materials or by deliberate chiral synthesis of target centers.

As will be readily understood, functional groups present on the compounds of Formula I may contain protecting groups. For example, the amino acid side chain substituents of the compounds of Formula I can be substituted with protecting groups such as benzyloxycarbonyl or t-butoxycarbonyl groups. Protecting groups are known per se as chemical functional groups that can be selectively appended to and removed from functionalities, such as hydroxyl groups and carboxyl groups. These groups are present in a chemical compound to render such functionality inert to chemical reaction conditions to which the compound is exposed. Any of a variety of protecting groups may be employed with the present invention. Preferred protecting groups include the benzyloxycarbonyl (Cbz; Z) group and the tert-butyloxycarbonyl (Boc) group. Other preferred protecting groups according to the invention may be found in Greene, T.W. and Wuts, P.G.M., "Protective Groups in Organic Synthesis" 2d. Ed., Wiley & Sons, 1991.

The general routes to prepare the examples shown in Table 1 of the present invention are shown in **scheme A and in scheme B**. The reagents and starting materials are commercially available, or readily synthesized by well-known techniques by one of ordinary skill in the arts. All substituents in the synthetic Schemes, unless otherwise indicated, are as previously defined.

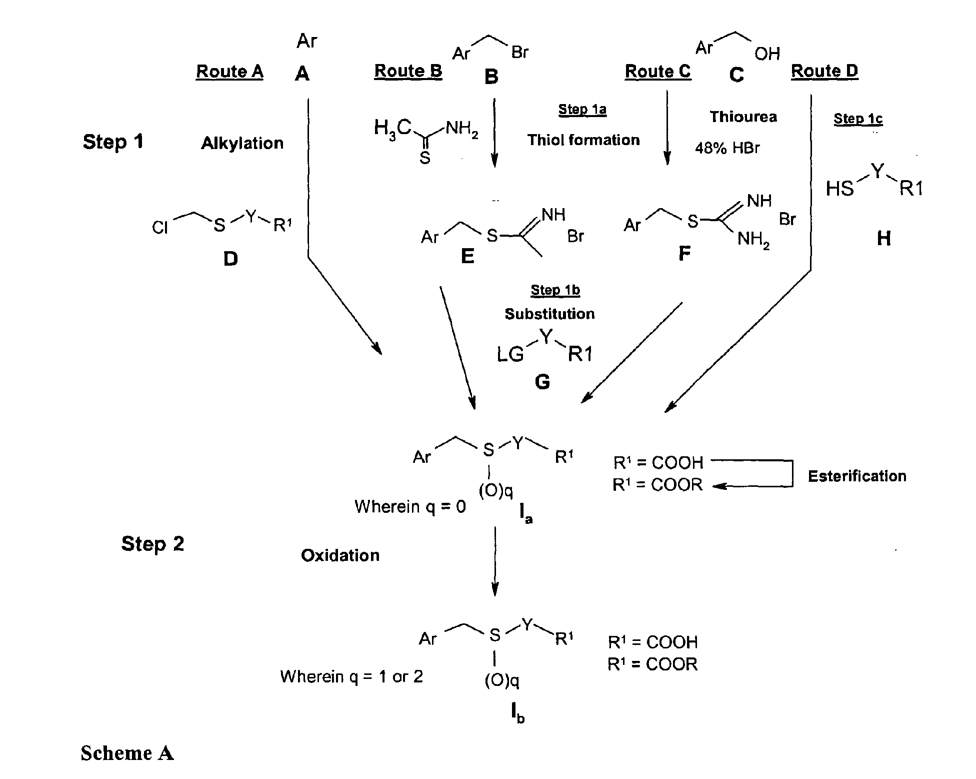

### Schema A: Synthesis of compounds of general structure I (Ia and Ib)

### Step 1: Synthesis of compounds of general structure Ia wherein q is 0 Compounds of general formula Ia may be synthetized via routes A, B, C or D respectively.

### Route A:

An appropriate aryl or heteroaryl, optionally substituted, of general formula **A** wherein Ar is as defined in the final product is reacted with an appropriate compound of general formula D wherein Y and R1 are as defined in a polar aprotic solvent as methylenechloride and like (CHCl₃, CS₂, CCl₄) in presence of a lewis acid like Sn Cl₄, AlCl₃, ZnI₂ at 0°C, under a nitrogen atmosphere to give compound **Ia** wherein Ar, Y and R¹ are as defined. Upon completion, the reaction mixture is concentrated and compound **Ia,** is isolated by conventional methods commonly employed by those skilled in the art.

Optionally, ester of general structure Ia (R¹ = COOR) prepared previously may be hydrolysed in a presence of an inorganic base M-OH as NaOH, LiOH, NH₄OH and the like to obtain the corresponding acid Ia (R¹ = COOH).

### Route B:

*In Step 1a*, the appropriate compound **B** wherein Ar is as defined in the final product, dissolved in a aprotic solvent as chloroform and like (CH₂Cl₂, Toluene, CCl₄...) is be treated with thioacetamide, at a temperature between 60 and 100°C, preferably at reflux, for a period of time in the 2 to 5 hours range. The reaction mixture is cooled to room temperature (in some cases, an ice-bath might be needed) and the precipitated solid is optionally filtered and thoroughly washed with methylenechloride to generate the appropriate **E.**

*In Step 1b*, compound **E** undergoes a substitution reaction with an appropriate compound G of structure LG-Y-R¹ wherein ,Y and R¹ are as defined and LG is an appropriate leaving group (for example an halogene atom as Cl, Br) to generate compounds of general structure **Ia** wherein q=0, Y and R¹ are as defined.

Optionally, the ester of general structure **Ia** (R¹ = COOR) prepared previously may be hydrolysed in a presence of an inorganic base M-OH as NaOH, LiOH, NH4OH and the like to obtain the corresponding acid **Ia** (R¹ = COOH).

### Route C:

*In Step 1a*, the alcohol moiety of an appropriate compound **C** wherein Ar is as defined in the final product is converted to the corresponding thiouronium salt of general formula **F**.

In an aspect, the compound F is formed by reacting a compound C with thiourea and a suitable acid. Suitable acids include but are not limited to mineral acids, such as hydrobromic acid, hydrochloric acid, sulfuric acid.

For example, in Step 1a, an appropriate amount of thiourea in 48% HBr and water is warmed (preferably to 60 - 70 °C), followed by addition of compound **C**. The reaction mixture is refluxed and the stirring is continued for an additional period of time for completion of the reaction. The reaction mixture is cooled to room temperature (in some cases, an ice-bath might be needed) and the precipitated solid is optionally filtered and thoroughly washed with water to generate the appropriate thiouronium salt. Sometimes, there is an oil in place of the solid: in that case, the oil is thoroughly washed with water by decantation and used directly in Step 1b.

*In Step 1b*, the thiouronium salt is first converted into the corresponding thiol which further undergoes a substitution reaction with an appropriate reactant of generic structure structure LG-Y-R¹ (compound **G**) wherein Y and R¹ are as defined and LG is a suitable leaving group (for example an halogene atom as Cl, Br) to generate the corresponding compound **Ia** wherein q=0 and Y and R1 are as defined.

As an example the wet solid of structure **F** (or the oil with some remaining water) from the previous step is taken into additional water and treated with an aqueous base, preferably sodium hydroxide solution. The mixture is warmed preferably to 70 - 80 °C, but in some cases a higher temperature might be needed) and to it an appropriate amount of LG-Y-R¹ in water (or in some cases, an alcoholic solvent) is added. The reaction mixture is refluxed for an appropriate period of time, cooled, taken into water and washed with an organic solvent (preferably ether). The basic aqueous layer is acidified with an inorganic acid solution (e.g. aqueous HCl solution). The aqueous (acidic) solution is then extracted several times into an organic solvent (e.g. ether or ethyl acetate). The combined organic layer is washed with brine, dried (MgSO₄ or Na₂SO₄) and concentrated to give the crude product that may be used directly in the next step. However, purification could be achieved by employing known purification techniques (e.g. recrystallization or column chromatography) to provide pure compound **Ia** wherein q is 0, Y and R¹ are as defined in the final product.

### Route D:

*In Step 1c,* compound **C** wherein Ar is as defined in the final product is converted to the corresponding compound Ia by reacting with a appropriate thio derivative of general structure **H** wherein Y and R1 are as defined in the final product **Ia** in the presence of ZnI₂. Appropriately, the reaction may be conducted under a nitrogen atmosphere.

Alternatively, compound **Ia** wherein R¹ is COOH may be converted into the corresponding alkyl ester R¹ is COOR using methods known by people skilled in the art.

### Step 2: Synthesis of compounds of general structure Ib wherein q is 1 or 2

Compounds of structure Ia wherein q=0 may optionally be oxidized to generate compounds of structure **Ib** wherein q is 1 or 2. Compound **Ib** wherein q is 1 is prepared under mild oxidation conditions by reacting compound Ia wherein q is 0 in an appropriate solvent with an appropriate oxidizing agent. An appropriate oxidizing agent is one that oxidizes the sulphide group of compound Ia. The corresponding product is isolated and purified by methods well known in the art.

For example, to solution of compound **Ia** in acetic acid, an appropriate oxidizing agent (e.g. 30% ww H₂O₂, 1 equivalent) in the acetic acid is slowly added. Stirring is continued at low temperature until the disappearance of the starting material, as evidenced by various analytical techniques. The reaction mixture is concentrated. The desired product (compound **Ib** wherein q is 1) is purified, if needed, by employing known purification techniques (preferably by column chromatography and/or crystallization). In some cases, the oxidation is performed by employing 50% H₂O₂ in glacial acetic acid solvent.

Compound of formula **Ib** wherein q=2, may be obtained under more drastic reaction conditions such as H₂O₂ (more than 2 equivalents) in acidic medium, under heating, preferably at temperature comprise between room temperature and the boiling temperature of the solvent, preferably between 40 and 60°C, for a time sufficient to obtain the desired product, usually approximately between 2 and 10 hours, preferably approximately 8 hours.

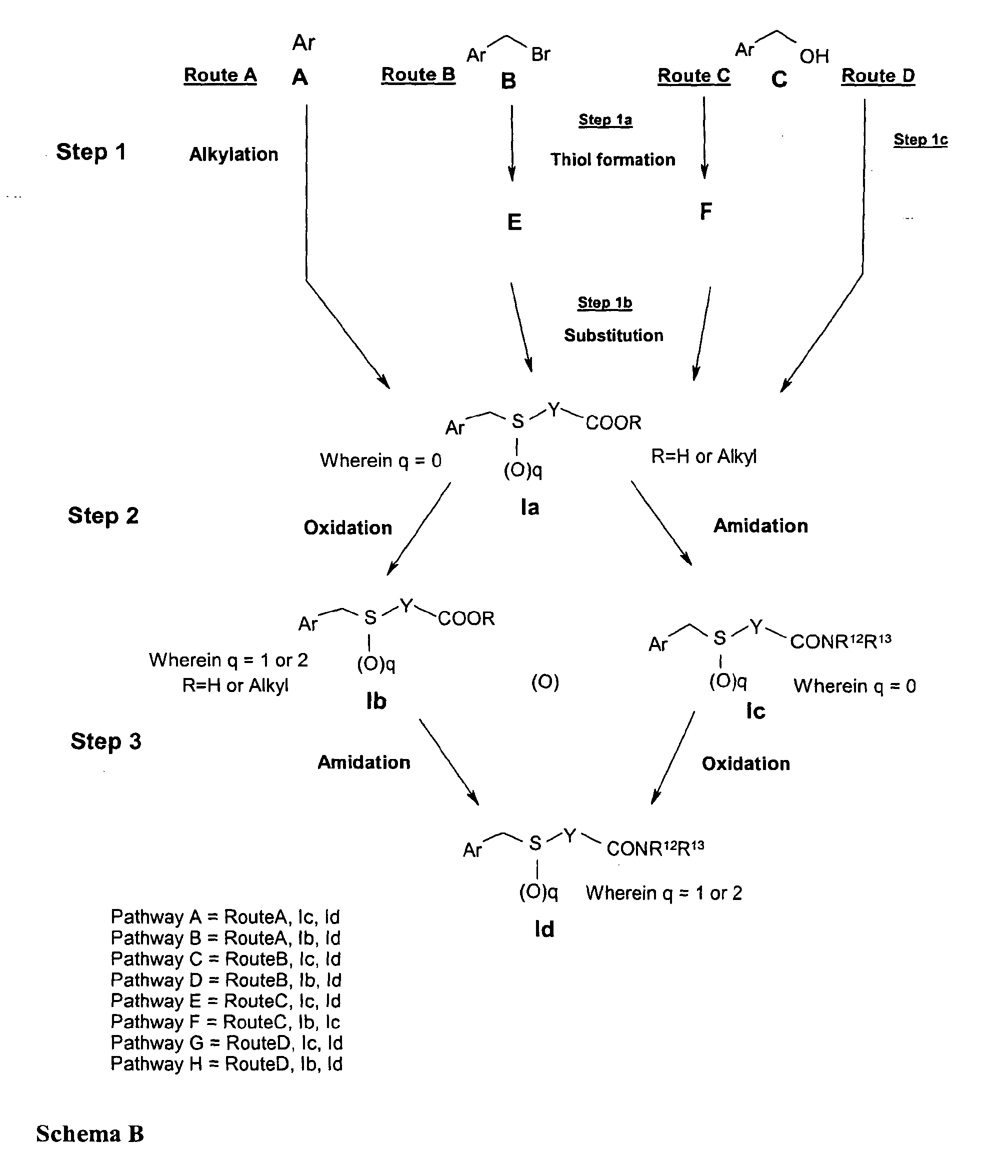

### Schema B: Synthesis of compounds of general Structure I (Ia, Ib, Ic and Id)

Compound **Ia** were prepared according to the general procedures described for Step 1 in Scheme A.Then two different synthetic routes may optionally be used to generate compounds **Id** wherein R¹ is C(=O)NR¹²R¹³.

### Step 2: Synthesis of compounds of general structure Ic wherein q=0:

In Step 2, the appropriate carboxylic ester of general formula **Ia** wherein q=0 and R is alkyl is reacted with an appropriate amine of general structure NHR¹²R¹³ and converted into the corresponding amide of general formula **Ic** wherein q=0, Y, Ar and R¹² and R¹³ are as defined in the final product. May be used aqueous ammonium hydroxide (28%) or methanolic solution of ammonia) or ammonia gas to give the desired compound **Ic** wherein R¹² = R¹³ = H.

Alternatively, in Step 2, the appropriate carboxylic acid of general formula **Ia** wherein q=0 and R = H may be reacted with an appropriate amine of general formula NH R¹² R¹³, a coupling reagent such as EDCI or DCCI, or a polymer supported coupling reagent (N-cyclohexyl carbodiimide), and optionally HOBT in an aprotic solvent as methylene chloride and like to provide amide of general formula **Ic** wherein q=0. An appropriate amine is one which correlates to R¹² and R¹³ as defined in the final product. In some cases, when the appropriate amide bears a protecting group as the tert-butyloxycarbonyl ("Boc") and like on a second nitrogen group, the protected group is further removed in a subsequent step. De-protection of "Boc" may be performed at room temperature by acid treatment such as 4N HCl in 1,4-dioxane or trifluoroacetic acid in CH₂Cl₂.

### Step 3: Synthesis of compounds of general structure Id wherein q=1 or 2:

Compounds of structure **Ic** wherein q=0 may optionally be oxidized to generate compounds of structure **Id** wherein q=1 or 2 according to the procedure described previously in Scheme A Step 2.

Alternatively, in Step 2, compound **Ia** wherein q=0 may be oxidized to generate the corresponding compound of general structure **Ib** wherein q=1 or 2, which, in turn, is amidified appropriately to give raise to compound **Id** in Step 3 of scheme B.

### Examples

Other features of the invention will become apparent in the course of the following descriptions of exemplary embodiments. These examples are given for illustration of the invention and are not intended to be limiting thereof.

### 1) Synthesis of compounds B

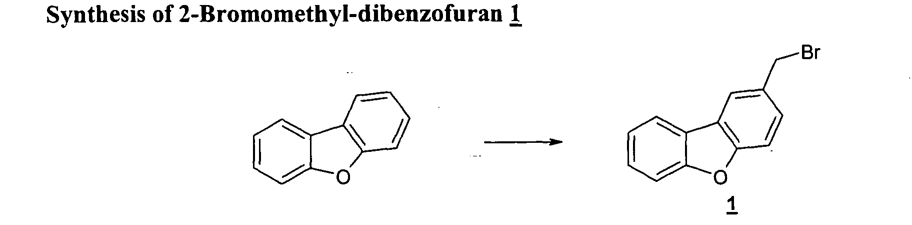

### Compound 1:

A mixture of dibenzofuran (93.5 g, 0.557 M), trioxane (20 g, 0.222 M) and myrystyltrimethylammonium bromide (6.2 g, 18.5 mmol) in 1 L acetic acid and 135 mL of 48%HBr was stirred at 50~60°C for 28 h, then concentrated; the residue was dissolved in CH₂Cl₂, washed with water, dried over Na₂SO₄, evaporated to give the crude bromide **1**. Compound 1 was used without further purification in the next step.

### 2) Synthesis of compounds C

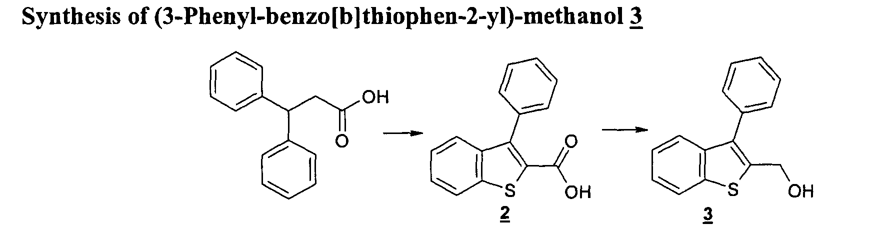

### Compound 2: Synthesis of (3-Phenyl-benzo[b]thiophen-2-yl)-carboxylic acid

To a mixture of 3,3-diphenylpropionic acid (20 g, 88.5 mmol) in 9 mL pyridine was added 8 mL SOCl₂ at room temperature. The resulting mixture was heated to 150~160°C, then additional 23 mL SOCl₂ were added dropwise during 1 h to give a brownish solution, the reaction was stirred at reflux for 2 h, cooled, poured into a mixture of H₂O /conc. HCl / THF (100 mL / 10 mL / 150 mL). The mixture was refluxed for 3 h then cooled. The organic layer was separated, washed with brine, dried over MgSO₄ and concentrated, the residue was treated with 200 mL ethyl ether, filtered and the filtrate concentrated. The crude product that was crystallized in toluene to furnish 10 g of compound **2**.
1H NMR (400 MHz, CHCl₃) δ 7.38 (3H, m), 7.5 (5H, m), 7.89 (1H, dd).

### Compound 3: Synthesis of 3-Phenyl-benzo[b]thiophen-2-yl)-methanol

To a mixture of compound **2** (21.9 g, 86 mmol) in 300 mL dry THF, was added dropwise a solution of 1 M BH₃-THF (105 mL, 105 mmol) at room temperature under N₂ during 15 minutes. The mixture was stirred at room temperature for 18 h, and then quenched by brine. The separated organic layer was washed with brine, dried over MgSO₄ and concentrated. The crude product was purified by flash chromatography (CH₂Cl₂ / methanol, 100 / 1) yielding 17.2 g of compound **3** as a colorless oil that crystallized on stand.
1H NMR (400 MHz, CHCl₃) δ 2 (1H, bs), 4.83 (2H, s), 7.33 (2H, m), 7.4 (3H, m), 7.5 (2H, m), 7.6 (1H, d), 7.82 (1H, dd).

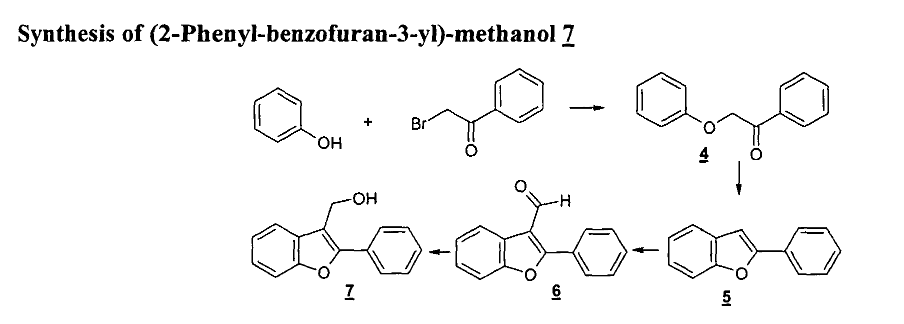

### Compound 4

A mixture of phenol (23.6 g, 0.25 M), 2-bromoacetophenone (50 g, 0.25 M) and K₂CO₃ (35 g, 0.25 M) in 150 mL of acetone was refluxed for 4 h, cooled, poured into 1.5L of water to give a suspension. The solid was collected by filtration, then crystallized in ethanol, dried under vacuum to give 33 g of compound **4** as a beige powder.

### Compound 5

To 150 mL of polyphosphoric acid heated at 130~140°C, was added compound **4** (20 g, 94.3 mmol), the reaction was kept at 130~140°C for 3 h and then poured into 1 L of water. The solid was filtered, rinsed with water, dried in vacuum to give crude product that was crystallized in ethanol to afford 12.2 g of compound **5** as a yellow crystal.
1H NMR (400 MHz, CHCl₃) δ 7.05 (1H, s), 7.25 (2H, m), 7.35 (1H, m), 7.45 (2H, t), 7.5 (1H, d), 7.6 (1H, d), 7.85 (2H, d).

### Compound 6

POCl₃ (10.5 mL, 112.5 mmol) was added to DMF (21 mL at 0~5°C). The mixture was stirred at room temperature for 10 minutes, then compound **5** (4g, 20.6 mmol) was added portionwise. The resultant mixture was heated at 80~90°C for 5 h, stirred overnight at room temperature, poured on ice, and then extracted into CH₂Cl₂, the organic layer was washed with water, dried over MgSO₄, concentrated to give an oil. The crude product was purified by flash chromatography (cyclohexane / ethyl acetate, 5 / 1) to afford 3.3 g of compound **6** as a yellow solid.
1H NMR (400 MHz, CHCl₃) δ: 7.4 (2H, m), 7.55 (4H, m), 7.83 (2H, m), 8.25 (1H, m), 10.33 (1H, s).

### Compound 7

To a solution of compound **6** (3.16 g, 14.2 mmol) in 30 mL of 2-propanol and 20 mL of THF, NaBH₄ (0.8 g, 21.1 mmol) was added at room temperature. 10 minutes later, the reaction mixture was concentrated and quenched with water. The resulting precipitate was filtrated, dried under vacuum to furnish 3.19 g of pure compound **7**.
1H NMR (400 MHz, CHCl₃) δ 4.95(2H, d), 7.25 (2H, m), 7.45 (1H, m), 7.5 (3H, m), 7.7 (1H, d), 7.85 (2H, d).

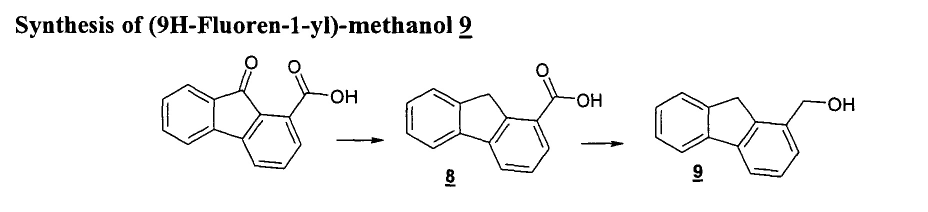

### Compound 8

A mixture of 9-fluorenone-1-caboxylic acid (6 g, 26.8 mmol), a 55% HI solution HI (10.5 mL) and red phosphorous (9,6 g, 310 mmol) in 400 mL acetic acid was refluxed for 48 h, then concentrated. Following addition of 200 mL of water; the mixture was stirred overnight and filtered. The cake was treated in 200 mL water and 5 mL concentrated NaOH and filtered to remove residual phosphorous. The filtrate was adjusted to pH 2 with 4 N HCl to give a suspension that was filtered, washed with, dried in vacuum to afford 5.47 g of compound **8** as a beige solid.
1H NMR (400 MHz, DMSO-d₆) δ 4.25 (2H, s), 7.35 (2H, m), 7.55 (1H, t), 7.63 (1H, d), 7.92 (1H, d), 7.96 (1H, d), 8.2 (1H, d).

### Compound 9

To a slurry of compound **8** (5.47 g, 26 mmol) in 50 mL dry THF, was added dropwise a 1 M solution BH₃-THF (28 mL) at room temperature over a 35 minutes period. The mixture was stirred at room temperature for 16 h then quenched by brine. The organic layer was washed with brine, dried over MgSO₄, concentrated to give 4.5 g of compound **9** as a yellow solid.
1H NMR (400 MHz, CHCl₃) δ 3.9(2H, s), 4.85 (2H, s), 7.35 (4H, m), 7.55 (1H, d), 7.75 (1H, d), 7.8 (1H, d).

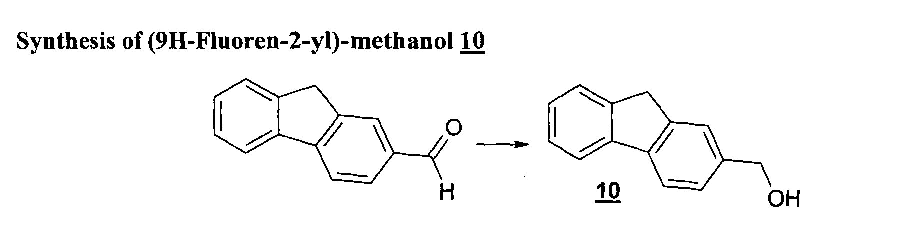

### Compound 10

Compound **10** was prepared according to the process described for as for 3-hydroxymethyl-2-phenyl-benzofuran **7.**
Reagents: Fluorene-2-carboxaldehyde (5.57 g, 28.7 mmol) and NaBH₄ (1.6 g, 42.3 mmol).
1H NMR (400 MHz, CHCl₃) δ 3.9(2H, s), 4.75 (2H, s), 7.3 (3H, m), 7.55 (2H, m), 7.75 (2H, t).

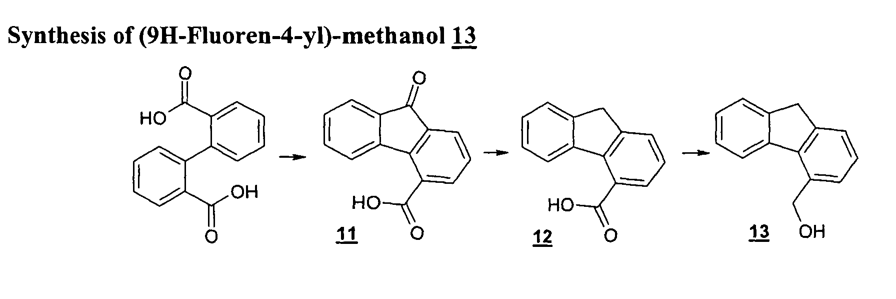

### Compound 11

To 200 mL of concentrated H₂SO₄, was added biphenic acid (70 g, 0.289 M). The resulting mixture was heated to 140 °C for 20 minutes, cooled, poured into ice-water (2 L ) to give a suspension. The mixture was filtered, washed with water, dried at 50°C under vacuum to afford 56 g of compound **11** as greenish yellow solid.
1H NMR (400 MHz, DMSO-d₆) δ: 7.45 (2H, m), 7.65 (2H, q), 7.8 (1H, d), 7.95 (1H, d), 8.25 (1H, d).

### Compound 12

Compound **12** was prepared according to the procedure described for 9-fluorenone-1-caboxylic acid **9.**
Reagents: Compound **11** (22.4 g, 0.1 M), red phosphorous (35.8 g, 1.15 M) and 58% HI (39 ml).
1H NMR (400 MHz, CHCl₃) δ 3.95(2H, s), 7.4 (3H, m), 7.55 (H, d), 7.75 (1H, d), 8.05 (1H, d), 8.6 (1H, d).

### Compound 13

Compound **13** was synthesized in a manner substantially the same as for 3-hydroxymethyl-2-phenyl-benzofuran **8**
Reagents: Compound **12** (9.66 g, 49.3 mmol) and NaBH₄ (1.9 g, 48.7 mmol).
1H NMR (400 MHz, CHCl₃) δ 2.13 (1H, t), 5.1 (2H, d), 7.33 (2H, m), 7.5 (2H, m), 7.6 (1H, d), 7.9 (1H, d), 7.95 ( 1H, d).

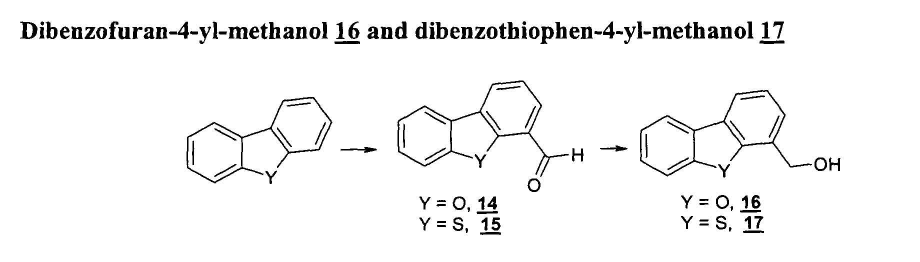

### Compound 14

To a solution of dibenzofuran (6.2 g, 36.9 mmol) in 100 mL dry THF, was added a solution of 1.6 M n-BuLi in hexane (26 mL, 41.6 mmol) at 5°C under a N₂ atmosphere over a 30 minutes period. The resulting solution was stirred at 5°C for 1 h, then DMF (3.2 mL, 41.6 mmol) was added and stirring maintained for 20 minutes at room temperature before adding brine. The organic layer was washed with, dried over MgSO₄ and concentrated. The crude product was recrystallized in 2-propanol to furnish 3.82 g of compound **14** as a yellow crystal.
1H NMR (400 MHz, CHCl₃) δ 7.4 (1H, t), 7.46 (1H, t), 7.53 (1H, t), 7.7 (1H, d), 7.95 (2H, dd), 8.2 (1H, d), 10.58 (1H, s).

### Compound 15

Compound **15** was prepared according to the procedure described for compound **14.**
Reagents : dibenzothiophene ( 36.8g, 226 mmol) and 1.6 M n-BuLi in hexane (140 mL, 224 mmol), DMF (16.5g, 226 mmol)
1H NMR (400 MHz, CHCl₃) δ 7.53 (2H, m), 7.68 (1H, t), 7.97 (1H, m), 7.99 (1H, dd), 8.22 (1H, m), 8.44 (1H, dd), 10.3 (1H, s).

### Compound 16

Compound **16** was synthesized in a manner substantially the same as for 3-hydroxymethyl-2-phenyl-benzofuran **7**
Reagents: Compound **14** (9.66 g, 49.3 mmol) and NaBH₄ (1.9 g, 48.7 mmol).
1H NMR (400 MHz, CHCl₃) δ 2.13 (1H, t), 5.1 (2H, d), 7.33 (2H, m), 7.5 (2H, m), 7.6 (1H, d), 7.9 (1H, d), 7.95 (1H, d).

### Compound 17

Compound **17** was prepared according to the procedure described for compound **16**
Reagents : compound **15** (22.2g, 104.7 mmol) and Na BH4 (4.5 g, 118 mmol)
1H NMR (400 MHz, CHCl₃) δ 1.95 (1H, t), 5 (2H, d), 7.5 (4H, m), 7.87 (1H, m), 8.1 (1H, m), 8.2 ( 1H, m) .

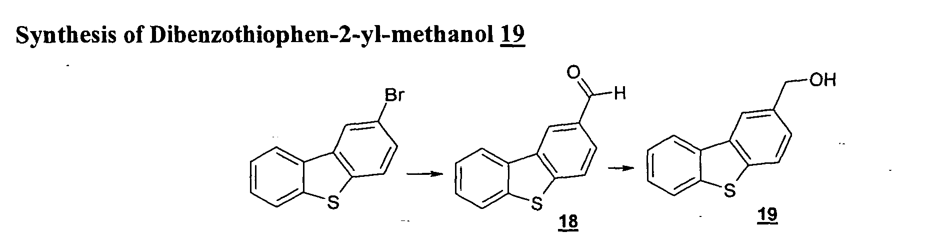

### Compound 18

To a solution of 2-bromodibenzofuran (33.7 g, 0.128 M) (*Bull. Soc. Chim. Fr*, 1973, 11, 3110~3115) in 300 mL of dry ethyl ether, a solution of 1.6 M n-BuLi in hexane (85 mL, 0.136 M) was added at 5°C under N₂ over a 30 minutes period, followed by the addition of DMF (10 g, 0.137 M). The mixture was stirred at room temperature for 40 minutes, then a saturated solution of NH₄Cl (300 mL) was added. The organic layer was washed with brine, dried over Na₂SO₄ and concentrated. The crude product that was recrystallized in ethanol to furnish 25.9 g of compound **18** as a yellow crystal.
1H NMR (400 MHz, CHCl₃) δ 7.55 (2H, m), 7.87 (1H, m), 8.0 (2H, m), 8.25 (1H, m), 8.7 (1H, s), 10.2 (1H, s).

### Compound 19

Compound **19** was synthesized in a manner substantially the same as for 3-hydroxymethyl-2-phenyl-benzofuran **7.**
Reagents: Compound **18** (20.6 g, 97.2 mmol) and NaBH₄ (4 g, 105.8 mmol).
1H NMR (400 MHz, CHCl₃) δ 4.86 (2H, s), 7.5 (3H, m), 7.86 (2H, m), 8.25 (1H, m), 8.2 (2H, m).

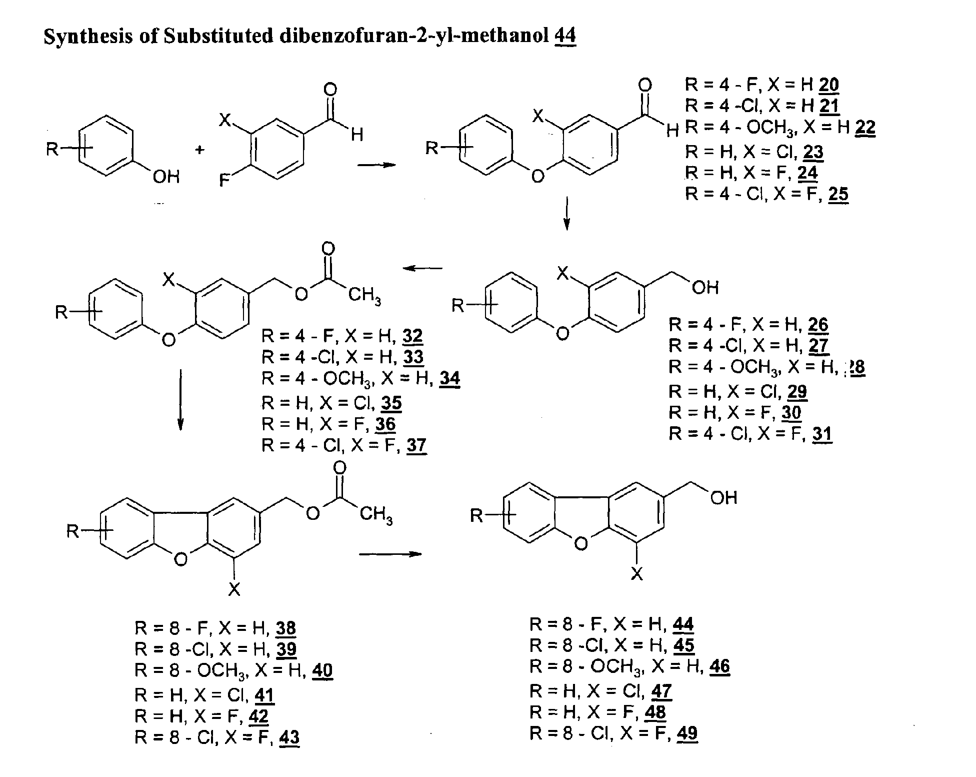

### Compounds 20 and 26

A mixture of 4-fluorophenol (41.3 g, 0.37 M), 4-fluorobenzaldehyde (45.7 g, 0.37 M) and K₂CO₃ (55 g) in 450 mL DMF was refluxed for 4 h, cooled, poured into ice water (1.5 L) to give a suspension. The mixture was filtered, washed with water, dried at 50°C under vacuum to generate 78.3 g of the aldehyde **20** as a brownish solid. This compound was slurried in 2-propanol (1 L), then NaBH₄ (14 g, 0.378 M) was added portionwise at room temperature. The resulting mixture was stirred at room temperature for 1 h, then concentrated,. The residue was poured into water (1.5 L) to give a suspension. After filtration, the resulting solid, washed with water, dried at 50°C under vacuum to give 76 g of 4-fluorophenoxybenzyl alcohol **26** as a yellowish crystal.
1H NMR (400 MHz, CHCl₃) δ 4.67 (2H, d), 7.0 (6H, m), 7.33 (2H, d).

### Compound 32

To a solution of compound **26** (21.8 g, 0.1 M) and triethylamine (14 mL, 0.1 M) in 300 mL CH₂Cl₂, acetyl chloride (8 g, 0.102 M) was added at room temperature. The mixture was kept at room temperature for 18 h, then washed with water, dried over Na₂SO₄, concentrated to afford 27.3 g of 4-chlorophenoxybenzyl acetate **32** as an oil.
1H NMR (400 MHz, CHCl₃) δ 2.1 (3H, s), 5.05 (2H, s), 6.93 (2H, d), 7.0 (4H, m), 7.3 (2H, d).

### Compound 38

A brownish mixture of **32** (22.2 g, 85.4 mmol) and palladium acetate (35 g, 156 mmol) in acetic acid (250 mL) was refluxed for 18 h, then filtered on Celite to eliminate palladium. The filtrate was concentrated to dryness. The crude producte was purified by flash chromatography (cyclohexane / ethyl acetate, 5 / 1) to generate 17.6 g of 8-fluoro-2-acetoxymethyldibenzofuran **38** as an off-white solid.
1H NMR (400 MHz, CHCl₃) δ 2.1 (3H, s), 5.2 (2H, s), 7.12 (1H, tt), 7.46 (2H, m), 7.5 (1H, d), 7.56 (1H, dd), 7.73 (1H, s).

### Compound 44

To a suspension of **38** (10.3 g, 40 mmol) in 150 mL of methanol and 50 mL of water, LiOH monohydrate (3.5 g, 83.3 mmol) was added at room temperature. The mixture was stirred at 50°C for 30 minutes, concentrated. The mixture was quenched with water (200 mL) to give a suspension that was filtered, washed with water, dried at 50°C under vacuum to give 8.4 g of 8-fluoro-2-hydroxymethyldibenzofuran **44** as a white crystal.
1H NMR (400 MHz, DMSO-d₆) δ 4.7 (2H, s), 7.33 (1H, m), 7.5 (1H, d), 7.67 (1H, d), 7.7 (1H, m), 8.03 (1H, dd), 8.11 (1H, s).

### Compounds 45 to 49

Compounds **45** to **49** were processed according to the procedure described for compound **44.**

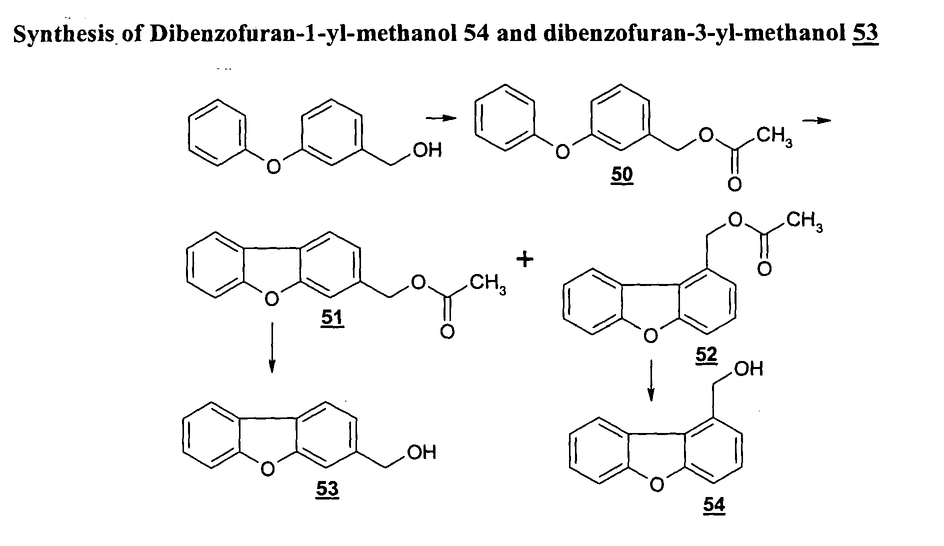

### Compounds 51 and 52

A mixture of 3-phenoxybenzyl acetate **50** (21.8 g, 90 mmol) (prepared by acetylation of commercial 3-phenoxybenzyl alcohol) and palladium acetate (40 g, 179 mmol) in acetic acid (300 mL) was refluxed for 6 h, cooled and filtered. The filtrate was evaporated to dryness and the residue treated with 100mL of a mixture cyclohexane / ethyl acetate (6 / 1) and filtered. The filtrate was concentrated to give a colored oil which was purified by chromatography on silica gel (cyclohexane / ethyl acetate, 6 / 1) to afford pure dibenzofuran-3-yl-methyl acetate **51** (3.4 g) and a mixture of dibenzofuran-3-yl-methyl acetate **51** (Rf = 0.6) and dibenzofuran-1-yl-methyl acetate **52** contaminated with compound 51 (Rf of compound 52= 0.53) (8.4 g) as a white solid. 52 will be used without any further purification in the next step
Compound **51****:** 1H NMR (400 MHz, CHCl₃) δ 2.15 (3H, s), 5.3 (2H, s), 7.33 (2H, t), 7.45 (1H, t), 7.56 (1H, d), 7.59 (1H, s), 7.96 (1H, t).

### Compounds 53 and 54

Acetyl group of Compound **51** is further removed to give pure compound **53** using the same procedure as described for preparation of compound **44.**

Starting from **52** (contaminated by some isomer **51**), a mixture of 53 and 54 was prepared according to the same method.
Compound **53 :** 1H NMR (400 MHz, CHCl₃) δ 2.0 (1H, bs), 4.84 (2H, s), 7.45 (1H, t), 7.53 (1H, d), 7.59 (1H, s), 7.92 (2H, dd).

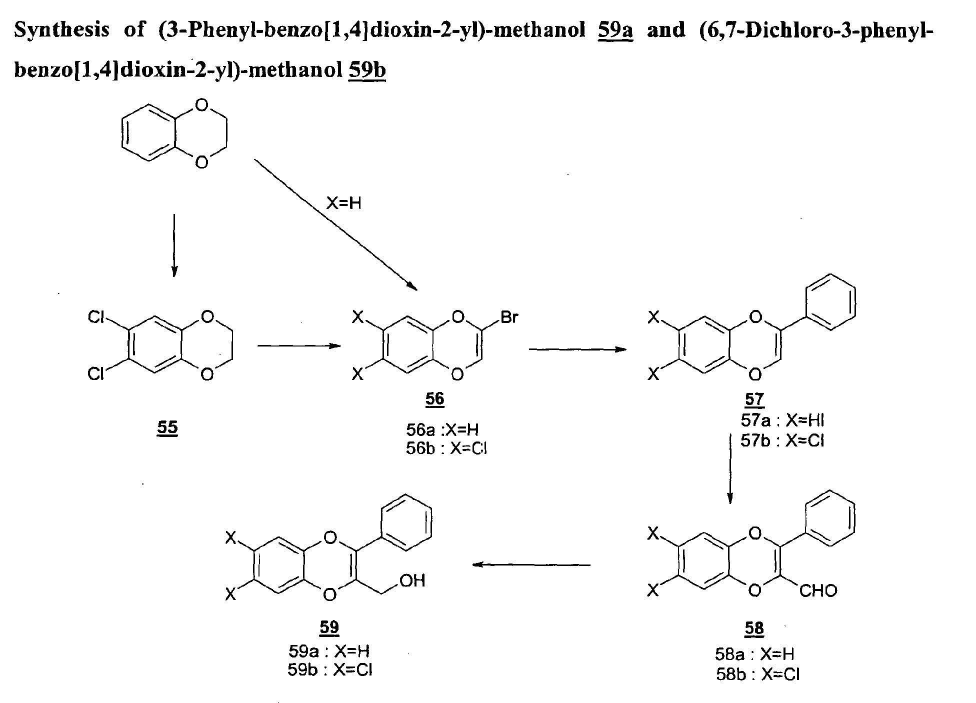

### Compound 55

Compound **55** was prepared according to the procedure described in *Organic Process Research & Development,* 5(2), **2001,** 116-121.
Reagents : 1,4-benzodioxane (10 g, 73.4 mmol), *N*-chlorosuccinimide (20.6 g, 154 mmol) and acetic acid (20 mL).
Yield = 68 % (m=10.2 g).
¹H NMR (400 MHz, CDCl₃) δ 4.3 (4H, s), 6.95 (2H, s).

### Compound 56 a (X=H)

Compound **56a** was prepared according to the procedure described in *Synthesis,* 1977, 755 and *Tetrahedron,* 46(3), 1990, 921-934.
Reagents : 1,4-benzodioxane (12 g, 88 mmol), N-bromosuccinimide (37,6 g, 211 mmol), AIBN (small amount), CCl₄ (120 ml), potassium t-butoxide_(15g, 134 mmol), Et₂O (250 ml).
Yield = 66 % (m=12.4 g).
¹H NMR (400 MHz, CDCl₃) δ 6.05 (1H, s), 6.6-6.9 (4H, m).

### Compound 56b (X=Cl)

Similary, compound **56b** was prepared.

Under nitrogen, a mixture of compound **55** (5.6 g, 27.4 mmol), N-bromosuccinimide (11.7 g, 65.8 mmol) and a small amount of AIBN in tetrachloromethane (340 ml) is refluxed for 5 h. After cooling, the solid material is filtered off and the solution is evaporated to give 2,3-dibromo-2,3-dihydro-1,4-benzodioxin. Under nitrogen, to a stirred suspension of potassium t-butoxide (9.22 g, 82 mmol) in anhydrous tetrahydrofuran (45 ml), was added dropwise at 0°C, a solution of 2,3-dibromo-2,3-dihydro-1,4-benzodioxin in tetrahydrofuran (35 ml), and the mixture was stirred for 4 h. The solid was filtered off on celite and the solution is concentrated. 200 ml of water was added and the aqueous layer was extracted with dichloromethane.The organic layers were dried over MgSO₄ and concentrated to afford a residue which was purified by column chromatography (petroleum ether) to give 6.26 g (yield=81 %) of compound **56b** (white powder).
¹H NMR (400 MHz, CDCl₃) δ 6.05 (1H, s), 6.80 (1H, s), 6.85 (1H, s).

### Compound 57a (X=H)

Compound **57a** was prepared according to the procedure described in *Tetrahedron*, 53(6), 1997, 2061-2074.
Reagents : compound **56a** (12.4 g, 58.2 mmol), toluene (200 ml), Na₂CO₃ 2M (58 ml), phenylboronic acid (14.2 g, 116 mmol), EtOH (70 ml), tetrakis(triphenylphosphine)palladium (2.7 g, 2.3 mmol).
Yield = 74 % (m=9 g).
¹H NMR (400 MHz, CDCl₃) δ 6.45 (1H, s), 6.6-6.9 (4H, m), 7.25-7.5 (5H, m).

### Compound 57b (X=Cl)

Similary, compound **57b** was prepared.

Under nitrogen, to a solution of compound **56b** (0.3 g, 1.07 mmol) in 4 ml of toluene, were added 1.1 ml of an aqueous solution o 2M Na₂CO₃, phenylboronic acid (0.26g, 2.14 mmol) in 1.3 ml of ethanol and tetrakis(triphenylphosphine)palladium (0.05 g, 0.043 mmol). The mixture was heated at 78°C for 3h.

After concentration, 30 ml of water was added and the aqueous layer was extracted with dichloromethane. The organic layers were dried over MgSO₄ and concentrated to afford a residue which was purified by column chromatography (petroleum ether) to give 0.24 g (yield=81 %) of compound **57b** (white powder).
¹H NMR (400 MHz, CDCl₃) δ 6.45 (1H, s), 6.80 (1H, s), 6.85 (1H, s), 7.25-7.5 (5H, m).

### Compound 58a (X=H)

Compound **58a** was prepared according to the procedure described in *Chem. of heterocyclic Compds,* 35(10), 1999, 1480-1481.
Reagents : compound **57a** (9g, 42.8 mmol), phosphorus oxychloride (7.88 g, 51.4 mmol), DMF (20 ml, 257 mmol).
Yield : 74% (7.5 g).
¹H NMR (400 MHz, CDCl₃) δ 6.8-7.1 (4H, m), 7.4-7.8 (5H, m), 9.15 (1H, s).

### Compound 58 b(X=Cl)

Similary, compound **58b** was prepared.

Under nitrogen, to a stirred solution of compound **57b** (5.30 g, 19 mmol) in 120 ml of DMF was added dropwise POCl₃ (2.12 mL, 22.8 mmol). The reaction mixture was stirred at 60°C during 22 h. Then, the mixture was treated with a solution of sodium acetate trihydrate (11.6 g) in water (14 mL) and heated with stirring until crystallization began. After cooling, water was added and the precipitate was filtered off and then washed with 2-propanol to give 4.9 g (yield = 84%) of compound **58b** (yellow powder).
¹H NMR (400 MHz, DMSO-d₆) δ 9.12 (1H, s), 7.75-7.52 (5H, m), 7.45 (1H, s), 7.40 (1H, s), 6.85 (1H, s),.

### Compound 59a (X=H)

Under nitrogen, to a stirred suspension of compound **58a** (7.5 g, 31.5 mmol) in 80 ml of methanol, was added at 0°C, by fraction, sodium borohydride (0.77 g, 20.5 mmol). After 45 min, 10 ml of water was added and the mixture was neutralized with HCl 2N and then methanol was evaporated. After extraction with CH₂Cl₂ (2*150 ml), the organic layer was dried over MgSO₄ and concentrated to give 7.6 g (yield=100%) of compound **59a** (white powder).
¹H NMR (400 MHz, CDCl₃) δ 3.95 (2H, d), 5.3 (1H, t), 6.8-7.1 (4H, m), 7.30-7.65 (5H, m).

### Compound 59b (X=Cl)

Similary, compound **59b** was prepared.

Under nitrogen at 0°C, to a stirred suspension of compound **58b** (5 g, 16.3 mmol) in 150 ml of methanol, was added portionwise sodium borohydride (0.50 g, 13 mmol). After 3 h, the reaction mixture was quenched with water (20 mL) and methanol was evaporated. The aqueous residue was neutralized with HCl 2N and extracted with CH₂Cl₂. The organic layer was dried over MgSO₄, concentrated and purified by flash chromatography (petroleum ether/dichloromethane 50/50) to give 3.1 g (yield=62%) of compound **59b** (white powder).
¹H NMR (400 MHz, DMSO-d₆) δ 7.57-7.43 (5H, m), 7.26 (1H, s), 7.23 (1H, s), 5.36 (1H, t), 3.96 (2H, d).

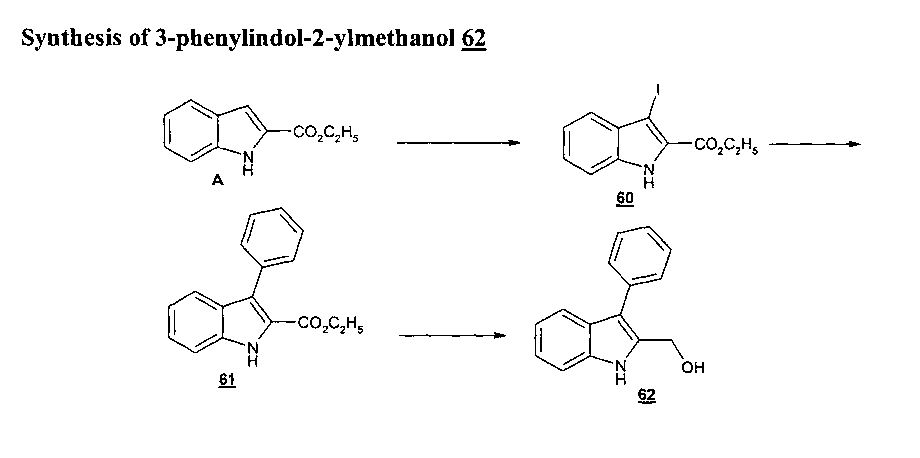

### Compound 60: Ethyl 3-iodoindole-2-carboxylate

A 1L round-bottom flask containing a magnetic stirring bar equipped with a reflux condenser was charged with 10g (0.053mol) of ethyl indole-2-carboxylate (A), 100mL DMF, 50mL of water, 30g (0.106mol) of iodine and 6.6g (0.106mol) of potassium hydroxide. The resulting mixture was heating at 70°C for 3 hours. 500mL of ice was poured into this flask, and the mixture was agitating for an hour. After filtration and drying, we obtained 15g (315,1g.mol⁻¹) of expected compound 60.
Yield : 90%.
1H NMR (400 MHz, CDC13) δ 1.45 (3H, t) 4.48 (2H, q) 7.22 (1H, q) 7.38 (2H, m) 7.57 (1H, q) 9.23 (1H, bs).

### Compound 61: Ethyl 3-phenylindole-2-carboxylate

A 250mL round-bottom flask containing a magnetic stirring bar equipped with a reflux condenser was charged with 4.8g (0.0152mol) of B, 100mL of toluol, 1.6g (10%) of palladium tetrakis, 50mL of ethanol, 2.0g (0.0167mol) of phenylboronic acid, 50mL of water and 5g of potassium carbonate. The resulting mixture was refluxed for 48 hours. At room temperature, 100mL of water was added, the mixture was extracted with 2×100mL of toluol. The organic layer was dried with magnesium sulfate, filtered and evaporated to dryness. The crude mixture was then triturated in 20mL of diethyl ether and filtered. We obtained 4g (265.31g.mol⁻¹) of the expected compound 61**.**
Yield: 99%.
1H NMR (400 MHz, CDC13) δ 1.23 (3H, t) 4.29 (2H, q) 7.15 (1H, m) 7.36 (2H, m) 7.43 (2H, m) 7.45 (2H, m) 7.55 (1H, m) 7.63 (1H, m) 8.99 (1H, m).

### Compound 62: 3-phenylindol-2-yl-methanol

A 250mL round-bottom flask containing a magnetic stirring bar equipped with a reflux condenser was charged with 1.4g of C (0.0053mol) and 100mL of dry THF. At room temperature, 10mL of LiAlH₄ (1M in THF) were added slowly over 15 minutes. The mixture was stirring for 15 minutes and then 20mL of crude ice was added. HCl (1M) was added until pH=1. The solution was evaporated to dryness. To the crude mixture obtained was added 50mL of water. The expected product was extracted with 3x50mL of ethyl acetate. The organic layer was dried over magnesium sulfate, filtered and evaporated to dryness. The product was purified by chromatographic column with ethyl acetate as éluant. We obtained 0.8g (223,27g.mol⁻¹) of compound 62.
Yield : 68%.
1H NMR (400 MHz, CDCl3) δ 1.83 (1H, bs) 4.94 (2H, s) 7.14 (2H, m) 7.23 (2H, m) 7.25 (1H, m) 7.38 (2H, m) 7.47 (2H, m) 7.72 (1H, m) 8.53 (1H, bs).

### 3) Synthesis of compounds Ia

### Example 1

Compound **Ia:** dibenzofuran-2-ylmethylsulfanyl-acetic acid ethyl ester.

To a solution of dibenzofuran (2.5 g, 14.9 mmol), ethyl chloromethylthioacetate (2.5 g, 14.8 mmol) [prepared according to Synthesis, 1984, 326] in 20 mL CH₂Cl₂, SnCl₄ (1.8 ml, 15.4 mmol) was added at 0°C under N₂. The reaction was concentrated after 10 minutes at 0°C and the residue was purified by flash chromatography (cyclohexane / ethyl acetate, 5 / 1) to afford 2.8 g of Exemple **1** as a colorless oil.
1H NMR (400 MHz, CHCl₃) δ 1.25 (3H, t), 3.1 (2H, s), 3.9 (2H, s), 4.15 (2H, q), 7.25 (1H, t), 7.4 (2H, m), 7.45 (1H, d), 7.5 (1H, d), 7.83 (1H, s), 7.87 (1H, d).

### Example 2

Compound **Ia:** dibenzofuran-2-ylmethylsulfanyl-acetic acid.

### Preparation from example 1 Scheme A Route A

Example 1 (10 g, 33.3 mmol) was dissolved in 80 mL of methanol, 80 mL of THF and 40 mL of H₂O, then LiOH monhydrate (3.1 g, 73.8 mmol) was added. The mixture was stirred at room temperature for 2 days. After solvants evaporation, water was added and the resulting solution acidified to pH 2. The precipitate was filtered, washed with water and dried under vacuum to afford 8.7 g of Example **2** as a white solid.
1H NMR (400 MHz, DMSO-d₆) δ 3.2 (2H, s), 4.0 (2H, s), 7.4 (1H, t), 7.47 (1H, dd), 7.5 (1H, t), 8.05 (1H, s), 8.15 (1H, d).

### Preparation via Scheme A Route B

Thioacetamide (30 g, 0.4 M) was added to a solution of compound **1** (prepared as described earlier on) in 900 mL CHCl₃,. The mixture was refluxed for 2 h. The resulting suspension was cooled, filtered, washed with CH₂Cl₂, dried under vacuum to afford 33 g of compound **E** wherein Ar correspond to 2-dibenzofuryl as an off-white solid.

A suspension of compound **E** (20.3 g, 60.4 mmol) in 23 mL 32%NaOH and 30 mL water was heated at 70°C, then a solution of chloroacetic acid (6.4 g, 68 mmol) in 4.5 mL 32%NaOH and 25 mL water was added to give a viscous suspension which was diluted with 50 mL water. The mixture was refluxed for 1 h, diluted with 500 mL of water, acidified with concentrated HCl until pH 2. The suspension was filtered and the crude product washed with water, dried under vacuum to afford 15.4 g of Example **2** as a white solid.
1H NMR (400 MHz, DMSO-d₆) δ 3.2 (2H, s), 4.0 (2H, s), 7.4 (1H, t), 7.47 (1H, dd), 7.5 (1H, t), 8.05 (1H, s), 8.15 (1H, d).

### Example 3

### Compound Ia: 2-(8-chlorodibenzofuran-2-yl-methylsulfanyl) acetic acid

To a solution of thiourea (3g, 39.5 mmol) in 48% HBr (25 mL), in an heated bath (100°C), 8-chloro-2-hydroxymethyldibenzofuran **45** (6.76 g, 29 mmol) was added portionwise. The mixture was diluted with water (20 mL), heated to 110°C for 1 h 40 minutes, cooled, then filtered. The precipitate was washed with water, dried at 50°C under vacuum to give 10.4 g of thiouronium hydrobromide as an off-white solid. This compound (8.4 g, 22.6 mmol) was treated in 32% NaOH (20 ml) at 90°C, diluted with water (30 mL), then a solution of chloroacetic acid (2.5 g, 26.5 mmol), NaHCO3 (2.3 g, 27.4 mmol) in water (20 mL) was added. The mixture was refluxed for 1 h, cooled, acidified with concentrated HCl at 5°C. the crude product was filtered, washed with water , dried under vacuum to afford 6.8 g of pure 8-chlorodibenzofuran-2-yl-methylsulfanic acid (Example **3**) as a white solid.
1H NMR (400 MHz, CHCl₃) δ 3.13 (2H, s), 4.05 (2H, s), 7.4 (1H, dd), 7.5 (3H, m), 7.91 (2H, d).

### Example 4

### Compound Ia: 2-(fluoren-4-ylmethylsulfanyl)acetic acid

To a solution of thiourea (4.2 g, 55.3 mmol) in 48% HBr (25 ml ) heated in a bath of 80°C, was added Compound **13** (9 g, 45.9 mmol) to give a thick suspension which was diluted with 15 ml of water. The resultant mixture was refluxed for 10 minutes, then cooled, filtered, washed with water to give a beige solid.

A mixture of above obtained compound in16% NaOH (40 ml) was heated at 70 °C, then a solution of chloroacetic acid (5 g, 52.9 mmol) in 1N NaOH (50 ml) was added. The resulting mixture was heated at reflux for 90 minutes, filtered while hot, the filtrate was cooled and acidified by concentrated HCl to pH 2 to give a suspension that was filtered, washed by water, dried in vacuum to afford 9 g of pure Example **4** as a brownish solid.
1H NMR (400 MHz, DMSO-d₆) δ : 3.25 (2H, s), 3.97 (2H, s), 4.25 (2H, s), 7.25 (2H, m), 7.33 (1H, t), 7.43 (1H, t), 7.53 (1H, d), 7.6 (1H, d), 8.0 (1H, d).

### Example 5

### Compound Ia: 2-(2-dibenzothiophen-methylsulfanyl)acetic acid

To a solution of thiourea (4.7 g, 61.8 mmol) in 48% HBr (30 ml ) heated in a bath of 80°C, was added Compound **19** (10.7 g, 50 mmol ) to give a very thick suspension which was diluted with 30 ml of 48% HBr and 20 ml water. The resultant mixture was refluxed for 2 hr, then cooled, filtered, washed with water, dried to give 17.3 of white solid.

The above obtained compound was mixed with 32% NaOH (25 ml) and 20 ml water, heated at 70 °C, then a solution of sodium chloroacetate (6 g, 51.5 mmol) in water (50 ml) was added to give a solution. The resulting mixture was heated at reflux for 60 minutes, cooled, diluted by water (200 ml) and acidified by concentrated HCl to pH 2 to give a suspension that was extracted by methylenechloride, the organic phase was washed by brine, dried over Na₂SO₄, evaporated to afford 13 g of pure Example **5** as a yellowish solid.
1H NMR (400 MHz, DMSO-d₆) δ : 3.2 (2H, s), 4.0 (2H, s), 7.5 (3H, m), 8.0 (1H, d), 8.03 (1H, m), 8.28 (1H, s), 8.33 (1H, m).

### Example 6

### Compound Ia: 2-(8-fluorodibenzofuran-2-yl-methylsulfanyl)acetic acid

To a solution of thiourea (3.7 g, 48.7 mmol) in 48% HBr (45 ml ) heated in a bath of 80°C, was added 8-fluoro-2-hydroxymethyldibenzofuran **44** (8.4 g, 38.9 mmol ) to give a very thick suspension which was diluted with 15 ml water. The resultant mixture was refluxed for 2 hr, then cooled, filtered, washed with water, dried to give a white solid.

The above obtained compound was mixed with 32% NaOH (20 ml), heated at 70 °C, then a solution of sodium chloroacetate (4.7 g, 40.3 mmol) in water (40 ml) was added to give a suspension. The resulting mixture was heated at reflux for 60 minutes, cooled, diluted by water (500 ml) and acidified by concentrated HCl to pH 2 to give a suspension that was filtered, rinsed with water dried in vacuum to afford 11 g of pure Example **6** as a beige solid.
1H NMR (400 MHz, DMSO-d₆) δ : 3.18 (2H, s), 4.0 (2H, s), 7.35 (1H, dt), 7.5 (1H, d), 7.66 (1H, d), 7.75 (1H, dd), 8.0 (1H, d), 8.03 (1H, dd), 8.1 (1H, s).

### Example 7

### Compound Ia : (3-phenyl-benzo[b]thiophen-2-ylmethylsulfanyl)-acetic acid .

To a mixture of thiourea (2.74g, 36 mmol) and 48% HBr (15.75mL) in water (3mL) at 60°C, compound **3** (7.2g, 30mmol) was added in one portion was added. The reaction mixture was then gently heated to reflux for 5mn, cooled. The mixture of HBr and water was decanted from the resulting oil, water was added and decanted again. To the resulting residue, aqueous NaOH (10N, 12mL) was added, and the mixture heated to 70°C. A solution of sodium chloroacetate (33 mmol) in 9mL of water was slowly added. The reaction mixture was then heated to 110°C for 1h, cooled, diluted with ice-water (100 mL) , and acidified with conc. hydrochloric acid (pH~2). The resulting acidic mixture was extracted into diethyl ether (2 x 150mL), the separated organic layer was washed with a solution of NaOH (4 N) and the aqueous layer acidified again (pH~2), extracted into diethyl ether (400mL). The combined organic layers were dried over Na₂SO₄, and solvent evaporated to generate 7g of compound 50 as a yellow oil.
Yield = 74%,
R_{f} = 0.4 (eluent: CH₂Cl₂/CH₃OH 9/1).

In the following examples **8** to **10** compound **Ia** from example **2****,** **5** and **6** where R = H were converted into their corresponding compound **Ia** where R = CH3 (methyl ester).

### Example 8

### Compound Ia: 2-(dibenzofuran-2-ylmethylsulfanyl)acetic acid-methyl ester

A mixture of the Example **2** (4.22 g, 15.5 mmol) in methanol (30 ml) and concentrated H₂SO₄ (1 ml) was refluxed for 2 hr and then evaporated. The residue was dissolved in 100 ml methylenechloride, washed by water, dried over Na₂SO₄, purified by flash chromatography (cyclohexane / ethyl acetate, 5 /1) to furnish 3.7 g of Example **8** as a yellowish oil.
HPLC: Ret. Time = 15.92 min (Column: Zorbax Eclipse XDB-C8; 4.6*150 mm, 5µm; Mobile Phase: A: 0.1% TFA in H₂O, B: 0.1% TFA in ACN; Gradient: 10-100%B in 20 min.; Flow Rate: 1 ml / min.; Temperature: 25 °C)
1H NMR (400 MHz, CHCl3) δ 3.1 (2H, s), 3.75 (3H, s), 3.9 (2H, s), 7.5 (3H, m), 7.25 (1H, ?), 7.4 (2H, m), 7.45 (1H, d), 7.5 (1H, d), 7.83 (1H, s), 7.87 (1H, d).

### Example 9

### Compound Ia: 2-(dibenzothiophen-2-ylmethylsulfanyl) acetic acid methyl ester

A mixture of the Example **5** (2.88 g, 10 mmol) in methanol (30 ml) and concentrated H₂SO₄ (1 ml) was heated at reflux for 2 hr, then evaporated, the residue was dissolved in 100 ml methylenechloride, washed by water, dried over Na₂SO₄, purified by flash chromatography (cyclohexane / ethyl acetate, 5 / 1) to furnish 2.72 g of Example **9** as a yellowish oil.
1H NMR (400 MHz, CHCl₃) δ 3.11 (2H, s), 3.75 (3H, s), 4.3 (2H, s), 7.5 (3H, m), 7.81 (1H, d), 7.84 (1H, m), 8.13 (1H, s), 8.18 (1H, m).

### Example 10

### Compound Ia: 2-(8-fluorodibenzofuran-2-ylmethylsulfanyl) acetic acid methyl ester

A mixture of the Example **6** (2.9 g, 10 mmol) in methanol (30 ml) and concentrated H₂SO₄ (1 ml) was heated at reflux for 2 hr, then evaporated, the residue was dissolved in 100 ml methylenechloride, washed by water, dried over Na₂SO₄, purified by flash chromatography (cyclohexane / ethyl acetate, 5 / 1) to furnish 2.34 g of Example **10** as a colorless oil.
1H NMR (400 MHz, CHCl₃) δ 3.11 (2H, s), 3.75 (3H, s), 4.3 (2H, s), 7.5 (3H, m), 7.81 (1H, d), 7.84 (1H, m), 8.13 (1H, s), 8.18 (1H, m).

### Example 11

### Compound Ia: 2-(8-methoxydibenzofuran-2-ylmethylsulfanyl) acetic acid ethyl ester

### (Synthesis: route D)

To a solution of compound **46** (5.48 g, 24 mmol) in methylenechloride (120 ml), were added at RT ethyl thioglycolate (3.1 g, 25.8 mmol) and ZnI₂ (8.5 g, 26.6 mmol) to give a suspension. The reaction was stirred at RT for 2 hr, quenched by water, the organic phase was dried over Na₂SO₄, evaporated, the residue was purified by flash chromatography (cyclohexane /ethyl acetate, 6 / 1) to furnish 3.58 g of Example **11** as a colorless oil.
HPLC: Ret. Time = 16.66 min (the same conditions as described for Example **8**)

### Example 12

### Compound Ia: 2-(2-benzofuran-2-yl-benzylsulfanyl) acetic acid ethyl ester

To a solution of compound **7** (3.18 g, 14.2 mmol) in methylenechloride (30 ml), were added at RT ethyl thioglycolate (1.75 g, 14.6 mmol) and ZnI₂ (4.7 g, 14.7 mmol) to give a suspension. The reaction was stirred at RT for 1 hr, quenched by water, the organic phase was dried over Na₂SO₄, evaporated, the residue was purified by flash chromatography (cyclohexane /ethyl acetate, 8 / 1) to furnish 3 g of Example **12** as a colorless oil.
1H NMR (400 MHz, CHCl₃) δ 1.25 (3H, t), 3.25 (2H, s), 4.13 (2H, q), 4.25 (2H, s), 7.3 (2H, m), 7.4 (1H, t), 7.5 (3H, t), 7.75 (1H, d), 7.86 (2H, d).

### Example 13

### Compound Ia: wherein Ar is 3-phenyl-1,4 benzodioxin, Y is CH₂,q is 0, substitution in position 2 and R¹ is COOCH₃.

To a solution of (3-phenyl-1,4-benzodioxin-2-yl)methanol (compound **C;** 7.60 g, 31.6 mmol) in dichloroethane (200 mL) under N₂, was added successively methyl thioglycolate (3.35 g, 31.6 mmol) and ZnI₂ (10.08 g, 31.6 mmol). The mixture was stirred at room temperature for 2h30 and then 60 mL of water was added. After decantation, the aqueous layer was extracted with dichloromethane (100 mL). The organic layers were dried over MgSO₄ and concentrated to furnish 10.2 g of Example **13** (yellow oil).
Yield = 98%,
¹H-NMR (400 MHz, CDCl₃) : δ 3.35 (2H, s), 3.47 (2H, s), 3.59 (3H, s), 6.7-6.9 (4H, m), 7.3-7.6 (5H,m).

### 4) Synthesis of compounds Ib

### Example 14

### Compound Ib: 2-(dibenzofuran-2-ylmethylsulfinyl)acetic acid

To a solution of Exemple **2** (13.6 g, 50 mmol) in acetic acid (130 mL), was added 30% H₂O₂ (8 mL, 79 mmol). The mixture was stirred at room temperature for 3 h. the obtained suspension was filtered, washed with acetic acid (50 mL) and methanol (50 mL), dried under vacuum to afford 12.5 g of Example **14** as a white solid.
1H NMR (400 MHz, DMSO-d₆) δ 3.6 (1H, d), 3.9 (1H, d), 4.25 (1H, d), 4.45 (1H, d), 7.45 (1H, t), 7.5 (1H, d), 7.65 (1H, t), 7.75 (2H, m), 8.1 (1H, s), 8.2 (1H, d).

### 5) Synthesis of compounds Ic

### Example 15

### Compound Ic wherein Ar is dibenzofuran -2-yl, Y is CH₂, q is 0, NR¹²R¹³ = 4-(2-hydroxyethyl)piperazin-1-yl.

To a mixture of Example **2** (0.5 g , 1.8 mmol), N-(2-hydroxyethyl)piperazine (0.25 g, 1.9 mmol), HOBt (0.25 g, 18.5 mmol) in 50 ml methylenechloride was added EDCI (0.46 g, 2.4 mmol) at RT. The reaction was maintained for 16 h, then washed with water, dried over MgSO₄, evaporated, the residue was chromatographied (methylenechloride / methanol, 10 / 1) to furnish 0.5 g of Example **16** as a colorless oil which crystallized on stand.
1H NMR (400 MHz, CHCl₃) δ 2.5 (6H, m), 3.2 (2H, s), 3.45 (2H, m), 3.65 (4H, m), 4.0 (2H, s), 7.35 (1H, t), 7.5 (4H, m), 7.95 (1H, d), 7.98 (1H, s).

### Example 16

### Compound Ic: 2-(dibenzofuran-2-ylmethylsulfanyl)acetamide

A mixture of Exemple **8** (3.7 g, 12.9 mmol) in methanol (100 ml) and 28% aqueous ammonia (50 ml) was stirred at RT for 16 hr to give a suspension that was filtered, washed by water, dried in vacuum to furnish 2.7 g of Example **16** as a white solid.
1H NMR (400 MHz, DMSO-d₆) δ 3.04 (2H, s), 4 (2H, s), 7.03 (1H, bs), 7.6 (4H, m), 7.7 (2H, dd), 8.08 (1H, s), 8.13 (1H, d).

### Example 17

### Compound Ic: 2-(8-methoxy-dibenzofuran-2-ylmethylsulfanyl)acetamide

A mixture of Example **11** (1.1 g, 3.3 mmol) in ethanol (20 ml) and 28% aqueous ammonia (30 ml) was stirred at RT for 18 hr to give a suspension that was filtered, washed by water, dried in vacuum to furnish 0.71 g ofExample **17** as a white solid.
HPLC: Ret. Time = 11.68 min. (the same conditions as described for Example **8**)

### Example 18

### Compound Ic: 2-(dibenzothiophen-2-ylmethylsulfanyl)acetamide

A mixture of Example **9** (2.72 g, 9 mmol) in methanol (50 ml) and 28% aqueous ammonia (30 ml) was stirred at room temperature for 16 hr to give a suspension. After evaporation of methanol, the aqueous residue was filtered, washed by water, dried in vacuum to furnish 2.1 g of Example **18** as a white solid.
1H NMR (400 MHz, DMSO-d₆) δ 3.04 (2H, s), 4 (2H, s), 7.03 (1H, bs), 7.5 (4H, m), 7.91 (2H, d), 8.02 (1H, m), 8.3 (1H, s), 8.33 (1H, m).

### Example 19

### Compound Ic: 2-(8-fluorodibenzofuran-2-ylmethylsulfanyl)acetamide

A mixture of Example **10** (2.34 g, 7.7 mmol) in methanol (50 ml) and 28% aqueous ammonia (30 ml) was stirred at room temperature for 16 hr to give a suspension. The methanol was evaporated and the aqueous residue was filtered, washed by water, dried in vacuum to furnish 1.68 g of Example **19** as a white solid.
1H NMR (400 MHz, DMSO-d₆) δ 3.04 (2H, s), 4 (2H, s), 7.03 (1H, bs), 7.35 (1H, dt), 7.43 (1H, bs), 7.5 (1H, d), 7.67 (1H, d), 7.75 (1H, dd), 8.01 (1H, dd), 8.1 (1H, s).

### Example 20

### Compound Ic: 2-(2-benzofuran-2-yl-benzylsulfanyl)- N,N-dimethyl acetamide

To a mixture of Example **12** (1.12 g, 3.43 mmol) and dimethylamine hydrochloride (0.3 g, 3.68 mmol) in methylenechloride (20 ml) was added at RT a 2 N solution of Al(CH₃)₃ in toluene (2 ml, 4 mmol). The resulting mixture was stirred at RT for 24 hr, then quenched by 0.1 N HCl (20 ml), the organic phase was washed by water, dried over MgSO₄, purified by flash chromatography (Methylenechloride / methanol, 40 / 1) to furnish 1 g of Example **20** as a yellowish oil.
1H NMR (400 MHz, CHCl₃) δ 2.95 (6H, d), 3.4 (2H, s), 4.25 (2H, s), 4.7 (1H, d), 7.3 (2H, m), 7.4 (1H, t), 7.5 (3H, t), 7.75 (1H, dd), 7.88 (2H, d).

### Example 21

### Compound Ic: 2-(8-chlorodibenzofuran-2-yl-methylsulfanyl)-1-[4-(2-hydroxyethyl) piperazin-1-yl] ethanone

To a mixture of Example **3** (1.48 g, 4.8 mmol), 4-(2-hydroxyethyl)-piperazine (0.65 g, 5 mmol) and HOBt (0.65 g, 4.8 mmol) in methylenechloride (50 ml), was added EDCI (1.2 g, 6.25 mmol) at RT. The reaction was maintained at RT for 16 hr, quenched by water, the organic phase was washed by water, dried over Na₂SO₄, evaporated to give 1.92 g of pureExample **21.**
HPLC: Ret. Time = 10.7 min. (the same conditions as described for Example **8**)

### Example 22

### Compound Ic: 4-[2-(8-chlorodibenzofuran-2-yl-methylsulfanyl)acetyl]-piperazine-1-carboxylic acid tret-butyl ester

To a mixture of Example 3 (2 g, 6.5 mmol), 4-(tert-butoxycarbonyl)-piperazine (1.3 g, 7 mmol) and HOBt (1.2 g, 8.9 mmol) in methylenechloride (50 ml), was added EDCI (1.7 g, 8.9 mmol) at RT. The reaction was maintained at RT for 1.5 hr, quenched by water, the organic phase was washed by water, dried over Na₂SO₄, evaporated, the residue was purified by flash chromatography (Methylenechloride / methanol, 40 / 1) to give 2.75 g of Example **22** as a white solid.
1H NMR (400 MHz, CHCl₃) δ 1.5 (9H, s), 3.25 (2H, s), 3.45 (6H, m), 3.59 (2H, m), 4.0 (2H, s), 7.4 (1H, dd), 7.5 (3H, m), 7.96 (2H, d).

### Example 23

### Compound Ic: 1-(4-acetyl-piperazin-1-yl)-2-(8-chlorodibenzofuran-2-yl-methylsulfanyl)-ethanone

To a mixture of acid Example **3** (1.83 g, 6 mmol), 4-(acetyl)-piperazine (0.8 g, 6.3 mmol) and HOBt (1 g, 4.8 mmol) in methylenechloride (50 ml), was added EDCI (1.5 g, 7.8 mmol) at RT. The reaction was maintained at RT for 5 hr, quenched by water, the organic phase was washed by water, dried over Na₂SO₄, evaporated, the residue was purified by flash chromatography (methylenechloride / methanol, 30 / 1) to give 2.14 g of Example **23**.
**HPLC:** Ret. Time = 12.81 min. (the same conditions as described for Example **8**)

### Example 24

### Compound Ic: 2-(9H-fluoren-4-ylmethylsulfanyl)-1-[4-(2-hydroxyethyl)-piperazin-1-yl]-ethanone

To a mixture of acid Example **4** (6 g, 22.2 mmol), 4-(2-hydroxyethyl)-piperazine (3 g, 23 mmol) and HOBt (2.5 g, 18.5 mmol) in methylenechloride (200 ml), was added EDCI (4.6 g, 24 mmol) at RT. The reaction was maintained at RT for 2 hr, quenched by water, the organic phase was washed by water, dried over Na₂SO₄, evaporated, the residue was purified by flash chromatography (methylenechloride / methanol, 10 / 1) to give 6.26 g of Example **24**.
1H NMR (400 MHz, CHCl₃) δ 2.43 (2H, m), 2.5 (4H, m), 3.3 (2H, s), 3.38 (2H, t), 3.63 (4H, m), 3.58 (2H, s), 3.97 (2H, s), 4.28 (2H, s), 7.25 (1H, m), 7.33 (2H, m), 7.41 (1H, t), 7.48 (1H, d), 7.57 (1H, d), 7.88 (1H, d).

### Example 25

### Compound Ic: 4-[2-(dibenzothiophen-2-ylmethylsulfanyl)-acetyl]-piperazine-1-carboxylic acid tert-butyl ester

To a mixture of acid Example **5** (5.76 g, 20 mmol), 4-(tert-butoxycarbonyl)-piperazine (3.75 g, 20.2 mmol) and HOBt (3.4 g, 25 mmol) in methylenechloride (150 ml), was added EDCI (4.8 g, 25 mmol) at RT. The reaction was maintained at RT for 3 hr, quenched by water, the organic phase was washed by 0.5 N HCl, water, dried over Na₂SO₄, evaporated, the residue was purified by flash chromatography (cyclohexane / ethyl acetate, 3 / 4) to give 8.3 g of Example **25** as a white solid.
1H NMR (400 MHz, CHCl₃) δ 1.5 (9H, s), 3.25 (2H, s), 3.45 (6H, m), 3.59 (2H, m), 4.0 (2H, s), 7.45 (3H, m), 7.8 (1H, d), 7.87 (1H, m), 8.16 (1H, s), 8.19 (1H, m).

### Example 26

### Compound Ic: 4-[2-(8-fluorodibenzofuran-2-yl-methylsulfanyl)acetyl]-piperazine-1-carboxylic acid tret-butyl ester

To a mixture of acid Example **6** (5.8 g, 20 mmol), 4-(tert-butoxycarbonyl)-piperazine (3.72 g, 20 mmol) and HOBt (3.4 g, 25 mmol) in methylenechloride (150 ml), was added EDCI (4.8 g, 25 mmol) at RT. The reaction was maintained at RT for 1 hr, quenched by water, the organic phase was washed by 0.5 N HCl, water, dried over Na₂SO₄, evaporated to give a beige solid which was recrystallized in ethyl acetate (30 ml) to give 6.46 g of Example **26** as a beige solid.
1H NMR (400 MHz, CHCl₃) δ 1.5 (9H, s), 3.25 (2H, s), 3.45 (6H, m), 3.59 (2H, m), 4.0 (2H, s), 7.18 (1H, dt), 7.45 (3H, m), 7.61 (1H, dd), 7.92 (1H, s).

### Example 27

### Compound Ic: 2-(3-phenyl-benzo[b]thiophen-2-ylmethylsulfanyl)-1-pyrrolidin-1-yl-ethanone.

To a cooled (ice-bath) solution of Example **7** (2.14g, 6.8mmol) in CH₂Cl₂ (40mL), was added successively pyrrolidine (0.63mL, 7.5mmol), EDCI (1.44g, 7.5mmol) and HOBT (1.012g, 7.5mmol). The cooling bath was removed and the mixture was stirred at room temperature for one night, diluted with CH₂Cl₂ (50mL), washed successively with water (50mL), aqueous Na₂CO₃ (50 mL) water (30mL) and dried over Na₂SO₄. On concentration, the solution generated a crude product that was purified by column chromatography (CH₂Cl₂/CH₃OH 9.6/0.4) to give 1.76g of Example **27** (orange oil).
Yield = 70%,
R_{f} = 0.8(eluent : CH₂Cl₂/CH₃OH 9/1).

The following examples were prepared according to the process as described for Example **27** :

### Example 28

### Compound Ic: N,N-dimethyl-2-(3-phenyl-benzo[b]thiophen-2-ylmethylsulfanyl)-acetamide.

Reagents : Example **7** (2.14g, 6.8mmol) in CH₂Cl₂ (40mL), 40% aqueous dimethylamine (0.337g, 0.85mL, 7.5mmol), EDCI (1.44g, 7.5mmol) and HOBT (1.012g, 7.5mmol).

Example **28** as a yellow orange oil was directly used in the next step without any further purification.
Yield = 96%,
R_{f}= 0.6 (eluent : CH₂Cl₂/CH₃OH 9.5/0.5).

### Example 29

### Compound Ic: N-isopropyl-2-(3-phenyl-benzo[b]thiophen-2-ylmethylsulfanyl)-acetamide.

Reagents : Example **7** (2.14g, 6.8mmol) in CH₂Cl₂ (40mL), isopropylamine (0.44g, 0.65mL, 7.5mmol), EDCI (1.44g, 7.5mmol) and HOBT (1.012g, 7.5mmol).

The crude product was crystallized in diisopropyl oxyde to give 0.62g of Example **29** (white powder)
Yield = 26%.
Rf(CH₂Cl₂/CH₃OH 9/1) = 0.8

### Example 30

### Compound Ic: 1-(4-hydroxy-piperidin-1-yl)-2-(3-phenyl-benzo[b]thiophen-2ylmethylsulfanyl)-ethanone.

Reagents : Example **7** (2.198g, 7mmol) in CH₂Cl₂ (40mL), *N*-hydroxypiperidine (0.788g, 7.7mmol), EDCI (1.474g, 7.7mmol) and HOBT (1.039g, 7.7mmol).
Yield = 39.5%,.1.1g of Example **30** as a yellow oil.
Rf(CH₂Cl₂/CH₃OH 9/1) = 0.5.

### Example 31

### Compound Ic: 1-(4-acetyl-piperazin-1-yl)-2-(3-phenyl-benzo[b]thiophen-2-ylmethylsulfanyl)-ethanone.

Reagents : Example **7** (3.01g, 9.6mmol) in CH₂Cl₂ (58mL), *N*-acetylpiperazine (1.39g, 10.86mmol), EDCI (2.02g, 10.86mmol) and HOBT (1.46g, 10.86mmol).

The crude product that was purified by column chromatography (CH₂Cl₂/CH₃OH 9.5/0.5) to give 1.38g of Example **31** (yellow oil)
Yield = 34.5,.
Rf (CH₂Cl₂/CH₃OH 9.5/0.5) = 0.2.

### Example 32

### Compound Ic N-(2-hydroxy-ethyl)-2-(3-phenyl-benzo[b]thiophen-2-ylmethylsulfanyl)-acetamide.

Reagents : Example **7** (2.36g, 7.51mmol) in CH₂Cl₂ (40mL), ethanolamine (0.506g, 8.3mmol), EDCI (1.59g, 8.3mmol) and HOBT (1.12g, 8.3mmol).

The crude product that was purified by column chromatography (CH₂Cl₂/CH₃OH 9.4/0.6) to give 0.72g of Example **32** as a solid.
Yield = 27%.
Rf (CH₂Cl₂/CH₃OH 9/1) = 0.8

### Example 33

### Compound Ic: 1-[4-(3-Phenyl-benzo[1,4]dioxin-2-ylmethylsulfanylmethyl)-piperazin-1-yl]-ethanone.

Under N₂, to a solution of Example **13** (1.5 g, 4.57 mmol) in dichloromethane (20 mL), were added successively N-acetylpiperazine (0.70 g, 5.48 mmol) in one portion and AlMe₃ 2N in toluene (2.74 mL) dropwise. The mixture was stirred at room temperature for one night and then refluxed for 3h. After cooling, few mL of water was added slowly. After decantation, the organic layer was dried over MgSO₄ and concentrated to afford the crude product which was purified by column chromatography (CH₂Cl₂/MeOH 98/2) to give 0.78 g (yield=40 %) of Example **33** (yellow oil).
¹H-NMR (400 MHz, CDCl₃) : δ 2.11 (3H, d), 3.3-3.7 (12H, m), 6.6-6.9 (4H, m), 7.3-7.6 (5H, m).

### 6) Synthesis of compounds Id

### Example 34

### Compound Id: 4-[2-(dibenzofuran-2-ylmethylsulfinyl)-acetyl]-piperazine-1-carboxylic acid ethyl ester

To a mixture of Exemple **14** (4.32 g , 15 mmol), N-(ethoxycarbony)piperazine (2.5 g, 15.8 mmol) and HOBt (2 g, 15 mmol) in 150 ml methylenechloride, was added EDCI (3.6 g, 18.8 mmol) at RT. The reaction was maintained for 2 h, then washed with 0.5 N HCl (100 ml) and water, dried over Na₂SO₄, evaporated to a white solid that was recrystallized in 15 ml ethyl acetate to afford 3.6 g of Example **34** as a white solid; additional 0.85 g of Example **34** were obtained from the filtrate by flash chromatography (methylenechloride / methanol, 10 / 1).
1H NMR (400 MHz, DMSO-d₆) δ 1.2 (3H, t), 3.35 (4H, m), 3.5 (4H, m), 4.0 (4H, m), 4.2 (1H, d), 4.45 (1H, d), 7.45 (1H, t), 7.5 (1H, d), 7.6 (1H, t), 7.75 (2H, dd), 8.1 (1H, s), 8.2 (1H, d).
MS : M + H = 429, M + Na = 451, M+ K = 467

### 7) Synthesis of compounds Id

### Example 35

### Compound Id: 2-(dibenzofuran-2-ylmethylsulfinyl)-1-[4-(2-hydroxyethyl)-piperazin-1-yl]-ethanone

To a solution of Example **15** (0.5 g, 1.3 mmol) in 20 ml acetic acid, was added 30% H₂O₂ (0.2 ml, 2 mmol) was added. The oxidation was maintained at RT for 18 h, then evaporated, the residue was purified by flash chromatography (methylenechloride / methanol, 9 / 1) to afford 0.39 g of Example **34** as a white solid.
1H NMR (400 MHz, CHCl₃) δ 2.5 (6H, m), 3.4 (2H, m), 3.65 (6H, m), 4.25 (1H, d), 4.5 (1H, d), 7.33 (1H, t), 7.5 (2H, m), 7.6 (1H, d), 7.92 (1H, d), 7.98 (1H, s).
MS:MS:M+H=401,M+Na=423

### Example 36

### Compound Id: 2-(dibenzofuran-2-ylmethylsulfinyl)acetamide

A mixture of Exemple **16** (2.7 g, 10 mmol) in acetic acid (40 ml) and 30% H₂O₂ (1.6 ml) was stirred at RT for 1.5 hr to give a solution that was evaporated to give white solid. The crystallization in ethanol (30 ml) furnished 2.6 g of Example **36** as a white solid.
1H NMR (400 MHz, DMSO-d₆) δ 3.43(1H, d), 3.68 (1H, d), 4.17 (1H, d), 4.43 (1H, d), 7.33 (1H, bs), 7.43 (1H, t), 7.47 (1H, d), 7.54 (1H, t), 7.68 (1H, bs), 7.73 (2H, m), 8.07 (1H, s), 8.17 (1H, d).
MS:M+Na=310

### Example 37

### Compound Id: 2-(8-methoxydibenzofuran-2-yhnethylsulfinyl)acetamide

A mixture of Example **17** (0.71 g, 2.36 mmol) in acetic acid (20 ml) and 30% H₂O₂ (0.45 ml) was stirred at RT for 1 hr to give a solution that was evaporated to give an oil. The crystallization in ethanol furnished 0.48 g of Example **37** as a white solid.
1H NMR (400 MHz, DMSO-d₆) δ 3.46(1H, d), 3.69 (1H, d), 3.87 (3H, s), 4.17 (1H, d), 4.41 (1H, d), 7.1 (1H, dd), 7.33 (1H, bs), 7.46 (1H, d), 7.59 (1H, d), 7.69 (3H, m), 8.06 (1H, s).
MS : M + Na = 340

### Example 38

### Compound Id: 2-(8-fluorodibenzofuran-2-ylmethylsulfinyl)acetamide

A mixture of Example **19** (1.68 g, 5.8 mmol) in acetic acid (30 ml) and 30% H₂O₂ (0.9 ml) was stirred at RT for 3 hr to give a solution that was evaporated to give an oil. The crystallization in ethanol (30 ml) furnished 1.36 g of Example **38** as a white powder.
1H NMR (400 MHz, DMSO-d₆) δ 3.46(1H, d), 3.69 (1H, d), 4.18 (1H, d), 4.43 (1H, d), 7.38 (2H, m), 7.53 (1H, d), 7.75 (3H, m), 8.03 (2H, m), 8.1 (1H, s).
MS : M + Na = 328

### Example 39

### Compound Id: 2-(dibenzothiophen-2-ylmethylsulfinyl)acetamide

A mixture of Example **18** (2.1 g, 7.3 mmol) in acetic acid (40 ml) and 30% H₂O₂ (1.1 ml) was stirred at RT for 2.5 hr to give a solution that was evaporated to give an oil. The crystallization in ethanol (50 ml) furnished 1.45 g of Example **39** as a beige solid.
1H NMR (400 MHz, DMSO-d₆) δ 3.5(1H, d), 3.75 (1H, d), 4.2 (1H, d), 4.48 (1H, d), 7.36 (1H, bs), 7.5 (1H, d), 7.54 (2H, m), 7.75 (1H, bs), 8.03 (2H, d), 8.28 (1H, s), 8.33 (1H, m).
MS : M + Na = 326

### Example 40

### Compound Id: 2-(8-chlorodibenzofuran-2-yl-methanesulfinyl)-[4-(2-hydroxyethyl)piperazin-1-yl] ethanone CEP 25557

A mixture of Example **21** (1.92 g, 4.6 mmol) in acetic acid (40 ml) and 30% H₂O₂ (0.85 ml) was stirred at RT for 2 hr, then evaporated to give a solid which was dissolved in methylenehloride (100 ml) and water (50 ml). The mixture was alkalized to pH 10 by 1 N NaOH. The organic phase was washed by water, dried over Na₂SO₄, purified by flash chromatography (methylenechloride / methanol, 9 / 1) to give 1.2 g Example **40** as a white solid.
1H NMR (400 MHz, CDCl₃) δ 2.5 (6H, m), 3.47(2H, m), 3.66 (5H, m), 3.74 (1H, m), 4.25 (1H, d), 4.5 (1H, d), 7.43 (1H, dd), 7.5 (2H, m), 7.58 (1H, d), 7.92 (2H, d).
MS : M + H = 435, M + Na = 457

### Example 41

### Compound Id: 2-(8-chlorodibenzofuran-2-yl-methanesulfinyl)-1-piperazin-1-ylethanone

A mixture of Example **22** (2.67 g, 5.6 mmol) in methylenechloride (15 ml) and trifluoroacetic acid (7 ml) was stirred at RT for 0.5 h, then evaporated to dryness. The residue was dissolved in 20 ml water and 20 ml methylenechloride, then neutralized to pH 8 by NaHCO₃ powder, the organic phase was washed by water, dried over Na₂SO₄, evaporated to give the deprotected intermediate which was mixed with acetic acid (40 ml) and 30% H₂O₂ (1 ml). The mixture was stirred at RT for 2 hr, evaporated, purified by flash chromatography (methylenechloride / methanol, 10 / 1 saturated by 28% aqueous ammonia) followed by crystallization in 10 ml ethyl acetate to give 1.95 g Example **41** as a white solid.
1H NMR (400 MHz, DMSO-d₆) δ 2.5 (4H, m), 3.25 (4H, m), 3.87 (2H, dd), 4.07 (1H, d), 4.31 (1H, d), 7.43 (2H, m), 7.63 (2H, dd), 8.0 (1H, s), 8.2 (1H, s).
MS : M + H = 391, M + Na = 413

### Example 42

### Compound Id: 1-(4-acetylpiperazin-1-yl)-2-(8-chlorodibenzofuran-2-yl-methanesulfinyl)-ethanone

A mixture of Example **23** (2.1 g, 5 mmol) in acetic acid (30 ml) and 30% H₂O₂ (0.8 ml) was stirred at RT for 2 hr, then diluted with 300 ml water to give a suspension that was heated to give a solution, cooled, filtered, rinsed with water, dried in vacuum to give 1.78 g Example **42** as a white solid.
1H NMR (400 MHz, CDCl₃) δ 2.16 (3H, d), 3.4~3.83 (10H, m), 4.25 (1H, dd), 4.5 (1H, dd), 7.43 (1H, dd), 7.5 (2H, m), 7.59 (1H, d), 7.91 (1H, s), 7.96 (1H, d).
MS : M + H = 433, M + Na = 455

### Example 43

### Compound Id: 2-(9H-fluoren-4-ylmethanesulfinyl)-1-[4-(2-hydroxyethyl)piperazin-1-yl]-ethanone

A mixture of Example **24** (2.9 g, 7.6 mmol) in acetic acid (40 ml) and 30% H₂O₂ (1.2 ml) was stirred at RT for 3 hr, then evaporated. The residue was dissolved in 50 ml water and neutralized at pH 7 by K₂CO₃ powder to give a solution that was extracted methylenechloride (3*50 ml). The extracts were washed by brine, dried over Na₂SO₄, evaporated. Flash chromatography (methylenechloride / methanol, 9 / 1) followed by recrystallization in ethyl acetate / methylenechloride (10 / 1) afforded 2 g Example **43** as a white crystal.
1H NMR (400 MHz, CDCl₃) δ 2.5 (7H, m), 3.43(2H, m), 3.61 (4H, m), 3.69 (1H, d), 3.86 (1H, d), 3.92 (2H, s), 4.61 (1H, d), 4.91 (1H, d), 7.3 (2H, m), 7.4 (2H, m), 7.56 (2H, m), 8.0 (1H, d).
MS:M+H=399,M+Na=421,M+K=437

### Example 44

### Compound Id: 2-(dibenzothiophen-2-yl-methanesulfinyl)-1-piperazin-1-yl-ethanone

A mixture of Example **25** (2 g, 4.4 mmol) in methylenechloride (20 ml) and trifluoroacetic acid (8 ml) was stirred at RT for 0.5 h, then evaporated to dryness. The residue was dissolved in 50 ml water and 20 ml methylenechloride, then neutralized to pH 8 by 0.5 N NaOH, the organic phase was washed by water, dried over Na₂SO₄, evaporated to give the deprotected intermediate which was mixed with acetic acid (30 ml) and 30% H₂O₂ (0.8 ml). The mixture was stirred at RT for 1.5 hr, evaporated, purified by flash chromatography (methylenechloride / methanol, 10 / 1 saturated by 28% aqueous ammonia) to give 0.6 g Example **44** as a white solid.
1H NMR (400 MHz, CDCl₃) δ 2.81 (2H, m), 2.87 (2H, m), 3.36(2H, m), 3.63 (4H, m), 4.28 (1H, d), 4.5 (1H, d), 7.46 (3H, m), 7.86 (2H, m), 8.17 (2H, m).
MS : M + H = 373, M + Na = 395

### Example 45

### Compound Id: 2-(8-fluorodibenzofuran-2-yl-methanesulfinyl)-1-piperazin-1-ylethanone

A mixture of Example **26** (6.4 g, 14 mmol) in methylenechloride (40 ml) and trifluoroacetic acid (20 ml) was stirred at RT for 0.5 h, then evaporated to dryness. The residue was dissolved in 100 ml water and 100 ml methylenechloride, then neutralized to pH 8 by 0.5 N NaOH, the organic phase was washed by water, dried over Na₂SO₄, evaporated to give the deprotected intermediate which was mixed with acetic acid (100 ml) and 30% H₂O₂ (2.5 ml). The mixture was stirred at RT for 2 hr, evaporated, purified by flash chromatography (methylenechloride / methanol, 15 / 1 saturated by 28% aqueous ammonia) and recrystallization in 50 ml ethyl acetate to give 4 g Example **45** as a white solid.
1H NMR (400 MHz, DMSO-d₆) δ 2.63 (4H, m), 3.25~3.5 (4H, m), 3.96 (2H, dd), 4.2 (1H, d), 4.43 (1H, d), 7.36 (1H, dt), 7.5 (1H, d), 7.75 (2H, m), 8.03 (1H, dd), 8.07 (1H, s).
MS : M + H = 375, M + Na = 397

### Example 46

### Compound Id: 2-(2-benzofuran-2-yl-phenylmethanesulfinyl)-N,N-dimethyl acetamide

A mixture of Example **20** (1 g, 3.1 mmol) in acetic acid (10 ml) and 30% H₂O₂ (0.35 ml) was stirred at RT for 4 hr to give a solution that was evaporated. The flash chromatography (methylenechloride / methanol, 20 / 1) furnished 0.71 g of Example **46** as a white solid.
1H NMR (400 MHz, CHCl₃) δ 2.95 (6H, s), 3.9 (2H, dd), 4.47 (1H, d), 4.7 (1H, d), 7.31 (2H, m), 7.43 (1H, m), 7.5 (3H, m), 7.75 (1H, d), 7.93 (2H, d).
MS : M+Na=364.

### Example 47

### Compound Id: 2-(3-phenyl-benzo[b]thiophen-2-ylmethanesulfinyl)-1-pyrrolidin-1-yl-ethanone.

To a solution of Example **27** (1.76g, 4.8mmol) in glacial acetic acid (5mL), 35% aqueous hydrogen peroxide (0.5mL) was added. The mixture was stirred until no more starting material was detected (TLC). After a 3h-stirring, the reaction mixture was concentrated, the resulting oil was diluted with water and ethyl acetate (50mL). The organic layer was washed successively with water (25mL), aqueous NaHCO₃ (25mL), water (25mL) and dried over Na₂SO₄. On concentration, the solution generated a yellow oil that was purified by column chromatography (CH₂Cl₂/CH₃OH 9.6/0.4) to give 0.638g of Example **47** (white meringue; yield = 35%).
¹H-NMR (DMSO) δ (ppm): 8.05 (d, 1H), 7.6-7.35 (m, 8H), 4.45 (q, 2H), 3.95 (q, 2H), 3.4 (m, 2H), 3.25 (m, 2H) 1.9-1.7 (m, 4H).
MS : M+H=384

The following examples were prepared according to the process as described for Example **47** : following Scheme B Step 3 Pathway E

### Example 48

### Compound Id: N,N-dimethyl-2-(3-phenyl-benzo[b]thiophen-2-ylmethanesulfinyl)-acetamide

Reagents : Example **28** (2.24g, 6.5mmol) in glacial acetic acid (6.5mL) and 35% aqueous hydrogen peroxide (0.66mL).

The crude product that was purified by column chromatography (CH₂Cl₂/CH₃OH 9.6/0.4) to give 0.38g of Example **48** (white meringue; yield = 16.4%).
¹H-NMR (DMSO) δ (ppm): 8.05 (d, 1H), 7.6-7.35 (m, 8H), 4.45 (q, 2H), 4.05 (s, 2H), 2.95 (s, 3H), 2.8 (s, 3H).
MS:M+Na=380,2M+Na=737

### Example 49

### Compound Id: N-isopropyl-2-(3-phenyl-benzo[b]thiophen-2-ylmethanesulfinyl)-acetamide

Reagents : Example **29** (0.62g, 1.8mmol) in glacial acetic acid (5mL) and 35% aqueous hydrogen peroxide (0.2mL).

Solvant evaporation generated a yellow oil that crystallized slowly on standing. The residue was stirred with diisopropyl oxyde, filtered and dried *in vacuo* to give 0.48g of Example **49** (white powder; yield = 72%).
¹H-NMR (DMSO) δ (ppm): 8.2 (d, 1H), 8.05 (d, 1H), 7.55-7.35 (m, 8H), 4.4 (q, 2H), 3.8 (h, 1H), 3.65 (q, 2H), 1 (t, 6H).
MS : M+Na=394, M+K=410

### Example 50

### Compound Id: -(4-hydroxy-piperidin-1-yl)-2-(3-phenyl-benzo[b]thiophen-2ylmethanesulfinyl) ethanone

Reagents : Example **30** (1.1g, 2.77mmol) in glacial acetic acid (3mL) and 35% aqueous hydrogen peroxide (0.3mL).

The crude product that was purified by column chromatography (CH₂Cl₂/CH₃OH 9.5/0.5) to give 0.625g of Example **50** (white meringue; yield = 56%).
¹H-NMR (DMSO) δ (ppm): 8.05 (d, 1H), 7.6-7.35 (m, 8H), 4.75 (t, 1H), 4.4 (q, 2H), 4.2-4 (m, 2H),3.85 (m, 1H), 3.75-3.55 (m, 2H), 3.2 (m, 1H), 3.05 (m, 1H), 1.7 (m, 2H), 1.4 (m, 1H), 1.25 (m, 1H).
MS:M+H=414

### Example 51

### Compound Id: 1-(4-acetyl-piperazin-1-yl)-2-(3-phenyl-benzo[b]thiophen-2-ylmethanesulfinyl)-ethanone

Reagents : Example **31** (1.38g, 3.25mmol) in glacial acetic acid (4mL) and 35% aqueous hydrogen peroxide (0.34mL).

The crude product that was purified by column chromatography (CH₂Cl₂/CH₃OH 9.2/0.8) to give 1.01g of Example **50** (white meringue; yield = 70%).
¹H-NMR (DMSO) δ (ppm): 8.05 (d, 1H), 7.6-7.35 (m, 8H), 4.45 (q, 2H), 4.15 (q, 2H), 3.4 (m, 8H), 2 (s, 3H).
MS : M + Na = 463

### Example 52

### Compound Id N-(2-hydroxy-ethyl)-2-(3-phenyl-benzo[b]thiophen-2-ylmethanesulfinyl)-acetamide

### Reagents : Example 32 (0.72g, 2mmol) in glacial acetic acid (3mL) and 35% aqueous hydrogen peroxide (0.23mL).

The crude product was purified by column chromatography (CH₂Cl₂/CH₃OH 9.2/0.8) to give after washing in diisopropyl oxyde 0.478g of Example **52** (white powder; yield = 64%).
¹H-NMR (DMSO) δ (ppm): 8.25 (t, 1H), 8.05 (d, 1H), 7.6-7.35 (m, 8H), 4.7 (t, 1H), 4.4 (q, 2H), 3.7 (q, 2H), 3.4 (m, 2H), 3.15 (m, 2H)
MS : M + Na = 396

### Example 53

### Compound Id: 1-[4-(3-Phenyl-benzo[1,4]dioxin-2-ylmethanesulfinylmethyl)-piperazin-1-yl]-ethanone.

To a solution of Example **33** (0.78 g, 1.84 mmol) in 3.6 mL of acetic acid, was added 30% aqueous hydrogen peroxide (0.20 mL). After 4 h of stirring at room temperature, the mixture was neutralized with aqueous NaHCO₃ and then extracted with CH₂Cl₂ (2x70 mL). The organic layer was dried over MgSO₄ and concentrated to afford the crude product which was purified by column chromatography (CH₂Cl₂/MeOH 98/2) to give 0.60 g (yield=74 %) of Example **53** (white powder).
¹H-NMR (400 MHz, CDCl₃) : δ 2.10 (3H, d), 3.3-4.1 (12H, m), 6.6-6.9 (4H, m), 7.3-7.7 (5H, m).
MS:M+Na=463;M+K=479

Examples **54** throught **150** were prepared following the same multistep general method as described in **schema B** utilizing the appropriate substituted amine - NR¹²R¹³ in Steps 2 or 3. The analytical data as well as the synthetic pathway used are presented by each compounds molecular formula and masse spectrum (M+H) or (M+Na) are shown in the following Table 2.

**Table 2**

| **Example n°** | **MF** | **MS** | **SYNTHETIC PATHWAY** |
|---|---|---|---|
| 54 | C₁₇H₁₇NO₃S | M+H = 316 | A |
| | | M+Na = 338 | |
| 55 | C₁₉H₁₉NO₃S | M+H = 342 | A |
| | | M+Na = 364 | |
| 56 | C₁₈H₁₉NO₃S | M+H = 330 | A |
| | | M+Na = 352 | |
| 57 | C₁₉H₂₀N₂O₃S | M+H = 357 | C |
| | | M+Na = 379 | |
| 58 | C₂₁H₂₂N₂O₄S | M+H = 399 | C |
| | | M+Na = 421 | |
| 59 | C₁₇H₁₇NO₄S | M+Na = 354 | C |
| 60 | C₂₀H₂₁NO₄S | M+H = 372 | C |
| | | M+Na = 394 | |
| 61 | C₁₉H₂₁NO₅S | M+Na = 398 | C |
| | | M+K = 414 | |
| 62 | C₂₀H₂₀N₂O₄S | M+H = 385 | C |
| | | M+Na = 407 | |
| | | M+K = 423 | |
| 63 | C₂₄H₂₈N₂O₅S | M+H = 457 | D |
| | | M+Na = 479 | |
| 64 | C₂₀H₂₂N₂O₃S | M+H = 371 | D |
| | | M+Na = 393 | |
| 65 | C₂₁H₂₄N₂O₃S | M+H = 385 | D |
| | | M+Na = 407 | |
| 66 | C₂₂H₂₆N₂O₃S | M+H = 399 | D |
| | | M+Na = 421 | |
| 67 | C₁₉H₂₀N₂O₄S | M+H = 373 | D |
| | | M+Na = 395 | |
| | | M+K=411 | |
| 68 | C₁₉H₂₁NO₄S | M+H = 360 | D |
| | | M+Na = 382 | |
| | | M+K = 398 | |
| 69 | C₁₉H₁₉NO₄S | M+H = 358 | D |
| | | M+Na = 380 | |
| | | M+K = 396 | |
| 70 | C₁₉H₁₈N₂O₄S | M+H = 371 | D |
| 71 | C₂₃H₂₇N₃O₄S | M+H = 442 | D |
| | | M+Na = 464 | |
| 72 | C₂₀H₂₁N₃O₄S | M+H = 400 | D |
| 73 | C₂₄H₂₇N₃O₄S | M+H = 454 | D |
| 74 | C₂₂H₂₅N₃O₄S | M+H = 428 | D |
| 75 | C₂₇H₂₆N₂O₅S | M+H = 491 | D |
| 76 | C₁₆H₁₅NO₃S | M+H = 302 | C |
| 77 | C₂₀H₂₂N₂O₃S | M+H = 371 | C |
| | | M+Na = 393 | |
| 78 | C₂₂H₂₄N₂O₄S | M+H = 413 | D |
| 79 | C₂₂H₂₄N₂O₄S | M+H = 413 | D |
| | | M+Na = 435 | |
| 80 | C₂₁H₂₄N₂O₃S | M+H = 385 | C |
| 81 | C₂₂H₂₆N₂O₃S | M+H = 399 | C |
| 82 | C₂₁H₂₄N₂O₄S | M+H = 401 | C |
| 83 | C₁₄H₁₂O₂S | M+H = 245 | |
| | | M+Na = 267 | |
| 84 | C₁₉H₁₉N₂O₃SCl | M+H = 391 | E |
| | | M+Na = 413 | |
| 85 | C₂₁H₂₁N₂O₄SCl | M+H = 433 | E |
| | | M+Na = 455 | |
| 86 | C₁₅H₁₂NO₃SCl | M+Na = 344 | E |
| 87 | C₂₀H₂₂N₂O₄S | M+H = 387 | E |
| | | M+Na = 409 | |
| 88 | C₂₂H₂₄N₂O₅S | M+Na = 451 | E |
| | | M+K = 467 | |
| 89 | C₂₁H₂₁N₂O₄SF | M+H = 417 | E |
| | | M+Na = 439 | |
| 90 | C₁₅H₁₂NO₃SCl | M+Na = 344 | E |
| 91 | C₁₅H₁₂NO₃SF | M+Na = 328 | E |
| 92 | C₂₁H₂₁N₂O₄SCl | M+H = 433 | E |
| | | M+Na = 455 | |
| 93 | C₂₁H₂₁N₂O₄SF | M+H = 417 | E |
| | | M+Na = 439 | |
| 94 | C₂₁H₂₀N₂O₄SClF | M+Na = 362 | E |
| | | 2M+Na = 701 | |
| 95 | C₁₇H₁₇NO₄S | M+Na = 354 | E |
| 96 | C₁₅H₁₃NO₃S | M+Na = 310 | E |
| 97 | C₂₁H₂₂N₂O₄S | M+Na = 421 | E |
| | | M+K = 437 | |
| 98 | C₂₀H₂₁NO₄S | M+Na = 394 | E |
| | | M+K = 410 | |
| 99 | C₁₉H₂₀N₂O₃S | M+H = 357 | E |
| 100 | C₂₁H₂₄N₂O₃S | M+H = 385 | E |
| 101 | C₂₂H₂₄N₂O₄S | M+H = 413 | F |
| | | M+Na = 435 | |
| 102 | C₂₂H₂₆N₂O₃S | M+H = 399 | E |
| 103 | C₂₁H₂₄N₂O₄S | M+H = 401 | E |
| 104 | C₂₁H₂₄N₂O₄S | M+H = 401 | E |
| | | M+Na = 423 | |
| 105 | C₁₉H₂₀N₂O₃S | M+H = 357 | E |
| | | M+Na = 379 | |
| 106 | C₁₉H₂₀N₂O₃S | M+H = 357 | E |
| | | M+Na = 379 | |
| 107 | C₂₁H₂₂N₂O₄S | M+H = 399 | E |
| | | M+Na = 421 | |
| 108 | C₂₁H₂₂N₂O₃S₂ | M+H = 415 | E |
| | | M+Na = 437 | |
| 109 | C₂₂H₂₄N₂O₄S₂ | M+H = 445 | E |
| | | M+Na = 467 | |
| 110 | C₂₁H₂₂N₂O₃S₂ | M+H = 415 | E |
| | | M+Na = 437 | |
| 111 | C₁₈H₁₉NO₃S | M+H = 330 | E |
| | | M+Na = 352 | |
| 112 | C₂₂H₂₄N₂O₃S | M+H = 397 | E |
| | | M+Na = 419 | |
| 113 | C₂₁H₂₃NO₃S | M+H = 370 | E |
| | | M+Na = 392 | |
| 114 | C₁₆H₁₅NO₂S | M+Na= 308 | E |
| | | M+K = 324 | |
| 115 | C₂₂H₂₆N₂O₃S | M+H = 399 | E |
| | | M+Na = 421 | |
| 116 | C₂₀H₂₂N₂O₂S | M+H = 355 | E |
| | | M+Na = 377 | |
| 117 | C₁₆H₁₅NO₂S | M-H = 284 | E |
| | | M+Na = 308 | |
| 118 | C₁₈H₁₉NO₂S | M+H= 314 | E |
| | | M+Na = 336 | |
| 119 | C₂₀H₂₁NO₂S | M+H= 340 | E |
| | | M+Na = 362 | |
| 120 | C₁₉H₂₁NO₂S | M+H= 328 | E |
| | | M+Na = 350 | |
| 121 | C₁₈H₁₉NO₃S | M+Na= 352 | E |
| | | M+K = 368 | |
| 122 | C₂₁H₂₃NO₃S | M+H= 370 | E |
| | | M+Na = 392 | |
| 123 | C₂₂H₂₄N₂O₃S | M+Na = 419 | E |
| 124 | C₁₆H₁₅NO₂S | M+H = 286 | E |
| 125 | C₁₈H₁₉NO₂S | M+H = 314 | E |
| 126 | C₁₉H₂NO₂S | M+H = 328 | E |
| 127 | C₁₈H₁₉NO₃S | M+Na = 352 | E |
| 128 | C₂₁H₂₃NO₃S | M+H = 370 | E |
| | | M+Na= 392 | |
| 129 | C₂₂H₂₄N₂O₃S | M+H = 397 | E |
| | | M+Na= 419 | |
| 130 | C₂₀H₂₂N₂O₂S | M+H = 355 | E |
| | | M+Na= 377 | |
| 131 | C₂₁H₂₂N₂O₃S | M+H = 383 | E |
| | | M+Na= 405 | |
| | | M+K = 421 | |
| 132 | C₁₇H₁₅NO₃S | M+Na = 336 | G |
| 133 | C₂₁H₂₁NO₃S | M+H = 368 | G |
| | | 2M+Na = 757 | |
| 134 | C₁₈H₁₇NO₃S | M+Na=350 | G |
| 135 | C₂₂H₂₃NO₃S | M+H = 382 | G |
| | | M+Na = 404 | |
| 136 | C₂₀H₂₁NO₃S | M+H = 356 | G |
| | | M+Na = 378 | |
| 137 | C₂₀H₂₁NO₃S | M-H = 354 | G |
| | | M+Na = 378 | |
| 138 | C₂₁H₂₃NO₃S | M+H = 370 | G |
| | | M+Na = 392 | |
| 139 | C₂₃H₂₄N₂O₄S | M+H = 425 | G |
| | | M+Na = 427 | |
| | | M+K = 463 | |
| 140 | C₂₄H₂₆N₂O₄S | M+Na = 461 | G |
| 141 | C₁₇H₁₅NO₂S₂ | M+Na = 352 | G |
| 142 | C₂₀H₂₁NO₄S | M+Na = 394 | G |
| | | M+K = 410 | |
| 143 | C₁₇H₁₅NO₄S | M+Na = 352 | G |
| | | 2M+Na = 681 | |
| 144 | C₁₇H₁₃Cl₂NO₄S | M+Na = 420 | G |
| | | 2M+Na = 817 | |
| 145 | C₂₃H₂₂Cl₂N₂O₅S | M+Na = 531 | G |

### Example 146

### Compound Id: 1-(4-acetyl-piperazin-1-yl)-2-(8-chloro-dibenzofuran-2-ylmethanesulfonyl)-ethanone.

To a solution of Example **23** (1 g, 2.4 mmol) in acetic acid (15 ml) and TFA (1.5 ml), was added 30% H₂O₂ (0.8 ml, 7.8 mmol). The mixture was stirred at 50°C for 5 h, then evaporated to dryness. Water (100 ml) was added and heated at 80°C to give a suspension that was filtered while hot, rinsed by water, dried in vacuum at 50°C. The crude product was recrystallized in acetonitrile (30 ml) and water (5 ml) to give 0.79 g of Example **146** as a white solid.
1H NMR (400 MHz, DMSO-d₆) δ 2.0 (3H, s), 3.5(8H, m), 4.5 (2H, d), 4.8 (2H, d), 7.65 (2H, t), 7.75 (2H, m), 8.2 (1H, s), 8.35 (1H, s).
MS : M + Na = 471

### Were also synthetized according to Pathway G (Scheme B):

### Example 147

### Compound Ia: Ethyl 2-{[(3-phenyl-1H-indol-2-yl)methyl]sulfanyl}acetate

A 250mL round-bottom flask containing a magnetic stirring bar equipped with a reflux condenser was charged with 1.6g (0.00717mol) of compound 62 , 0.8mL (0.00717mol) of thioglycolic acid and 50mL of 1,2-dichloroethane. At 0°C, 1.4mL (0.0107mol) of BF₃.Et₂O dissolved in 10mL of 1,2-dichloroethane was added slowly. The resulting mixture was stirred for 15 minutes and then 100mL of water and 10mL of HCl (1N) were added. The product was extracted with 2x50mL of ethyl acetate. The organic layer was dried over magnesium sulfate, filtered and evaporated to dryness. The crude product was used for the next step (preparation of Example **148**).

### Example 148

### Compound Ic: 2-{[(3-phenyl-1H-indol-2-yl)methyl]sulfanyl}acetamide

A 250mL round-bottom flask containing a magnetic stirring bar equipped with a reflux condenser was charged with the crude ester Example **147** , 100mL of ethanol and 50mL of an aqueous solution of NH₃ (28%). The mixture was stirred for 5 days and then evaporated to dryness. After chromatographic purification (ethyl acetate / petroleum ether: 9/1), we obtained 0.6g (296,38g.mol⁻¹) of Example **148**.
1H NMR (400 MHz, CDC13) δ 3.16 (2H, s) 4.03 (2H, s) 5.52 (1H, bs) 5.93 (1H, bs) 7.12 (1H, m) 7.20 (1H, m) 7.25 (1H, m) 7.39 (2H, m) 7.42 (2H, m) 7.54 (1H, m) 8.29 (1H, bs).

### Example 149

### Compound Id : 2-{[(3-phenyl-1H-indol-2-yl)methyl]sulfinyl}acetamide.

A 100mL round-bottom flask containing a magnetic stirring bar equipped with a reflux condenser was charged with 0.6g (0.00202mol) of Example **148**, 50mL of methanol, 10mL of water and 0.48g (0.00223mol) of sodium periodate. The reaction mixture was stirred for 16 hours at 0°C. After evaporation to dryness, the resulting mixture was treated with 100mL of water, triturated for 30 minutes, filtered and dried. Were obtained 0.50g (312.38g.mol⁻¹) of the expected Example **149**.
Yield: 79%.
1H NMR (400 MHz, CDCl3) δ 3.34 (2H, s) 3.67 (1H, dd) 4.34 (1H, dd) 7.04 (1H, m) 7.16 (1H, m) 7.32 (1H, m) 7.39 (1H, bs) 7.47 (3H, m) 7.55 (1H, m) 7.75 (2H, m) 11.45 (1H, bs). m/z 335 [M+Na⁺], m/z 351 [M+K⁺].

### Biological data

### Methodology: Evaluation of Wake Promoting Activity in Rats

The methodology utilized for evaluating wake promoting activity of test compounds is based on that described by Edgar and Seidel, *Journal of Pharmacology and Experimental Therapeutics,* 283:757-769, 1997, and incorporated herein in its entirety by reference.

**Animal Surgery.** Adult, male Wistar rats (275-320g from Charles River Laboratories, Wilmington, MA) were anesthetized (Nembutal, 45 mg/kg, ip.) and surgically prepared with implants for recording of chronic EEG (encephalographic) and EMG (electromyographic) recording. The EEG implants were made from commercially available components (Plastics One, Roanoke, VA). EEG signals were recorded from stainless steel screw electrodes: 2 frontal (+3.0 mm AP from bregma, ±2.0 mm ML), and 2 occipital (-4.0 mm AP from bregma, ±2.0 mm ML). Two Teflon-coated stainless steel wires were positioned under the nuchal trapezoid muscles for EMG recording. All electrode leads were inserted into a connector pedestal and the pedestal affixed to the skull by application dental acrylic. Antibiotic was administered post surgically and antibiotic cream was applied to the wound edges to prevent infection. At least one week elapsed between surgery and recording.

**Recording environment.** Postsurgically, rats were housed in pairs in an isolated room. Food and water were available *ad libitum,* ambient temperature was 21°C, and humidity was 55%. At least 24 hrs prior to recording, they were placed in Nalgene containers (31 x 31 x 31 cm) with a wire-grid top, and entry to the room was prohibited during the day of recording except for dosing. The containers were placed on a rack with two shelves, 4 containers per shelf. Fluorescent overhead room lights were set to a 24 hr. light/dark cycle (on at 7 AM, off at 7 PM). Light levels inside the containers were 38 and 25 lux for the top and bottom shelves respectively. Background white-noise (68db inside the containers) was present in the room to mask ambient sounds.

**Data acquisition**. EEG and EMG signals were led via cables to a commutator (Plastics One) and then to pre-amplifiers (model 1700, A-M Systems, Carlsborg, WA). EEG and EMG signals were amplified (10K and 1K respectively) and bandpass filtered between 0.3 and 500 Hz for EEG and between 10 and 500 Hz for EMG. These signals were digitized at 128 samples per second using ICELUS sleep research software (M. Opp, U. Texas; see Opp, Physiology and Behavior 63:67-74, 1998, and Imeri, Mancia, and Opp, *Neuroscience* 92:745-749, 1999, incorporated by reference herein in their entirety) running under Labview 5.1 software and data acquisition hardware (PCI-MIO-16E-4; National Instruments, Austin, TX). On the day of dosing, data was recorded for 6 to 10 hours beginning at 11 AM.

**Drug administration and study design**. Compounds were evaluated on groups of from 4 to 8 rats carried out over one or two separate test sessions. Each animal was tested with a different compound or vehicle for up to 10 weeks with at least 7 days between successive tests. A vehicle group was included in all experiments, and each animal received vehicle every 4^{th} test. Test compounds were suspended in sterile 0.25% methylcellulose (pH=6.2; Upjohn Co., Kalamazoo, MI) at 30 mg/mL. Although compounds can be administered at dosages greater than 100 mg/kg and are expected to be active under the selection criteria of data analysis, unless otherwise noted, compounds were administered at a single dose of 100 mg/kg. Dosing was carried out at noon, while the rats were predominantly asleep. Each rat was lifted out of its container, given an intraperitoneal injection in a volume of 5 mL/kg, and replaced. Dosing required approximately 30 sec per rat.

**Sleep / wake scoring.** Sleep and wake activity were determined manually using ICELUS software. This program displays the EEG and EMG data in blocks of 6 sec along with the EEG frequency spectrum. Arousal state was scored as awake, rapid eye-movement (REM), or slow-wave or non-REM sleep (NREM) according to visual analysis of EEG frequency and amplitude characteristics and EMG activity (Opp and Krueger, 1994; Van Gelder, *et al*., 1991; Edgar, *et al*., 1991, 1997; Seidel, *et al*, 1995, incorporated by reference herein in their entirety). Essentially, waking activity consists of relatively low-amplitude EEG activity with relatively lower power in the frequency band from 0.5 - 6 Hz, accompanied by moderate to high level EMG activity. In a particular waking state ("theta-waking"), EEG power can be relatively focused in the 6-9 Hz (theta) range, but significant EMG activity is always present. NREM sleep is characterized by relative high-amplitude EEG activity with relatively greater power in the low frequency band from 0.5 - 6 Hz, accompanied by little or no EMG activity. REM sleep is characterized by moderate and constant amplitude EEG focused in the theta (6-9 Hz) range, similar to waking theta, but with no EMG activity.

**Data analysis and statistics**. Two basic outcome measures were used to ascertain whether a compound exhibited wake-enhancing activity. The first was the percent time spent awake for each 30 min period following dosing. The second was the total time spent awake in the first 3 hrs following dosing (3 hr AUC; maximum 180 min). For purposes of ascertaining activity of a test compound, wake activity values were compared against corresponding vehicle values. The vehicle values were of two types. The first type was the corresponding within-experiment vehicle, that is, a value for the vehicle group run concurrently with the test compound. A second "reference" vehicle value consisted of the mean 3 hr AUC value calculated from 234 animals in 59 separate experiments carried out during the same time period as the evaluations of the test compounds (mean ± SD = 69.22 ± 20.12; 95% confidence limits = 66.63 - 71.81). Two-tailed, unpaired t-tests were performed on the wake time values for drug versus vehicle treated animals, and compounds with p ≤0.05 were deemed significantly wake-promoting. A test compound was considered "active" if it met one of the following three criteria.
(i) The 3 hr AUC value for the test compound was significantly greater (p ≤0.05) than the mean wake value for the reference vehicle group (N = 234).
(ii) The 3 hr AUC value for the test compound was significantly greater (p ≤0.05) than the corresponding value for the vehicle group within the same experiment.
(iii) One or more of the half-hour wake time values from 0.5 to 2 hrs after dosing was significantly greater (p ≤0.05) in the test compound group than in the corresponding vehicle group within the same experiment.

### Results :

Compounds of the inventionn either have demonstrated or are expected to demonstrate utility for wake promoting activity.

As example, the three-hours AUC values (mean ± sem) for the reference vehicle group and for the test compounds are reported Table 3 for Examples 26, 99 and 130. These test compounds were administered by i.p. route at a 100 mg/kg dose and the time-course of the percent of time awake as function of time was estimated from 1 hr prior to 5 hours post dosing.

**Table 3:**

| Mean AUC 0-3h values (± sem) for the reference vehicle group and for test compounds | | | | | |
|---|---|---|---|---|---|
| | Vehicle | | Test compound | | p |
| | Mean | sem | Mean | sem | |
| Example 26 | 73.6 | 7.7 | 132.0 | 13.2 | 0.002 |
| Example 99 | 53.0 | 3.3 | 129.1 | 17.3 | 0.022 |
| Example 130 | 76.2 | 17.5 | 149.2 | 2.9 | 0.006 |
| AUC 0-3h (% of waiking time x hr) - n = 4 Rats per test compound and 8 rats per control groups. | | | | | |

As compared to the control groups, compounds of Example 26, 99 and 130 produced a significantly greated wakefulness than that observed in the vehicle-treated animals (p< 0.05).

**References.** The following references, to the extent that they provide exemplary procedural or other details supplementary to those set forth herein, are specifically incorporated in their entirety herein by reference:
Touret, *et al., Neuroscience Letters,* 189:43-46, 1995.
Van Gelder, R.N. *et al., Sleep* 14:48-55, 1991.
Edgar, D.M., *J. Pharmacol. Exp. Ther*. 282:420-429, 1997.
Edgar and Seidel, *J. Pharmacol. Exp. Ther.,* 283:757-69, 1997.
Hernant *et al., Psychopharmacology*, 103:28-32, 1991.
Lin *et al., Brain Research*, 591:319-326, 1992.
Opp and Krueger, *American Journal of Physiology* 266:R688-95, 1994
Panckeri *et al., Sleep,* 19(8):626-631, 1996.
Seidel, W.F., *et al., J . Pharmacol. Exp. Ther*. 275:263-273, 1995.
Shelton *et al., Sleep* 18(10):817-826, 1995.
Welsh, D.K., *et al., Physiol. Behav*. 35:533-538, 1985.

### Utility

The present invention provides a method of treating diseases and conditions in a subject in need thereof comprising administering to said subject a therapeutically effective amount of a compound of formula (I). For example, the compounds of of the present invention are use in the treatment of diseases, including treatment of sleepiness, promotion of wakefulness, treatment of Parkinson's disease, cerebral ischemia, stroke, sleep apneas, eating disorders, stimulation of appetite and weight gain, treatment of attention deficit hyperactivity disorder ("ADHD"), enhancing function in disorders associated with hypofunctionality of the cerebral cortex, including, but not limited to, depression, schizophrenia, fatigue, in particular, fatigue associated with neurologic disease, such as multiple sclerosis, chronic fatigue syndrome, and improvement of cognitive dysfunction.

### Dosage and Formulation

The compounds of the present invention can be administered for therapeutic purposes by any means that results in the contact of the active agent with the agent's site of action in a subject. The compounds may be administered by any conventional means available for use in conjunction with pharmaceuticals, either as individual therapeutic agents or in a combination with other therapeutic agents, such as, for example, analgesics, or in combination with antidepressants, including but are not limited to tricyclic antidepressants ("TCAs"), Selective Serotonin Reuptake Inhibitors ("SSRIs"), Serotonin and Noradrenaline Reuptake Inhibitors ("SNRIs"), Dopamine Reuptake Inhibitors ("DRIs"), Noradrenaline Reuptake Inhibitors ("NRUs"), Dopamine, Serotonin and Noradrenaline Reuptake Inhibitors ("DSNRIs") and Monoamine Oxidase Inhibitors ("MAOIs) including reversible inhibitors of monoamine oxidase type A (RIMAs). The compounds of the present invention are preferably administered in therapeutically effective amounts for the treatment of the diseases and disorders described herein.

A therapeutically effective amount can be readily determined by the attending diagnostician, as one skilled in the art, by the use of conventional techniques. The effective dose will vary depending upon a number of factors, including the pharmacodynamics of the active agent, the type and extent of progression of the disease or disorder, the age, weight and health of the particular patient, the formulation of the active and its mode and frequency of administration, and the desired effect with a minimization of side effects. Typically, the compounds are administered at lower dosage levels, with a gradual increase until the desired effect is achieved.

Typical dose ranges are from about 0.01 mg/kg to about 100 mg/kg of body weight per day, with a preferred dose from about 0.01 mg/kg to 10 mg/kg of body weight per day. A typical daily dose for adult humans can range from about 1 to about 1000 mg of the active agent, particularly from about 1 to about 400 mg, and including 25, 50, 85, 100, 150, 170, 200, 255, 250, 255, 340, 400, 425, 500, 600, 700, 750, 800, and 900 mg doses, and equivalent doses for a human child.

The compounds may be administered in one or more unit dose forms, and they may be administered in a single daily dose or in two, three or four doses per day. The unit dose ranges from about 1 to about 1000 mg, particlularly from about 1 to about 400 mg, and including 25, 50, 85, 100, 150, 170, 200, 255, 250, 255, 340, 400, 425, 500, 600, 700, 750, 800, and 900 mg unit doses, and equivalent unit doses for a human child. In particular, the unit dosages range from about 1 to about 500 mg administered one to four times a day, preferably from about 10 mg to about 300 mg, two times a day. In an alternate method of describing an effective dose, an oral unit dose is one that is necessary to achieve a blood serum level of about 0.05 to 20 µg/ml in a subject, and preferably about 1 to 20 µg/ml.

The compounds of the present invention may be formulated into pharmaceutical compositions by admixture with one or more pharmaceutically acceptable excipients. The active agent may be present in about 0.5-95% by weight of the composition. The excipients are selected on the basis of the chosen route of administration and standard pharmaceutical practice, as described, for example, in *Remington: The Science and Practice of Pharmacy,* 20^{th} ed.; Gennaro, A. R., Ed.; Lippincott Williams & Wilkins: Philadelphia, PA, 2000.

The compositions can be prepared for administration by oral means, including tablets, pills, powders, capsules, troches and the like; parenteral means, including intravenous, intramuscular, and subcutaneous means; topical or transdermal means, including patches, creams, ointments, lotions, pastes, gels, solutions, suspensions, aerosols, and powders and the like; transmucosal means, including nasal, rectal, vaginal, sublingual and buccal means; ophthalmic or inhalation means. Preferably the compositions are prepared for oral administration, particularly in the form of tablets, capsules or syrups; parenteral administration, particularly in the form of liquid solutions, suspensions or emulsions; intranasal administration, particularly in the form of powders, nasal drops, or aerosols; or for topical use, such as patches, creams, ointments, and lotions.

For oral administration, the tablets, pills, powders, capsules, troches and the like can contain one or more of the following: diluents or fillers such as starch, or cellulose; binders such as microcrystalline cellulose, gelatins, or polyvinylpyrrolidone; disintegrants such as starch or cellulose derivatives; lubricants such as talc or magnesium stearate; glidants such as colloidal silicon dioxide; sweetening agents such as sucrose or saccharin; and flavoring agents such as peppermint or cherry flavoring. Capsules may contain any of the above ingredients, and may also contain a semi-solid or liquid carrier, such as a polyethylene glycol. The solid oral dosage forms may have coatings of sugar, shellac, or enteric agents. Liquid preparations may be in the form of aqueous or oily suspensions, solutions, emulsions, syrups, elixirs, etc., or may be presented as a dry product for reconstitution with water or other suitable vehicle before use. Such liquid preparations may contain conventional additives such as surfactants, suspending agents, emulsifying agents, diluents, sweetening and flavoring agents, dyes and preservatives.

The compositions may also be administered parenterally. The pharmaceutical forms acceptable for injectable use include, for example, sterile aqueous solutions, or suspensions. Aqueous carriers include mixtures of alcohols and water, buffered media, and the like. Nonaqueous solvents include alcohols and glycols, such as ethanol, and polyethylene glycols; oils, such as vegetable oils; fatty acids and fatty acid esters, and the like. Other components can be added including surfactants; such as hydroxypropylcellulose; isotonic agents, such as sodium chloride; fluid and nutrient replenishers; electrolyte replenishers; agents which control the release of the active compounds, such as aluminum monostearate, and various co-polymers; antibacterial agents, such as chlorobutanol, or phenol; buffers; suspending agents; thickening agents; and the like. The parenteral preparations can be enclosed in ampules, disposable syringes or multiple dose vials. Other potentially useful parenteral delivery systems for the active compounds include ethylene-vinyl acetate copolymer particles, osmotic pumps, implantable infusion systems, and liposomes.

Other possible modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for topical use are in the form of an ointment, cream, or gel. Typically these forms include a carrier, such as petrolatum, lanolin, stearyl alcohol, polyethylene glycols, or their combinations, and either an emulsifying agent, such as sodium lauryl sulfate, or a gelling agent, such as tragacanth. Formulations suitable for transdermal administration can be presented as discrete patches, as in a reservoir or microreservoir system, adhesive diffusion-controlled system or a matrix dispersion-type system. Formulations for buccal administration include, for example lozenges or pastilles and may also include a flavored base, such as sucrose or acacia, and other excipients such as glycocholate. Formulations suitable for rectal administration are preferably presented as unit-dose suppositories, with a solid based carrier, such as cocoa butter, and may include a salicylate.

The compositions of the present invention may be formulated to control and/or delay the release of the active agent(s). Such controlled-, delayed, sustained-, or extended-release compositions are well-known in the art, and may include, for example, reservoir or matrix diffusion products, as well as dissolution systems. Some compositions may utilize, for example biocompatible, biodegradable lactide polymer, lactide/glycolide copolymer, or polyoxyethylene-polyoxypropylene copolymers as excipients.

Although the present invention has been described in considerable detail, those skilled in the art will appreciate that numerous changes and modifications may be made to the embodiments and preferred embodiments of the invention and that such changes and modifications may be made without departing from the spirit of the invention. It is therefore intended that the appended claims cover all equivalent variations as fall within the scope of the invention.

## Claims

1. A compound of formula (A): 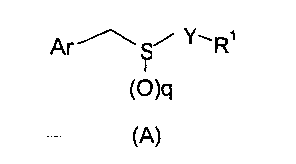 wherein :
Ar is: 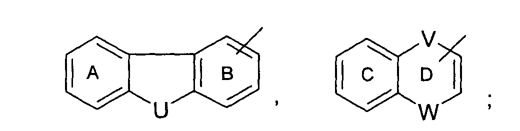 wherein:
U is CH₂, CR²⁵R²⁶, O, S(O)_{y}, NR¹⁰, C(=O), C(=S), CHOH, CHOR¹⁴, C=NOR¹⁴, or C=NNR¹²R¹³;
V and W are independently selected from a bond, CH₂, CR²⁵R²⁶, O, S(O)_{y}, NR¹⁰, C(=O), C(=S), CHOH, CHOR¹⁴, C=NOR¹⁴, or C=NNR¹²R¹³;
rings A, B, and C are optionally substituted with one to three groups selected from F, Cl, Br, I, OR²², OR²⁷, NR²³R²⁴, NHOH, NO₂, CN, CF₃, C₁-C₆ alkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
ring D is optionally substituted with one group selected from C₁-C₆ alkyl, phenyl, and 5-10 membered heteroaryl;
Y is C₁-C₆ alkylene; or
(C₁-C₄ alkylene)ₘ-Z-(C₁-C₄ alkylene)ₙ;
wherein said alkylene groups are optionally substituted with one to three R²⁰ groups;
Z is O, NR^{10A}, S(O)_{y}, CR²¹=CR²¹, C≡C, C₆-C₁₀ arylene, 5-10 membered heteroarylene, C₃-C₆ cycloalkylene, or 3-6 membered heterocycloalkylene; wherein said arylene, heteroarylene, cycloalkylene, and heterocycloalkylene groups are optionally substituted with one to three R²⁰ groups;
R¹ is selected from H, NR¹²R¹³, NR²¹C(=O)R¹⁴, C(=O)R¹⁴, CO₂R¹¹, OC(=O)R¹¹, C(=O)NR¹²R¹³, C(=NR¹¹)NR¹²R¹³, OC(=O)NR¹²R¹³, NR²¹S(O)₂R¹¹, NR²¹C(=O)NR¹²R¹³, NR²¹(SO₂)NR¹²R¹³, and C(=O)NR¹¹ OR²²;
R¹⁰ and R^{10A} are each independently selected from H, C₁-C₆ alkyl, C₆-C₁₀ aryl, C(=O)R¹⁴, and S(O)_{y}R¹⁴; wherein said alkyl and aryl groups are optionally substituted with one to three R²⁰ groups;
R¹¹ at each occurrence is independently selected from H, C₁-C₆ alkyl, and C₆-C₁₀ aryl; wherein said alkyl and aryl groups are optionally substituted with one to three R²⁰ groups;
R¹² and R¹³ at each occurrence are each independently selected from H, C₁-C₆ alkyl, C₆-C₁₀ aryl, and NR²³R²⁴, or R¹² and R¹³, together with the nitrogen to which they are attached, form a 3-7 membered heterocyclic ring;
wherein said alkyl and aryl groups and heterocyclic ring are optionally substituted with one to three R²⁰ groups;
R¹⁴ at each occurrence is independently selected from C₁-C₆ alkyl, C₆-C₁₀ aryl, and alkylaryl; wherein said alkyl, aryl and alkylaryl groups are optionally substituted with one to three R²⁰ groups;
R²⁰ at each occurrence is independently selected from F, Cl, Br, I, OR²², OR²⁷, NR²³R²⁴, NHOH, NO₂, CN, CF₃, C₁-C₆ alkyl optionally substituted with OH, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²², OC(=O)R²², C(=O)NR²³R²⁴, NR²¹C(=O)R²², OC(=O)NR²³R²⁴, NR²¹C(=S)R²², and S(O)_{y}R²²;
R²¹ at each occurrence is independently selected from H and C₁-C₆ alkyl;
R²² at each occurrence is independently selected from H, C₁-C₆ alkyl optionally substituted with OH, arylalkyl and C₆-C₁₀ aryl;
R²³ and R²⁴ at each occurrence are each independently selected from H, C₁-C₆ alkyl, and C₆-C₁₀ aryl, or R²³ and R²⁴, together with the nitrogen to which they are attached, form a 3-7 membered heterocyclic ring optionally substituted with =O;
R²⁵ and R²⁶ at each occurrence are each independently selected from H, C₁-C₆ alkyl, and C₆-C₁₀ aryl, or R²⁵ and R²⁶, together with the carbon to which they are attached, form a 3-7 membered heterocyclic ring;
R²⁷ at each occurrence is independently the residue of an amino acid after the hydroxyl group of the carboxyl group is removed;
m is 0 or 1;
n is 0 or 1;
q is 0, 1, or 2;
y is 0, 1, or 2;
with the exclusion of the compounds wherein:
U is CH₂, C(=O), CH(CH₃), S or C = NNHPh ; and
Y is CH₂ ; and
R¹ isH;
and with the exclusion of the compounds wherein :
U is CH₂ ; and
Y is C₁-C₆ alkylene optionally substituted with C₁-C₆ alkylene ; and
R¹ is CONH₂, or CO₂R¹¹ with R¹¹ = H or C₁-C₆ alkyl ;
and with the exclusion of the compounds :
■ 3-[(methylthio)methyl]-2-phenyl-1H-inden-1-one
■ 3-[(methylsulfinyl)methyl]-2-phenyl-1H-inden-1-one
and the stereoisomeric forms, mixtures of stereoisomeric forms or pharmaceutically acceptable salts forms thereof.

2. The compound according to claim 1, wherein q is 1.

3. The compound according to claim 1 or 2, wherein R¹ is C(=O)NR¹²R¹³, wherein R¹² and R¹³ are each independently selected from H, C₁-C₆ alkyl and NR²³R²⁴ or form together with the nitrogen to which they are attached a 3-7 membered heterocyclic ring, wherein said heterocyclic ring is optionally substituted with one R²⁰ group.

4. The compound according to any of claims 1 to 3, wherein Ar is:

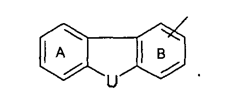

5. The compound according to any of claims 1 to 4, wherein Ar is:

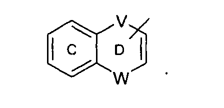

6. The compound according to claims 1 to 5, wherein Y is C₁-C₆ alkylene.

7. The compound according to any of claims 1 to 6 selected in accordance with the following table: 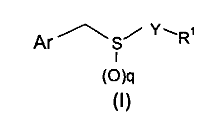 wherein Ar, q, Y-R¹ are defined in the table 1 below ;
| **Ex.n°** | **Ring Ar** | **q** | **Y-R**^{**1**} |
|---|---|---|---|
| 16 | Dibenzofuran-2-yl | 0 | CH₂CONH₂ |
| 36 | Dibenzofuran-2-yl | 1 | CH₂CONH₂ |
| | Dibenzofuran-2-yl | 0 | CH₂CON(CH₃)₂ |
| 54 | Dibenzofuran-2-yl | 1 | CH₂CON(CH₃)₂ |
| | Dibenzofuran-2-yl | 0 | CH₂CO-N-pyrrolidinyl |
| 55 | Dibenzofuran-2-yl | 1 | CH₂CO-N-pyrrolidinyl |
| | Dibenzofuran-2-yl | 0 | CH₂CONHCH(CH₃)₂ |
| 56 | Dibenzofuran-2-yl | 1 | CH₂CONHCH(CH₃)₂ |
| | Dibenzofuran-2-yl | 0 | CH₂CO-1-(4-tert-butoxycarbonyl)-piperazinyl |
| 57 | Dibenzofuran-2-yl | 1 | CH₂CO-1-piperazinyl |
| | Dibenzofuran-2-yl | 0 | CH₂CO-1-(4-acetyl)-piperazinyl |
| 58 | Dibenzofuran-2-yl | 1 | CH₂CO-1-(4-acetyl)-piperazinyl |
| | Dibenzofuran-2-yl | 0 | CH₂CONHCH₂CH₂OH |
| 59 | Dibenzofuran-2-yl | 1 | CH₂CONHCH₂CH₂OH |
| | Dibenzofuran-2-yl | 0 | CH₂CO-1-(4-hydroxy)piperidinyl |
| 60 | Dibenzofuran-2-yl | 1 | CH₂CO-1-(4-hydroxy)piperidinyl |
| | Dibenzofuran-2-yl | 0 | CH₂CONHCH₂CH₂OCH₂CH₂OH |
| 61 | Dibenzofuran-2-yl | 1 | CH₂CONHCH₂CH₂OCH₂CH₂OH |
| 15 | Dibenzofuran-2-yl | 0 | CH₂CO-1-[4-(2-hydroxyethyl)-piperazinyl) |
| 34 | Dibenzofuran-2-yl | 1 | CH₂CO-1-[4-(2-hydroxyethyl)-piperazinyl] |
| | Dibenzofuran-2-yl | 0 | CH₂CO-1-(4-formyl)-piperazinyl |
| 62 | Dibenzofuran-2-yl | 1 | CH₂CO-1-(4-formyl)-piperazinyl |
| 63 | Dibenzofuran-2-yl | 1 | CH₂CO-1-(4-tert-butoxycarbonyl)-piperazinyl |
| 35 | Dibenzofuran-2-yl | 1 | CH₂CO-1-(4-ethoxycarbonyl)-piperazinyl |
| 64 | Dibenzofuran-2-yl | 1 | CH₂CO-1-(4-methyl)-piperazinyl |
| | Dibenzofuran-2-yl | 0 | CH₂CO-1-(4-ethyl)-piperazinyl |
| 65 | Dibenzofuran-2-yl | 1 | CH₂CO-1-(4-ethyl)-piperazinyl |
| | Dibenzofuran-2-yl | 0 | CH₂CO-1-(4-propyl)-piperazinyl |
| 66 | Dibenzofuran-2-yl | 1 | CH₂CO-1-(4-propyl)-piperazinyl |
| | Dibenzofuran-2-yl | 0 | CH₂CON-morpholinyl |
| 67 | Dibenzofuran-2-yl | 1 | CH₂CON-morpholinyl |
| | Dibenzofuran-2-yl | 0 | CH₂CO-N-ethyl-N-(2-hydroxy-ethyl) |
| 68 | Dibenzofuran-2-yl | 1 | CH₂CO-N-ethyl-N-(2-hydroxy-ethyl) |
| | Dibenzofuran-2-yl | 0 | CH₂CONHN-morpholinyl |
| 69 | Dibenzofuran-2-yl | 1 | CH₂CONHN-morpholinyl |
| | Dibenzofuran-2-yl | 0 | CH₂CO-4-(2-oxo-piperazinyl) |
| 70 | Dibenzofuran-2-yl | 1 | CH₂CO-4-(2-oxo-piperazinyl) |
| 71 | Dibenzofuran-2-yl | 1 | CH₂CO-1-(4-isopropylaminocarbonyl)piperazinyl |
| 72 | Dibenzofuran-2-yl | 1 | CH₂CO-1-(4-aminocarbonyl)-piperazinyl |
| 73 | Dibenzofuran-2-yl | 1 | CH₂CO-1-(4-pyrrolidinylcarbonyl)-piperazinyl |
| 74 | Dibenzofuran-2-yl | 1 | CH₂CO-1-(4-dimethylaminocarbonyl)piperazinyl |
| 75 | Dibenzofuran-2-yl | 1 | CH₂CO-1-(4-benzyloxycarbonyl)-piperazinyl |
| | Dibenzofuran-2-yl | 0 | CH₂CH₂CONH₂ |
| 76 | Dibenzofuran-2-yl | 1 | CH₂CH₂CONH₂ |
| | Dibenzofuran-2-yl | 0 | CH₂CH₂CO-1-piperazinyl-N-Boc |
| 77 | Dibenzofuran-2-yl | 1 | CH₂CH₂CO-1-piperazinyl |
| 78 | Dibenzofuran-2-yl | 1 | CH₂CH₂CO-1-(4-acetyl)-piperazinyl |
| 79 | Dibenzofuran-2-yl | 1 | CH₂CON-[3-(2-oxo-pyrrolidin-1-yl)-propyl] |
| | Dibenzofuran-2-yl | 0 | CH₂CON-(2-pyrrolidin-1-yl-ethyl) |
| 80 | Dibenzofuran-2-yl | 1 | CH₂CON-(2-pyrrolidin-1-yl-ethyl) |
| | Dibenzofuran-2-yl | 0 | CH₂CON-(2-piperidin-1-yl-ethyl) |
| 81 | Dibenzofuran-2-yl | 1 | CH₂CON-(2-piperidin-1-yl-ethyl) |
| | Dibenzofuran-2-yl | 0 | CH₂CON-(2-morpholin-4-yl-ethyl) |
| 82 | Dibenzofuran-2-yl | 1 | CH₂CON-(2-morpholin-4-yl-ethyl |
| | Dibenzofuran-2-yl | 0 | H |
| 83 | Dibenzofuran-2-yl | 1 | H |
| | 6-Chloro-dibenzofuran-2-yl | 0 | CH₂CO-1-piperazinyl-N-Boc |
| 84 | 6-Chloro-dibenzofuran-2-yl | 1 | CH₂CO-1-piperazinyl |
| | 6-Chloro-dibenzofuran-2-yl | 0 | CH₂CO-1-(4-acetyl)-piperazinyl |
| 85 | 6-Chloro-dibenzofuran-2-yl | 1 | CH₂CO-1-(4-acetyl)-piperazinyl |
| 22 | 8-Chloro-dibenzofuran-2-yl | 0 | CH₂CO-1-piperazinyl-N-Boc |
| 41 | 8-Chloro-dibenzofuran-2-yl | 1 | CH₂CO-1-piperazinyl |
| | 8-Chloro-dibenzofuran-2-yl | 0 | CH₂CONH₂ |
| 86 | 8-Chloro-dibenzofuran-2-yl | 1 | CH₂CONH₂ |
| 23 | 8-Chloro-dibenzofuran-2-yl | 0 | CH₂CO-1-(4-acetyl)-piperazinyl |
| 42 | 8-Chloro-dibenzofuran-2-yl | 1 | CH₂CO-1-(4-acetyl)-piperazinyl |
| 146 | 8-Chloro-dibenzofuran-2-yl | 2 | CH₂CO-1-(4-acetyl)-piperazinyl |
| 21 | 8-Chloro-dibenzofuran-2-yl | 0 | CH₂CO-1-(4-hydroxyethyl)-piperazinyl |
| 40 | 8-Chloro-dibenzofuran-2-yl | 1 | CH₂CO-1-(4-hydroxyethyl)-piperazinyl |
| 17 | 8-Methoxy-dibenzofuran-2-yl | 0 | CH₂CONH₂ |
| 37 | 8-Methoxy-dibenzofuran-2-yl | 1 | CH₂CONH₂ |
| | 8-Methoxy-dibenzofuran-2-yl | 0 | CH₂CO-1-piperazinyl-N-Boc |
| 87 | 8-Methoxy-dibenzofuran-2-yl | 1 | CH₂CO-1-piperazinyl |
| | 8-Methoxy-dibenzofuran-2-yl | 0 | CH₂CO-1-(4-acetyl)-piperazinyl |
| 88 | 8-Methoxy-dibenzofuran-2-yl | 1 | CH₂CO-1-(4-acetyl)-piperazinyl |
| 26 | 8-Fluoro-dibenzofuran-2-yl | 0 | CH₂CO-1-piperazinyl-N-Boc |
| 45 | 8-Fluoro-dibenzofuran-2-yl | 1 | CH₂CO-1-piperazinyl |
| | 8-Fluoro-dibenzofuran-2-yl | 0 | CH₂CO-1-(4-acetyl)-piperazinyl |
| 89 | 8-Fluoro-dibenzofuran-2-yl | 1 | CH₂CO-1-(4-acetyl)-piperazinyl |
| 19 | 8-Fluoro-dibenzofuran-2-yl | 0 | CH₂CONH₂ |
| 38 | 8-Fluoro-dibenzofuran-2-yl | 1 | CH₂CONH₂ |
| | 4-Chloro-dibenzofuran-2-yl | 0 | CH₂CONH₂ |
| 90 | 4-Chloro-dibenzofuran-2-yl | 1 | CH₂CONH₂ |
| | 4-Fluoro-dibenzofuran-2-yl | 0 | CH₂CONH₂ |
| 91 | 4-Fluoro-dibenzofuran-2-yl | 1 | CH₂CONH₂ |
| | 4-Chloro-dibenzofuran-2-yl | 0 | CH₂CO-1-(4-acetyl)-piperazinyl |
| 92 | 4-Chloro-dibenzofuran-2-yl | 1 | CH₂CO-1-(4-acetyl)-piperazinyl |
| | 4-Fluoro-dibenzofuran-2-yl | 0 | CH₂CO-1-(4-acetyl)-piperazinyl |
| 93 | 4-Fluoro-dibenzofuran-2-yl | 1 | CH₂CO-1-(4-acetyl)-piperazinyl |
| | 4-Fluoro-8-chloro-dibenzofuran-2-yl | 0 | CH₂CONH₂ |
| 94 | 4-Fluoro-8-chloro-dibenzofuran-2-yl | 1 | CH₂CONH₂ |
| | Dibenzofuran-4-yl | 0 | CH₂CONHCH₂CH₂OH |
| 95 | Dibenzofuran-4-yl | 1 | CH₂CONHCH₂CH₂OH |
| | Dibenzofuran-4-yl | 0 | CH₂CONH₂ |
| 96 | Dibenzofuran-4-yl | 1 | CH₂CONH₂ |
| | Dibenzofuran-4-yl | 0 | CH₂CO-1-(4-acetyl)-piperazinyl |
| 97 | Dibenzofuran-4-yl | 1 | CH₂CO-1-(4-acetyl)-piperazinyl |
| | Dibenzofuran-4-yl | 0 | CH₂CO-1-(4-hydroxy)piperidinyl |
| 98 | Dibenzofuran-4-yl | 1 | CH₂CO-1-(4-hydroxy)piperidinyl |
| | Dibenzofuran-4-yl | 0 | CH₂CO-1-piperazinyl-N-Boc |
| 99 | Dibenzofuran-4-yl | 1 | CH₂CO-1-piperazinyl |
| | Dibenzofuran-4-yl | 0 | CH₂CON-(2-pyrrolidin-1-yl-ethyl) |
| 100 | Dibenzofuran-4-yl | 1 | CH₂CON-(2-pyrrolidin-1-yl-ethyl) |
| | Dibenzofuran-4-yl | 0 | CH₂CON-[3-(2-oxo-pyrrolidin-1-yl)-propyl] |
| 101 | Dibenzofuran-4-yl | 1 | CH₂CON-[3-(2-oxo-pyrrolidin-1-yl)-propyl] |
| | Dibenzofuran-4-yl | 0 | CH₂CON-(2-piperidin-1-yl-ethyl) |
| 102 | Dibenzofuran-4-yl | 1 | CH₂CON-(2-piperidin-1-yl-ethyl) |
| | Dibenzofuran-4-yl | 0 | CH₂CON-(2-morpholin-4-yl-ethyl) |
| 103 | Dibenzofuran-4-yl | 1 | CH₂CON-(2-morpholin-4-yl-ethyl) |
| | Dibenzofuran-4-yl | 0 | CH₂CO-1-(4-hydroxyethyl)-piperazinyl |
| 104 | Dibenzofuran-4-yl | 1 | CH₂CO-1-(4-hydroxyethyl)-piperazinyl |
| | Dibenzofuran-3-yl | 0 | CH₂CO-1-piperazinyl-N-Boc |
| 105 | Dibenzofuran-3-yl | 1 | CH₂CO-1-piperazinyl |
| | Dibenzofuran-1-yl | 0 | CH₂CO-1-piperazinyl-N-Boc |
| 106 | Dibenzofuran-1-yl | 1 | CH₂CO-1-piperazinyl |
| | Dibenzofuran-3-yl | 0 | CH₂CO-1-(4-acetyl)-piperazinyl |
| 107 | Dibenzofuran-3-yl | 1 | CH₂CO-1-(4-acetyl)-piperazinyl |
| 25 | Dibenzothiophen-2-yl | 0 | CH₂CO-1-piperazinyl-N-Boc |
| 44 | Dibenzothiophen-2-yl | 1 | CH₂CO-1-piperazinyl |
| | Dibenzothiophen-2-yl | 0 | CH₂CO-1-(4-acetyl)-piperazinyl |
| 108 | Dibenzothiophen-2-yl | 1 | CH₂CO-1-(4-acetyl)-piperazinyl |
| | Dibenzothiophen-2-yl | 0 | CH₂CO-1-(4-ethoxycarbonyl)-piperazinyl |
| 109 | Dibenzothiophen-2-yl | 1 | CH₂CO-1-(4-ethoxycarbonyl)-piperazinyl |
| 18 | Dibenzothiophen-2-yl | 0 | CH₂CONH₂ |
| 39 | Dibenzothiophen-2-yl | 1 | CH₂CONH₂ |
| | Dibenzothiophen-4-yl | 0 | CH₂CO-1-(4-acetyl)-piperazinyl |
| 110 | Dibenzothiophen-4-yl | 1 | CH₂CO-1-(4-acetyl)-piperazinyl |
| | Fluoren-1-yl | 0 | CH₂CONHCH₂CH₂OH |
| 111 | Fluoren-1-yl | 1 | CH₂CONHCH₂CH₂OH |
| | Fluoren-1-yl | 0 | CH₂CO-1-(4-acetyl)-piperazinyl |
| 112 | Fluoren-1-yl | 1 | CH₂CO-1-(4-acetyl)-piperazinyl |
| | Fluoren-1-yl | 0 | CH₂CO-1-(4-hydroxy)piperidinyl |
| 113 | Fluoren-1-yl | 1 | CH₂CO-1-(4-hydroxy)piperidinyl |
| | Fluoren-1-yl | 0 | CH₂CONH₂ |
| 114 | Fluoren-1-yl | 1 | CH₂CONH₂ |
| | Fluoren-1-yl | 0 | CH₂CO-1-(4-hydroxyethyl)-piperazinyl |
| 115 | Fluoren-1-yl | 1 | CH₂CO-1-(4-hydroxyethyl)-piperazinyl |
| | Fluoren-1-yl | 0 | CH₂CO-1-piperazinyl-N-Boc |
| 116 | Fluoren-1-yl | 1 | CH₂CO-1-piperazinyl |
| | Fluoren-2-yl | 0 | CH₂CONH₂ |
| 117 | Fluoren-2-yl | 1 | CH₂CONH₂ |
| | Fluoren-2-yl | 0 | CH₂CON(CH₃)₂ |
| 118 | Fluoren-2-yl | 1 | CH₂CON(CH₃)₂ |
| | Fluoren-2-yl | 0 | CH₂CO-N-pyrrolidinyl |
| 119 | Fluoren-2-yl | 1 | CH₂CO-N-pyrrolidinyl |
| | Fluoren-2-yl | 0 | CH₂CONHCH(CH₃)₂ |
| 120 | Fluoren-2-yl | 1 | CH₂CONHCH(CH₃)₂ |
| | Fluoren-2-yl | 0 | CH₂CONHCH₂CH₂OH |
| 121 | Fluoren-2-yl | 1 | CH₂CONHCH₂CH₂OH |
| | Fluoren-2-yl | 0 | CH₂CO-1-(4-hydroxy)piperidinyl |
| 122 | Fluoren-2-yl | 1 | CH₂CO-1-(4-hydroxy)piperidinyl |
| | Fluoren-2-yl | 0 | CH₂CO-1-(4-acetyl)-piperazinyl |
| 123 | Fluoren-2-yl | 1 | CH₂CO-1-(4-acetyl)-piperazinyl |
| | Fluoren-4-yl | 0 | CH₂CONH₂ |
| 124 | Fluoren-4-yl | 1 | CH₂CONH₂ |
| | Fluoren-4-yl | 0 | CH₂CON(CH₃)₂ |
| 125 | Fluoren-4-yl | 1 | CH₂CON(CH₃)₂ |
| | Fluoren-4-yl | 0 | CH₂CONHCH(CH₃)₂ |
| 126 | Fluoren-4-yl | 1 | CH₂CONHCH(CH₃)₂ |
| | Fluoren-4-yl | 0 | CH₂CONHCH₂CH₂OH |
| 127 | Fluoren-4-yl | 1 | CH₂CONHCH₂CH₂OH |
| | Fluoren-4-yl | 0 | CH₂CO-1-(4-hydroxy)piperidinyl |
| 128 | Fluoren-4-yl | 1 | CH₂CO-1-(4-hydroxy)piperidinyl |
| | Fluoren-4-yl | 0 | CH₂CO-1-(4-acetyl)-piperazinyl |
| 129 | Fluoren-4-yl | 1 | CH₂CO-1-(4-acetyl)-piperazinyl |
| | Fluoren-4-yl | 0 | CH₂CO-1-piperazinyl-N-Boc |
| 130 | Fluoren-4-yl | 1 | CH₂CO-1-piperazinyl |
| 24 | Fluoren-4-yl | 0 | CH₂CO-1-(4-hydroxyethyl)-piperazinyl |
| 43 | Fluoren-4-yl | 1 | CH₂CO-1-(4-hydroxyethyl)-piperazinyl |
| | Fluoren-4-yl | 0 | CH₂CO-1-(4-formyl)-piperazinyl |
| 131 | Fluoren-4-yl | 1 | CH₂CO-1-(4-formyl)-piperazinyl |
| | 2-Phenylbenzofuran-3-yl | 0 | CH₂CONH₂ |
| 132 | 2-Phenylbenzofuran-3-yl | 1 | CH₂CONH₂ |
| | 2-Phenylbenzofuran-3-yl | 0 | CH₂CO-N-pyrrolidinyl |
| 133 | 2-Phenylbenzofuran-3-yl | 1 | CH₂CO-N-pyrrolidinyl |
| | 2-Phenybenzofuran-3-yl | 0 | CH₂CH₂CONH₂ |
| 134 | 2-Phenylbenzofuran-3-yl | 1 | CH₂CH₂CONH₂ |
| | 2-Phenylbenzofuran-3-yl | 0 | CH₂CH₂CO-N-pyrrolidinyl |
| 135 | 2-Phenylbenzofuran-3-yl | 1 | CH₂CH₂CO-N-pyrrolidinyl |
| 20 | 2-Phenylbenzofuran-3-yl | 0 | CH₂CON(CH₃)₂ |
| 46 | 2-Phenylbenzofuran-3-yl | 1 | CH₂CON(CH₃)₂ |
| | 2-Phenylbenzofuran-3-yl | 0 | CH₂CH₂CON(CH₃)₂ |
| 136 | 2-Phenylbenzofuran-3-yl | 1 | CH₂CH₂CON(CH₃)₂ |
| | 2-Phenylbenzofuran-3-yl | 0 | CH₂CONHCH(CH₃)₂ |
| 137 | 2-Phenylbenzofuran-3-yl | 1 | CH₂CONHCH(CH₃)₂ |
| | 2-Phenylbenzofuran-3-yl | 0 | CH₂CH₂CONHCH(CH₃)₂ |
| 138 | 2-Phenylbenzofuran-3-yl | 1 | CH₂CH₂CONHCH(CH₃)₂ |
| | 2-Phenylbenzofuran-3-yl | 0 | CH₂CO-1-(4-acetyl)-piperazinyl |
| 139 | 2-Phenylbenzofuran-3-yl | 1 | CH₂CO-1-(4-acetyl)-piperazinyl |
| | 2-Phenylbenzofuran-3-yl | 0 | CH₂CH₂CO-1-(4-acetyl)-piperazinyl |
| 140 | 2-Phenylbenzofuran-3-yl | 1 | CH₂CH₂CO-1-(4-acetyl)-piperazinyl |
| | 3-Phenylbenzothiophen-2-yl | 0 | CH₂CONH₂ |
| 141 | 3-Phenylbenzothiophen-2-yl | 1 | CH₂CONH₂ |
| 27 | 3-phenylbenzothiophen-2-yl | 0 | CH2-CO-*N*-pyrrolidinyl |
| 28 | 3-phenylbenzothiophen-2-yl | 0 | CH2-CON(CH₃)₂ |
| 29 | 3-phenylbenzothiophen-2-yl | 0 | CH2-CONHCH(CH₃)₂ |
| 30 | 3-phenylbenzothiophen-2-yl | 0 | CH2-CO-1-(4-hydroxy)-piperidinyl |
| 31 | 3-phenylbenzothiophen-2-yl | 0 | CH2-CO-1-(4-acetyl)-piperazinyl |
| 32 | 3-phenylbenzothiophen-2-yl | 0 | CH2-CONH(CH₂)₂OH |
| 47 | 3-phenylbenzothiophen-2-yl | 1 | CH2-CO-*N*-pyrrolidinyl |
| 48 | 3-phenylbenzothiophen-2-yl | 1 | CH2-CON(CH₃)₂ |
| 49 | 3-phenylbenzothiophen-2-yl | 1 | CH2-CONHCH(CH₃)₂ |
| 50 | 3-phenylbenzothiophen-2-yl | 1 | CH2-CO-1-(4-hydroxy)-piperidinyl |
| 51 | 3-phenylbenzothiophen-2-yl | 1 | CH2-CO-1-(4-acetyl)-piperazinyl |
| 52 | 3-phenylbenzothiophen-2-yl | 1 | CH2-CONH(CH₂)₂OH |
| 33 | 3-phenyl-1,4-benzodioxin-2-yl | 0 | CH₂CO-1-(4-acetyl)-piperazinyl |
| 53 | 3-phenyl-1,4-benzodioxin-2-yl | 1 | CH₂CO-1-(4-acetyl)-piperazinyl |
| | 3-phenyl-1,4-benzodioxin-2-yl | 0 | CH₂CONHCH(CH₃)₂ |
| 142 | 3-phenyl-1,4-benzodioxin-2-yl | 1 | CH₂CONHCH(CH₃)₂ |
| | 3-phenyl-1,4-benzodioxin-2-yl | 0 | CH₂CONH₂ |
| 143 | 3-phenyl-1,4-benzodioxin-2-yl | 1 | CH₂CONH₂ |
| | 6,7-dichloro-3-phenyl-1,4-benzodioxin-2-yl | 0 | CH₂CONH₂ |
| 144 | 6,7-dichloro-3-phenyl-1,4-benzodioxin-2-yl | 1 | CH₂CONH₂ |
| | 6,7-dichloro-3-phenyl-1,4-benzodioxin-2-yl | 0 | CH₂CO-1-(4-acetyl)-piperazinyl |
| 145 | 6,7-dichloro-3-phenyl-1,4-benzodioxin-2-yl | 1 | CH₂CO-1-(4-acetyl)-piperazinyl |
| 148 | 3-phenyl-1H-indol-2-yl | 0 | CH₂CONH₂ |
| 149 | 3-phenyl-1H-indol-2-yl | 1 | CH₂CONH₂ |

8. A use of a compound of formula (A) : 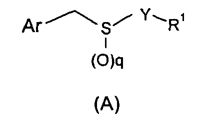 wherein :
Ar is: 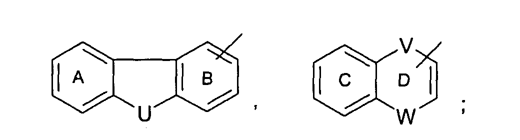 wherein:
U is CH₂, CR²⁵R²⁶, O, S(O)_{y}, NR¹⁰, C(=O), C(=S), CHOH, CHOR¹⁴, C=NOR¹⁴, or C=NNR¹²R¹³;
V and W are independently selected from a bond, CH₂, CR²⁵R²⁶, O, S(O)_{y}, NR¹⁰, C(=O), C(=S), CHOH, CHOR¹⁴, C=NOR¹⁴, or C=NNR¹²R¹³;
rings A, B, and C are optionally substituted with one to three groups selected from F, Cl, Br, I, OR²², OR²⁷, NR²³R²⁴, NHOH, NO₂, CN, CF₃, C₁-C₆ alkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
ring D is optionally substituted with one group selected from C₁-C₆ alkyl, phenyl, and 5-10 membered heteroaryl;
Y is C₁-C₆ alkylene; or
(C₁-C₄ alkylene)ₘ-Z-(C₁-C₄ alkylene)ₙ;
wherein said alkylene groups are optionally substituted with one to three R²⁰ groups;
Z is O, NR^{10A}, S(O)_{y}, CR²¹=CR²¹, C≡C, C₆-C₁₀ arylene, 5-10 membered heteroarylene, C₃-C₆ cycloalkylene, or 3-6 membered heterocycloalkylene; wherein said arylene, heteroarylene, cycloalkylene, and heterocycloalkylene groups are optionally substituted with one to three R²⁰ groups;
R¹ is selected from H, NR¹²R¹³, NR²¹C(=O)R¹⁴, C(=O)R¹⁴, CO₂R¹¹, OC(=O)R¹¹, C(=O)NR¹²R¹³, C(=NR¹¹)NR¹²R¹³, OC(=O)NR¹²R¹³, NR²¹S(O)₂R¹¹, NR²¹C(=O)NR¹²R¹³, NR²¹(SO₂)NR¹²R¹³, and C(=O)NR¹¹OR²²;
R¹⁰ and R^{10A} are each independently selected from H, C₁-C₆ alkyl, C₆-C₁₀ aryl, C(=O)R¹⁴, and S(O)_{y}R¹⁴; wherein said alkyl and aryl groups are optionally substituted with one to three R²⁰ groups;
R¹¹ at each occurrence is independently selected from H, C₁-C₆ alkyl, and C₆-C₁₀ aryl; wherein said alkyl and aryl groups are optionally substituted with one to three R²⁰ groups;
R¹² and R¹³ at each occurrence are each independently selected from H, C₁-C₆ alkyl, C₆-C₁₀ aryl, and NR²³R²⁴, or R¹² and R¹³, together with the nitrogen to which they are attached, form a 3-7 membered heterocyclic ring;
wherein said alkyl and aryl groups and heterocyclic ring are optionally substituted with one to three R²⁰ groups;
R¹⁴ at each occurrence is independently selected from C₁-C₆ alkyl, C₆-C₁₀ aryl, and alkylaryl; wherein said alkyl, aryl and alkylaryl groups are optionally substituted with one to three R²⁰ groups;
R²⁰ at each occurrence is independently selected from F, Cl, Br, I, OR²², OR²⁷, NR²³R²⁴, NHOH, NO₂, CN, CF₃, C₁-C₆ alkyl optionally substituted with OH, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
R²¹ at each occurrence is independently selected from H and C₁-C₆ alkyl;
R²² at each occurrence is independently selected from H, C₁-C₆ alkyl optionally substituted with OH, arylalkyl and C₆-C₁₀ aryl;
R²³ and R²⁴ at each occurrence are each independently selected from H, C₁-C₆ alkyl, and C₆-C₁₀ aryl, or R²³ and R²⁴, together with the nitrogen to which they are attached, form a 3-7 membered heterocyclic ring optionally substituted with =O;
R²⁵ and R²⁶ at each occurrence are each independently selected from H, C₁-C₆ alkyl, and C₆-C₁₀ aryl, or R²⁵ and R²⁶, together with the carbon to which they are attached, form a 3-7 membered heterocyclic ring;
R²⁷ at each occurrence is independently the residue of an amino acid after the hydroxyl group of the carboxyl group is removed;
m is 0 or 1;
n is 0 or 1;
q is 0, 1, or 2;
y is 0, 1, or 2;
and the stereoisomeric forms, mixtures of stereoisomeric forms or pharmaceutically acceptable salts forms thereof,
for the treatment of sleepiness associated with a sleep affecting disease or disorder such as narcolepsy, obstructive sleep apnea or shift work disorder; a neurological disease or disorder such as Parkinson's disease; Alzheimer's disease; attention deficit disorder; attention deficit hyperactivity disorder; depression; and fatigue.

9. A pharmaceutical composition comprising a compound of formula (A): 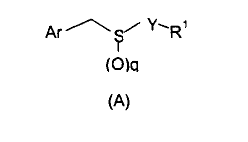 wherein :
Ar is: 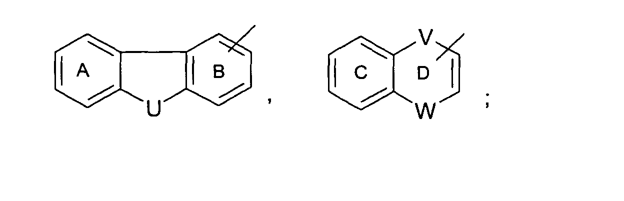 wherein:
U is CH₂, CR²⁵R²⁶, O, S(O)_{y}, NR¹⁰, C(=O), C(=S), CHOH, CHOR¹⁴, C=NOR¹⁴, or C=NNR¹²R¹³;
V and W are independently selected from a bond, CH₂, CR²⁵R²⁶, O, S(O)_{y}, NR¹⁰, C(=O), C(=S), CHOH, CHOR¹⁴, C=NOR¹⁴, or C=NNR¹²R¹³;
rings A, B, and C are optionally substituted with one to three groups selected from F, Cl, Br, I, OR²², OR²⁷, NR²³R²⁴, NHOH, NO₂, CN, CF₃, C₁-C₆ alkyl, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
ring D is optionally substituted with one group selected from C₁-C₆ alkyl, phenyl, and 5-10 membered heteroaryl;
Y is C₁-C₆ alkylene; or
(C₁-C₄ alkylene)ₘ-Z-(C₁-C₄ alkylene)ₙ;
wherein said alkylene groups are optionally substituted with one to three R²⁰ groups;
Z is O, NR^{10A}, S(O)_{y}, CR²¹=CR²¹, C≡C, C₆-C₁₀ arylene, 5-10 membered heteroarylene, C₃-C₆ cycloalkylene, or 3-6 membered heterocycloalkylene; wherein said arylene, heteroarylene, cycloalkylene, and heterocycloalkylene groups are optionally substituted with one to three R²⁰ groups;
R¹ is selected from H, NR¹²R¹³, NR²¹C(=O)R¹⁴, C(=O)R¹⁴, CO₂R¹¹, OC(=O)R¹¹, C(=O)NR¹²R¹³, C(=NR¹¹)NR¹²R¹³, OC(=O)NR¹²R¹³, NR²¹S(O)₂R¹¹, NR²¹C(=O)NR¹²R¹³, NR²¹(SO₂)NR¹²R¹³ and C(=O)NR¹¹ OR²²;
R¹⁰ and R^{10A} are each independently selected from H, C₁-C₆ alkyl, C₆-C₁₀ aryl, C(=O)R¹⁴, and S(O)_{y}R¹⁴; wherein said alkyl and aryl groups are optionally substituted with one to three R²⁰ groups;
R¹¹ at each occurrence is independently selected from H, C₁-C₆ alkyl, and C₆-C₁₀ aryl; wherein said alkyl and aryl groups are optionally substituted with one to three R²⁰ groups;
R¹² and R¹³ at each occurrence are each independently selected from H, C₁-C₆ alkyl, C₆-C₁₀ aryl, and NR²³R²⁴, or R¹² and R¹³, together with the nitrogen to which they are attached, form a 3-7 membered heterocyclic ring;
wherein said alkyl and aryl groups and heterocyclic ring are optionally substituted with one to three R²⁰ groups;
R¹⁴ at each occurrence is independently selected from C₁-C₆ alkyl, C₆-C₁₀ aryl, and alkylaryl; wherein said alkyl, aryl and alkylaryl groups are optionally substituted with one to three R²⁰ groups;
R²⁰ at each occurrence is independently selected from F, Cl, Br, I, OR²², OR²⁷, NR²³R²⁴, NHOH, NO₂, CN, CF₃, C₁-C₆ alkyl optionally substituted with OH, C₂-C₆ alkenyl, C₂-C₆ alkynyl, C₃-C₇ cycloalkyl, 3-7 membered heterocycloalkyl, phenyl, 5 or 6 membered heteroaryl, arylalkyl, =O, C(=O)R²², CO₂R²², OC(=O)R²², C(=O)NR²³R²⁴, NR²¹C(=O)R²², NR²¹CO₂R²², OC(=O)NR²³R²⁴, NR²¹C(=S)R²², and S(O)_{y}R²²;
R²¹ at each occurrence is independently selected from H and C₁-C₆ alkyl;
R²² at each occurrence is independently selected from H, C₁-C₆ alkyl optionally substituted with OH, arylalkyl and C₆-C₁₀ aryl;
R²³ and R²⁴ at each occurrence are each independently selected from H, C₁-C₆ alkyl, and C₆-C₁₀ aryl, or R²³ and R²⁴, together with the nitrogen to which they are attached, form a 3-7 membered heterocyclic ring optionally substituted with =O;
R²⁵ and R²⁶ at each occurrence are each independently selected from H, C₁-C₆ alkyl, and C₆-C₁₀ aryl, or R²⁵ and R²⁶, together with the carbon to which they are attached, form a 3-7 membered heterocyclic ring;
R²⁷ at each occurrence is independently the residue of an amino acid after the hydroxyl group of the carboxyl group is removed;
m is 0 or 1;
n is 0 or 1;
q is 0, 1, or 2;
y is 0, 1, or 2;
and the stereoisomeric forms, mixtures of stereoisomeric forms or pharmaceutically acceptable salts forms thereof,
in admixture with one or more pharmaceutically acceptable excipients.

10. A method for preparing a compound of claims 1 to 7, comprising the steps of :
a1) reacting a compound F with a compound G to form a compound of formula (Ia) : 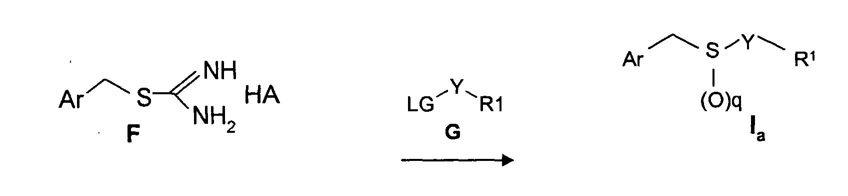 wherein Ar, Y, R¹ are as defined in claim 1, q = 0 and LG is a leaving group ; and optionally
b1) isolating the formed compound (Ia).
